# EUROPEAN PATENT SPECIFICATION

(11) **EP 1 078 053 B1**
(45) Date of publication and mention of the grant of the patent: **28.09.2005**
(21) Application number: 99928176.9
(22) Date of filing: 14.05.1999
(51) Int. Cl.: C12N 15/11, A61K 39/39, A61K 31/70, C07H 21/04, A61K 45/00

(54) **METHODS FOR REGULATING HEMATOPOIESIS USING CpG-OLIGONUCLEOTIDES**
VERFAHREN ZUR REGULIERUNG DER HEMATOPOIESE MIT HILFE VON CPG OLIGONUKLEOTIDE
PROCEDES DE REGULATION DE L'HEMATOPOIESE AU MOYEN D'OLIGONUCLEOTIDES A CpG

(30) Priority: 14.05.1998 US 85516 P; 02.02.1999 US 241653
(43) Date of publication of application: 28.02.2001
(73) Proprietor: Coley Pharmaceutical GmbH, 40764 Langenfeld (DE); Coley Pharmaceutical Group, Inc., Wellesley, MA 02481 (US)
(72) Inventor: WAGNER, Hermann Institute of Med. Microbio., 81675 Munich (DE); LIPFORD, Grayson, Coley Pharmaceutical Group, Inc., Wellesley, MA 02481 (US)
(74) Representative: Gill, Siân Victoria
(86) International application number: PCT/IB1999/001285
(87) International publication number: WO 1999/058118

(56) References cited:
- WO-A-96/02555
- WO-A-97/28259
- WO-A-98/11211
- WO-A-98/18810
- AGRAWAL S. ET AL.: "Toxicologic effects of an oligodeoxynucleotide phosphorothioate and its analogs following intravenous administration in rats." ANTISENSE NUCLEIC ACID DRUG DEV 1997 DEC;7(6):575-84, XP002122934 cited in the application
- G. B. LIPFORD ET AL: "Immunostimulatory DNA: sequence-dependent production of potentially harmful or useful cytokines" EUROPEAN JOURNAL OF IMMUNOLOGY, vol. 12, no. 27, page 3420-3426 XP002077483 ISSN: 0014-2980 cited in the application
- KRIEG AM. ET AL.: "CpG DNA induces sustained IL-12 expression in vivo and resistance to Listeria monocytogenes challenge." J IMMUNOL 1998 SEP 1;161(5):2428-34, XP002122935
- JAKOB T. ET AL.: "Bacterial DNA and CpG-containing oligodeoxynucleotides activate cutaneous dendritic cells and induce IL-12 production: implications for the augmentation of Th1 responses." INT ARCH ALLERGY IMMUNOL 1999 FEB-APR;118(2-4):457-61, XP002122936

## Description

### Field Of The Invention

The present invention relates to methods for regulating hematopoiesis using CpG containing oligonucleotides. In particular, the invention relates to methods of treating thrombopoiesis and anemia by regulating hematopoiesis. The invention also relates to methods of regulating immune system remodeling by administering CpG oligonucleotides to manipulate hematopoiesis.

### Background Of The Invention

Radiation or chemotherapeutic treatment produces severe reversible thrombocytopenia, anemia and neutropenia. The depletion of hematopoietic precursors in the bone marrow (BM) associated with chemotherapy and irradiation result in hemorrhagic and infectious complications. Severe suppression of the hematopoietic system is a major factor in limiting chemotherapy use and dose escalation. A number of hematopoietic cytokines are currently in clinical trials as treatments to prevent or reduce such complications.

Hematopoietic development is considered to be regulated by two categories of factors. One category includes colony-stimulating factors (CSFs), which promote colony formation and proliferation of cells of various lineages. Another is potentiators, which potentiate maturation or differentiation. For example, Megakaryocyte-CSFs (Meg-CSFs) reportedly include IL-3, granulocyte-macrophage colony-stimulating factor (GM-CSF) and stem cell factor (SCF), and Megakaryocyte potentiators (Meg-Pot) reportedly include IL-6, IL-7, IL-11, erythropoietin (EPO) and leukemia inhibitory factor (LIF). Platelet production is a terminal phenomenon in the development of megakaryocytes *in vivo.* Thrombopoietin (TPO) was reported to posses both Meg-CSF and Meg-Pot.

In early days of interferon (IFN) research Isaacs et al. postulated that foreign DNA induces IFN. Rotem, Z et al. (1963) Nature 197:564-566; Jensen, KE et al. (1963) Nature 200:433-434. Later it was discovered that synthetic double-stranded RNA was able to induce IFN and to activate both natural killer (NK) cells and macrophages. Field, AK et al. (1967) Proc Natl Acad Sci USA 58:1004-1010. Subsequently, Yamamoto, Tokunaga and colleagues discovered immunostimulatory DNA by a series of studies originally aimed at analyzing bacille Calmette-Guerin (BCG) mediated tumor resistance in mice. A fraction extracted from BCG (designated MY-1) was shown to exhibit anti-tumor activity *in vivo,* augment NK cell activity and trigger type I and type II IFN release from murine spleen cells or human peripheral blood lymphocytes (PBL) *in vitro.* Tokunaga, T et al. (1984) J Natl Cancer Inst 72:955-962; Yamamoto, S et al. (1988) Jpn J Cancer Res 79:866-873; Mashiba, H et al. (1988) Jpn J Med Sci Biol 41:197-202. These activities could be destroyed by DNase pretreatment of MY-1, but not by RNase treatment. Pisetsky and co-workers independently observed that normal mice, as well as humans, respond to bacterial DNA, but not vertebrate DNA, by producing anti-DNA antibodies. Messina, JP et al. (1991) J Immunol 147:1759-1764. They realized that bacterial DNA was mitogenic for murine B cells and postulated that this activity resulted from "non-conserved structural determinants." The differential stimulative capacity of bacterial DNA versus vertebrate DNA was also demonstrated for induction of NK cell activity by Yamamoto et al. Yamamoto, S et al. (1992) Microbiol Immunol 36:983-997. HPLC analysis of BCG extracts showed that the MY-1 fraction was composed of a broad size range of DNA fragments with a peak at 45 bases. Synthetic 45-mer oligodeoxynucleotides (ODNs) derived from BCG cDNA sequences were positive for IFN-inducing capacity and augmentation of NK cytotoxicity. Tokunaga, T et al. (1992) Microbiol Immunol 36:55-66.

Concurrently, investigators studying antisense ODN observed sequence-dependent immune stimulatory effects. Subsequently, Krieg et al. formulated a framework for understanding the pattern recognition of bacterial or synthetic DNA. Krieg, AM et al. (1995) Nature 374:546-549. Using sequence-specific CpG-containing ODN-mediated mitogenicity to B cells as an assay, they discovered that certain CpG dinucleotides, specifically within DNA motifs displaying a 5'-Pu-Pu-CpG-Pyr-Pyr-3' base sequence, were biologically active.

Bacterial DNA, some viral DNA, and invertebrate DNA seem to differ structurally from vertebrate DNA. Bacterial DNA has the expected frequency of CpG dinucleotides of 1:16. In contrast, mammalian DNA exhibits CpG suppression and has only about one-fourth as many CpG as predicted by random base usage. Bird, AP (1986) Nature 321:209-213. The usage of the 5'-Pu-Pu-CpG-Pyr-Pyr-3' motif is even more suppressed in mammals compared with the genome of *Escherichia coli.* Krieg, AM et al. (1995) Nature 374:546-549. Furthermore, eukaryotic 5'-CpG-3' motifs are preferentially methylated, and sequence-specific methylation of 5'-CpG-3' abolishes their stimulatory potential. Krieg, AM et al. (1995) Nature 374:546-549; Bird, AP (1986) Nature 321:209-213. The realization that these sequences are under-represented in vertebrate DNA offers an explanation for several biological observations in the context of non-self pattern recognition by the immune system.

### CpG DNA induced in vivo immune responses

The immunogenicity of proteinaceous natural and recombinant purified antigens is poor unless aided by adjuvants. Because of the apparent recognition and response to foreign DNA by the immune system, the potential of CpG DNA to serve as an adjuvant was previously tested. Mice were challenged subcutaneously with liposome-encapsulated ovalbumin (used as antigen) and CpG-ODN (used as adjuvant) using a protocol described by Lipford et al. Lipford, GB et al. (1997) Eur J Immunol 27:3420-3426. The mice which were co-administered CpG-ODN developed strong peptide-specific cytotoxic T lymphocyte (CTL) activity in the draining lymph nodes (LNs). Furthermore, not only was the antibody response augmented, but CpG-ODN switched the isotype pattern to a Th1-type profile, in that antigen-specific IgG2a became dominant. Lipford, GB et al. (1997) Eur J Immunol 27:3420-3426. This pattern of strong CTL induction and Th1 biasing in the antibody repertoire has been extended to other protein antigens. Subsequently, it has been found that the use of liposome as antigen carriers is not necessary for CTL induction. This observation was unexpected because typically soluble protein antigens can not enter the major histocompatibility complex (MHC) class I presentation pathway and therefore can not be presented to precursor CTL by antigen-presenting cells (APCs).

The Th1 biasing of CpG DNA when co-administered with protein antigen has now been fully documented. International Publication No. WO 98/18810 discloses the fact that nucleic acid sequences containing immunostimulatory CpG oligonucleotides stimulate a Th1 pattern of immune activation, cytokine production, NK lytic activity and B cell proliferation. Such immunostimulatory CpG oligonucleotides may be administered slightly before or at the same time as a vaccine to treat immune system deficiencies. Roman et al. demonstrated the dominance of antigen-specific IgG2a induction when using as the adjuvant either CpG-ODN, plasmid DNA containing CpG motifs, or bacterial DNA. The Th1-promoting adjuvanticity of CpG-ODN may be useful for the redirection to protective, or even curative, responses in Th2-driven disorders. A model is the CpG-ODN modulation of Th2 driven airway inflammation in a murine model of asthma induced with *Schistosoma mansoni* eggs. Airway eosinophilia, Th2 cytokine induction, IgE production and bronchial hyperreactivity were prevented by CpG-ODN co-administration with egg sensitization. Additionally, egg sensitized mice treated at day 7 post sensitization with CpG-ODN and antigen were protected from airway eosinophilia. Similar results were obtained in an infection model for the redirection of Th2 responses to protective Th1 responses supplied by our demonstration that CpG-ODN protected BALB/c mice against lethal *Leishmania major* infections. Lipford, GB et al. (1997) Eur J Immunol 27:2340-2344; Zimmermann, S et al. (1998) J Immunol 160:3627-3630. Post *L. major* infection C57BL/6 mice develop a Th1 driven response that is protective, however BALB/c mice develop a Th2 driven response that is not protective. In this system of Th2 driven infectious disorder, administration of CpG-ODN cured *Leishmania major* infected BALB/c mice when applied as late as 15 days after infection. The phenotype of the response post CpG intervention was Th1-like although the initial response to *L. major* challenge was Th2-like. CpG-ODN triggers the release of IL-12 into the serum post injection and IL-12 is a known inducer of Th1 differentiation. The wave of IL-12 is transient, however, peaking at 2-4 h and returning to near baseline by 24 h. Experimental treatment with anti-IL-4 or IL-12 following with *L. major* is effective only within the first 3 days; at later time points, even multiple injections of IL-12 fail to influence the course of infection. Since CpG-ODN effectively stimulate NK cells to produce IFN-γ, and since there is evidence that IFN-γ can redirect Th2 responses *in vitro* and *in vivo,* this may be a possible explanation. However, the actual cellular and molecular mechanism of the curative effects are yet poorly understood.

### Induction of splenomegaly by ODN

Splenomegaly is a well-recognized phenomenon accompanying some oligonucleotide injections. Branda et al. observed that mice developed massive splenomegaly and polyclonal hypergammaglobulinemia within 2 days after intravenous injection of a phosphorothioate oligomer that is antisense to a portion of the *rev* region of the HIV-1 genome. Branda, RF et al. (1993) Biochem Pharmacol 45:2037-2043. Histologic examination of spleens from injected animals showed marked expansion of a uniform-appearing population of small lymphocytes. Flow cytometry analysis indicated that the responding cells were predominantly B-lymphocytes. Mojcik et al. observed that injection of mice with antisense to the initiation region of the *env* gene resulted in (i) increased spleen cell numbers, primarily due to an increase in splenic B cells, (ii) increased class II MHC expression on B cells, (iii) increased RNA and DNA synthesis, and (iv) increased numbers of immunoglobulin (Ig)-producing cells. Mojcik, CF et al. (1993) Clin Immunol Immunopathol 67:130-136. They concluded that products of certain endogenous retroviral sequences regulate lymphocyte activation *in vivo*. In efforts to test the efficacy of NF-κB p65 oligonucleotides *in vivo,* McIntyre et al. unexpectedly observed that the control p65-sense, but not the p65-antisense, oligonucleotides caused massive splenomegaly in mice. McIntyre, KW et al. (1993) Antisense Res Dev 3:309-322. In this study they demonstrated a sequence-specific stimulation of splenic cell proliferation, both *in vivo* and *in vitro,* by treatment with p65-sense oligonucleotides. Cells expanded by this treatment were primarily B-220+, sIg+ B cells. The secretion of immunoglobulins by the p65-sense oligonucleotide-treated splenocytes was also enhanced. In addition, the p65-sense-treated splenocytes, but not several other cell lines, showed an upregulation of NF-κB-like activity in the nuclear extracts, an effect not dependent on new protein or RNA synthesis. Zhao et al. concluded that phosphorothioate ODN induce splenomegaly due to B cell proliferation. Zhao, Q et al. (1996) Biochem Pharmacol 51:173-182. In a follow-up study Zhao et al. found administration of the 27-mer-phosphorothioate oligonucleotide into mice resulted in splenomegaly and an increase in IgM production 48 hr post-administration. Zhao, Q et al. (1996) Biochem Pharmacol 52:1537-1544.

Agrawal et al. evaluated the *in vivo* toxicological effects of phosphorothioate oligodeoxynucleotides (PS oligo). Agrawal, S et al. (1997) Antisense Nucleic Acid Drug Dev 7:575-584. Oligodeoxynucleotides were administrated intravenously to male and female rats at doses of 3, 10, and 30 mg/kg/day for 14 days. Rats were killed on day 15, blood samples were collected for hematology and clinical chemistry determinations, and tissues, including lymph nodes, spleens, livers, and kidneys, were subjected to pathologic examinations. The toxicity profiles of four oligodeoxynucleotides were very similar, but differed in magnitude. Alterations in hematology parameters included thrombocytopenia, anemia, and neutropenia. Dose-dependent enlargements of spleen, liver, and kidney were observed. Pathologic studies showed a generalized hyperplasia of the reticuloendothelial system in the tissues examined.

Krieg et al. reported that bacterial DNA and synthetic oligodeoxynucleotides containing unmethylated CpG dinucleotides induce murine B cells to proliferate and secrete immunoglobulin *in vitro* and *in vivo.* Krieg, AM et al. (1995) Nature 374:546-549. This activation is enhanced by simultaneous signals delivered through the antigen receptor. Optimal B cell activation requires a DNA motif in which an unmethylated CpG dinucleotide is flanked by two 5' purines and two 3' pyrimidines. Oligodeoxynucleotides containing this CpG motif induce more than 95% of all spleen B cells to enter the cell cycle. In a study by Monteith et al., treatment of rodents with phosphorothioate oligodeoxynucleotides induced a form of immune stimulation characterized by splenomegaly, lymphoid hyperplasia, hypergammaglobulinemia and mixed mononuclear cellular infiltrates in numerous tissues. Monteith, DK et al. (1997) Anticancer Drug Des 12:421-432. Immune stimulation was evaluated in mice with *in vivo* and *in vitro* studies using a review of historical data and specific *in vivo* and *in vitro* studies. All phosphorothioate oligodeoxynucleotides evaluated induced splenomegaly and B lymphocyte proliferation. Splenomegaly and B-lymphocyte proliferation increased with dose or concentration of oligodeoxynucleotide. The rank order potencies for B-lymphocyte proliferation *in vitro* and splenomegaly correlated well for the oligodeoxynucleotides tested. Thus the overriding evidence provided by the literature concludes that the phenomenon of splenomegaly induced by ODN is probably sequence dependent and explained by B cell mitogenicity.

Hematopoietic development is considered to be regulated by colony-stimulating factors, which promote colony formation and proliferation of cells of various primitive lineages, and potentiators, which potentiate maturation or differentiation into various blood cells. In general, the observation of splenomegaly is explained by direct ODN B cell mitogenicity in a sequence specific manner.

### Cytokine production and hematopoiesis

As described above the cytokine repertoire induced by CpG-ODN injection is Th1 in nature, and ample evidence suggests this exerts a strong Th1 biasing effect to the subsequent immune response development. Zhao et al. administered to mice a 27-mer phosphorothioate oligonucleotide (sequence 5'-TCG TCG CTG TCT CCG CTT CTT CTT GCC-3' SEQ ID NO:109), which had previously been shown to cause splenomegaly and hypergammaglobulinemia upon *in vivo* administration in mice, and studied the pattern and kinetics of cytokine production at both the splenic mRNA and serum protein levels. Zhao et al. (1997) Antisense Nucleic Acid Drug Dev 7:495-502. Following i.p. administration of 50 mg/kg of oligonucleotide, significant increases in the splenic mRNA levels of IL-6, IL-12 p40, IL-1β, and IL-1Ra and serum levels of IL-6, IL-12, MIP-1β, and MCP-1 were observed. In contrast, no significant differences in splenic mRNA levels of IL-2, IL- 4, IL-5, IL-9, IL-13, IL-15, IFN-γ, or MIF or serum levels of IL-2, IL-4, IL-5, IL-10, IFN-γ, or GM-CSF were detected. These studies show a distinct pattern and kinetics of cytokine production following oligonucleotide administration and further demonstrate that cytokine induction is not a general property of phosphorothioate oligonucleotides but is dependent on the sequence and dose of the oligonucleotides. Serum release of IL-1, IL-6, IL-12 and TNF-α was also confirmed by Lipford et al. Lipford, GB et al. (1997) Eur J Immunol 27:2340-2344.

Hendrzak and Brunda demonstrated that administration of IL-12 in mice caused thrombocytopenia, splenomegaly, and mononuclear cell infiltration, an explanation for the splenomegaly. Hendrzak and Brunda (1995) Lab Invest 72:619-637. IL-12 has been shown to be released in response to CpG-ODN and is an inducer of IFN-γ. Control of intracellular bacterial infections requires IFN-γ both for establishing a Th1 T-cell response and for activating macrophages to kill the bacteria. Murray et al. observed that exposure of mice deficient in IFN-γ to mycobacterial infection produces an immune response characterized by a Th2 T-cell phenotype, florid bacterial growth, and death. Murray, PJ et al. (1998) Blood 91:2914-2924. They reported that IFN-γ-deficient mice infected with mycobacteria also undergo a dramatic remodeling of the hematopoietic system. Myeloid cell proliferation proceeds unchecked throughout the course of mycobacterial infection, resulting in a transition to extramedullary hematopoiesis. The splenic architecture of infected IFN-γ-deficient mice is completely effaced by expansion of macrophages, granulocytes, and extramedullary hematopoietic tissue. These features coincide with splenomegaly, an increase in splenic myeloid colony-forming activity, and marked granulocytosis in the peripheral blood. Systemic levels of cytokines are elevated, particularly IL-6 and granulocyte colony-stimulating factor (G-CSF). These results suggest that in addition to its central role in cellular immunity, IFN-γ may be a key cytokine in the coordinate regulation of immune effector cells and myelopoiesis. Several studies have noted the *in vitro* inhibition of colony forming units by IFN-γ. Thus according to the prior art strong Th1 responses as characterized by IFN-γ release may be inhibitory for hematopoiesis events.

Although it has been believed that IL-3/GM-CSF/IL-5 (Th0 and Th2 cytokines) produced by activated T cells play a major role in expansion of hematopoietic cells in emergency, results indicate that the entire function of IL-3/GM-CSF/IL-5 is dispensable for hematopoiesis in emergency as well as in the steady state. Thus, there must be an alternative mechanism to produce blood cells in both situations. IL-13, a recently identified Th2 cytokine, shares some, but not all, IL-4 functions, including inhibition of monocyte and macrophage activation, stimulation of human B cells, and induction of growth and differentiation of mouse bone marrow cells *in vitro*. Lai et al. tested the *in vivo* effects of recombinant mouse IL-13 (rIL-13) from stably transfected, high expressing BW5147 thymoma cells. Lai, YH et al. (1996) J Immunol 156:3166-3173. After purification by anion exchange chromatography, rIL-13 was administered in the peritoneal cavity of BALB/c mice via osmotic pump for 7 days. Spleens from the rIL-13-treated mice were significantly enlarged compared with control spleens due to increased cellularity. In particular, increased numbers of immature erythroblasts and megakaryocytes were observed in splenic sections after rIL-13 treatment. Spleen cells from rIL-13-treated mice showed greatly increased responsiveness *in vitro* to recombinant forms of mouse IL-3, mouse granulocyte-macrophage CSF, or human CSF-1 and, to a lesser extent, to mouse IL-4 or IL-13. Moreover, the rIL-13-treated mice also showed significant increases in CFU-E, CFU-C, and erythroid burst colonies in the spleen, further indicating the presence of increased numbers of hematopoietic precursors. Hematologic analyses indicated that rIL-13 treatment induced slight anemia and striking monocytosis. Finally, spleen cells from rIL-13-treated mice produced significantly more IL-6 upon LPS stimulation. Interestingly, the strong Th2 response induced by *Nippostrongylus brasiliensis* infection was also accompanied by an increase in hematopoietic precursor frequencies in the spleen. Collectively, these data indicate that exogenous rIL-13 induces extramedullary hematopoiesis in mice and suggest that endogenous IL-13, a Th2 cytokine, may contribute to replenishment of effector cells during strong Th2 responses.

### Summary Of The Invention

The prior art as a whole implies that Th2 driven responses are strongly predisposing for extramedullary hematopoiesis. CpG-ODN injection is Th1-biasing and Th2-suppressive. In addition, IFN-γ, the hallmark Th1 cytokine, is considered suppressive for hematopoietic colony forming, and IL-13, a Th2 cytokine has been shown to induce hematopoiesis. Thus the prior art would not suggest to one of skill in the art that the cytokine repertoire released by CpG-ODN injection will lead to hematopoiesis. To the contrary, ODN administration has been shown to lead to thrombocytopenia, anemia, and neutropenia. Additionally the administration of IL-12, a central cytokine in CpG-ODN effects, induces thrombocytopenia. The phenomenon of splenomegaly has been repeatedly correlated with B cell mitogenicity of ODN, suggesting that the ODN induces splenomegaly through B cell activation rather than hematopoiesis.

In one aspect of the present invention there is provided a use of a CpG oligonucleotide having at least 8 nucleotides, for the manufacture of a medicament for inducing an antigen-specific immune response, wherein the CpG oligonucleotide is to be administered to a subject and the subject is exposed to an antigen at least 3 days after administration of the CpG oligonucleotide to the subject, to produce an antigen-specific immune response. Hematopoiesis may be used to treat immune system disorders. The use of CpG oligonucleotide for inducing an antigen-specific immune response is based on the finding that a CpG oligonucleotide can be used to induce remodeling of the immune system by regulating hematopoiesis. After a CpG oligonucleotide and antigen are administered together to a subject an initial immune response occurs. It has been discovered according to the invention that this initial immune response declines rapidly and a new immune response develops after approximately 48 hours. Unexpectedly, when antigen is administered 48 hours or more after the administration of CpG an antigen specific immune response will be mounted to the antigen. This immune response is due to a repopulation of lymph nodes and/or spleen with primed immune cells.

Thus, in one aspect the invention there is provided a use of a CpG oligonucletoide for the manufacture of a medicament for inducing an antigen-specific immune response by administering to a subject an oligonucleotide, having a sequence including at least the following formula:

5' X₁CGX₂ 3'

wherein the oligonucleotide includes at least 8 nucleotides wherein C and G are unmethylated and wherein X₁ and X₂ are nucleotides, and exposing the subject to an antigen at least 3 days after the oligonucleotide is administered to the subject to produce an antigen-specific immune response.

The subject may be exposed to the antigen at least 48 hours after the CpG oligonucleotide is administered to the subject. It has been discovered that immune system remodeling begins to occur within 48 hours of CpG administration. It has also been discovered that the primed immune cells are still capable of responding to antigen even 30 days after CpG administration. In one embodiment the antigen is administered at least 4 days after the oligonucleotide is administered to the subject. In another embodiment the antigen is administered at least 7 days after the oligonucleotide is administered to the subject. In another embodiment the antigen is administered at least 15 days after the oligonucleotide is administered to the subject. In yet another embodiment the antigen is administered at least 30 days after the oligonucleotide is administered to the subject.

The antigen may be any type of antigen known in the art. For instance, in some embodiments the antigen may be cells, cell extracts, proteins, peptides, polysaccharides, polysaccharide conjugates, lipids, glycolipids, carbohydrate, viral extracts, viruses, bacteria, fungi, parasites, and allergens. In other embodiments the antigen may be a nucleic acid encoding an antigen.

In a preferred embodiment the antigen is an allergen and the CpG oligonucleotide is used for the manufacture of a medicament for treating allergy. In another embodiment the antigen is derived from an infectious organism selected from the group consisting of infectious bacteria, infectious viruses, and infectious fungi and the CpG oligonucleotide is used for the manufacture of a medicament for treating an infectious disease.

The subject is exposed to an antigen. The subject may be actively exposed to the antigen. In one embodiment when the subject is actively exposed to the antigen the antigen may be delivered in conjunction with a colloidal dispersion system. The colloidal dispersion system is selected from the group consisting of macromolecular complexes, nanocapsules, microspheres, beads, and lipid-based systems in another embodiment. A lipid-based system is preferably selected from the group consisting of oil-in-water emulsions, micelles, mixed micelles, and liposomes. In another embodiment the antigen may be administered in conjunction with an adjuvant.

The subject may also be passively exposed to the antigen. In one embodiment the subject is a subject at risk of developing cancer. In another embodiment the subject is at risk of developing an allergic reaction. In yet another embodiment the subject is an asthmatic.

The antigen specific immune response is a Th1 type immune response in another embodiment.

The subject is a vertebrate animal. Preferably, the subject is a human. In some embodiments, however, the subject is a nonhuman vertebrate animal. In one embodiment, the vertebrate nonhuman animal is selected from the group consisting of a dog, cat, horse, cow, pig, sheep, goat, chicken, primate, fish, rat, and mouse.

Hematopoiesis may be treated by administering a CpG oligonucleotide to a subject having or at risk of developing a hematopoietic disorder. A hematopoietic disorder is a disorder involving a loss or decrease in numbers of one or more hematopoietic cells. Hematopoietic cells include erythrocytes, leukocytes and platelets.

Platelet counts may be increased in a subject having thrombocytopenia by administering to a subject having thrombocytopenia an oligonucleotide, having a sequence including at least the following formula:

5' X₁CGX₂ 3'

wherein the oligonucleotide includes at least 8 nucleotides wherein C and G are unmethylated and wherein X₁ and X₂ are nucleotides, in an amount effective to increase platelet counts in the subject. In one embodiment the thrombocytopenia is a non-chemotherapeutic induced thrombocytopenia.

A subject at risk of developing thrombocytopenia can be treated by administering to a subject at risk of developing thrombocytopenia an oligonucletoide, having a sequence including at least the following formula:

5' X₁CGX₂ 3'

wherein the oligonucleotide includes at least 8 nucleotides wherein C and G are unmethylated and wherein X₁ and X₂ are nucleotides, in an amount effective to prevent a decrease in platelet counts ordinarily expected under platclet-depleting conditions in The subject when the subject is exposed to platelet-depleting conditions.

In one embodiment the oligonucleotide is administered in an amount effective to increase platelet counts in the subject by at least 10,000 platelets per microliter. In another embodiment the oligonucleotide is administered in an amount effective to increase platelet counts in the subject by at least 20,000 platelets per microliter. In yet another embodiment the oligonucleotide is administered to the subject in an amount effective to increase the platelet counts in the subject by 100 percent.

The thrombocytopenia is any type of thrombocytopenia known in the art. In one embodiment the thrombocytopenia is a drug-induced thrombocytopenia. According to another embodiment the thrombocytopenia is due to an autoimmune disorder such as idiopathic thrombocytopenic purpura. In yet another embodiment the thrombocytopenia is a thrombocytopenia resulting from accidental radiation exposure. The thrombocytopenia is a thrombocytopenia resulting from therapeutic radiation exposure in yet another embodiment.

Anemia can be treated by administering to a subject having anemia an oligonucleotide, having a sequence including at least the following formula:

5' X₁CGX₂ 3'

wherein the oligonucleotide includes at least 8 nucleotides wherein C and G are unmethylated and wherein X₁ and X₂ are nucleotides, in an amount effective to induce erythropoiesis in the subject.

In one embodiment the oligonucleotide is administered in an amount effective to increase erythroblast counts in the subject by at least 10 percent. In another embodiment the oligonucleotide is administered in an amount effective to increase erythroblast counts in the subject by at least 20 percent. According to yet another embodiment the oligonucleotide is administered to the subject in an amount effective to increase erythroblast counts in the subject by 100 percent.

The anemia can be any type of anemia known in the art. In one embodiment the anemia is a drug-induced anemia. In another embodiment the anemia is selected from the group consisting of an immunohemolytic disorder, genetic disorders such as hemoglobinopathy and inherited hemolytic anemia; inadequate production despite adequate iron stores; chronic disease such as kidney failure; and chronic inflammatory disorder such as rheumatoid arthritis.

The subject having or at risk of having a hematopoietic disorder is a vertebrate animal. In a preferred embodiment, the subject is a human. In another preferred embodiment, the subject is a dog. In yet other embodiments, the subject is a nonhuman vertebrate animal selected from the group consisting of a cat, horse, cow, pig, sheep, goat, chicken, primate, fish, rat, and mouse.

In each of the aspects of the invention described above the CpG oligonucleotide is an oligonucleotide, having a sequence including at least the following formula:

5' X₁CGX₂ 3'.

In some embodiments the oligonucleotide is 8 to 100 nucleotides in length. In other embodiments the oligonucleotide is 8 to 30 nucleotides in length.

Preferably the oligonucleotide is a stabilized oligonucleotide. In one embodiment the oligonucleotide includes a phosphate backbone modification which is a phosphorothioate or phosphorodithioate modification. In a preferred embodiment the phosphate backbone modification occurs at the 5' end of the oligonucleotide. In another preferred embodiment the phosphate backbone modification occurs at the 3' end of the oligonucleotide.

According to a preferred embodiment of the invention the CpG oligonucleotide has a sequence including at least the following formula:

5' X₁X₂CGX₃X₄ 3'

wherein X₁X₂ are nucleotides selected from the group consisting of:GpT, GpG, GpA, ApA, ApT, ApG, CpT, CpA, CpG, TpA, TpT, and TpG; and X₃X₄ are nucleotides selected from the group consisting of: TpT, CpT, ApT, TpG, ApG, CpG, TpC, ApC, CpC, TpA, ApA, and CpA.

In another embodiment the CpG oligonucleotide has a sequence including at least the following formula:

5' TCNTX₁X₂CGX₃X₄ 3'

wherein X₁, X₂, X₃, and X₄ are nucleotides, N is a nucleic acid sequence composed of from about 0-25 nucleotides.

X₁X₂ are nucleotides selected from the group consisting of: GpT, GpG, GpA and ApA and X₃X₄ are nucleotides selected from the group consisting of: TpT and CpT in another embodiment.

Each of the limitations of the invention can encompass various embodiments of the invention. It is, therefore, anticipated that each of the limitations of the invention involving any one element or combinations of elements can be included in each aspect of the invention.

### Brief Description Of The Drawings

Figure 1 is a graph depicting the kinetics of increased spleen weight induced by CpG-ODN.
Figure 2 is a graph depicting the changes in phenotype of spleen cells after stimulation with CpG-ODN.
Figure 3 is a graph depicting the CpG-ODN induced changes in splenic cell number, number of splenic and BM GM-CFU.
Figure 4 is a graph depicting the dose titration of CpG-ODN.
Figure 5 is a graph depicting the increased number of BFU-E induced by CpG-ODN.
Figure 6 is a graph depicting the determination of spleen colony forming units of normal vs. CpG-ODN induced spleen cells (CFU-S Assay).
Figure 7 is a graph depicting the increased number of CM-CFU and enhanced CTL function after ODN-injection correlates with increased resistance towards lethal listeriosis in sublethally irradiated mice.
Figure 8 is a pair of graphs depicting spleen weights and spleen cell counts 5 days following 5 fluorouracil administration to mice, with or without coadministration of CpG-ODN.
Figure 9 is a graph depicting the splenic T lymphocyte counts on days 4, 7, and 10 following 5 fluorouracil administration to mice, with or without coadministration of CpG-ODN.
Figure 10 is a graph depicting the splenic B lymphocyte counts on days 4, 7, and 10 following 5 fluorouracil administration to mice, with or without coadministration of CpG-ODN.
Figure 11 is a graph depicting the white blood cell counts on days 4, 7, and 10 following 5 fluorouracil administration to mice, with or without coadministration of CpG-ODN.
Figure 12 is a graph depicting the red blood cell counts on days 4, 7, and 10 following 5 fluorouracil administration to mice, with or without coadministration of CpG-ODN.
Figure 13 is a graph depicting the platelet counts on days 4, 7, and 10 following 5 fluorouracil administration to mice, with or without coadministration of CpG-ODN.
Figure 14 is a graph depicting the induction of a cytotoxic T lymphocyte (CTL) response to specific antigen (ovalbumin, OVA) 10 days after administration of 5 fluorouracil, with or without coadministration of CpG-ODN.
Figure 15 is a pair of graphs depicting (left) the greater splenic population of dendritic cells 7 days following administration of CpG-ODN to mice, and (right) the larger outgrowth of dendritic cells from splenocytes in culture after CpG-ODN, compared to control treatment with phosphate buffered saline (PBS).
Figure 16 is a graph depicting the enhanced and extended induction of antibody in response to delayed antigen exposure in mice pretreated with CpG-ODN compared to PBS-pretreated mice.
Figure 17 is a graph depicting the kinetic profile of CTL induction in response to delayed antigen exposure in mice pretreated with CpG-ODN compared to PBS-pretreated mice.
Figure 18 is a graph depicting the kinetic profile of CTL induction in response to delayed antigen exposure in mice pretreated with CpG-ODN compared to PBS-pretreated mice.

### Detailed Description Of The Invention

Hematopoiesis refers to the generation of blood cells. The process of generating new blood cells is controlled through the complex interaction of immune factors such as interleukin and CSF. Using these factors the immune system is able to regulate the levels of each of the cellular components in blood in response to physiological changes.

Erythrocytes, leukocytes and platelets are the essential cells of the human hematopoietic system. The primary function of erythrocytes, also known as red blood cells, is to transport hemoglobin, which in turn carries oxygen from the lungs to tissues. Oxygenated hemoglobin gives the erythrocytes a red color. Leukocytes, also referred to as myeloid cells, are a heterogenous group of cells that mediate immune responses and which include granulocytes, including eosinophils, basophils, and neutrophils; monocytes; and T and B lymphocytes. These cells are found predominately in the blood, bone marrow, lymphoid organs and epithelium. Leukocytes are referred to as white blood cells because of a lack of natural pigment which gives the cells a whitish or transparent appearance. Platelets play a role in hemostasis, or the regulation of bleeding.

Many factors are capable of influencing the hematopoietic system causing deficiencies or malignancies of particular types of blood cells. Disorders of the hematopoietic system vary depending on the factor causing the disorder as well as the cell type affected.

It has been discovered that CpG containing oligonucleotides can regulate hematopoieses to inhibit loss of blood cells in response to physiological disorders caused by genetic abnormalities, environmental factors or medical therapies. It has also been discovered that hematopoiesis can be manipulated using CpG oligonucleotides to induce immune system remodeling in order to stimulate an antigen specific immune response.

The process of immune system remodeling is based on the generation of immune cells in response to a stimulus in preparation for generating a strong antigen specific immune response. The stimulus is a CpG oligonucleotide. It has been discovered that when a CpG oligonucleotide is administered to a subject, after an initial delay, the immune system of the subject undergoes a repopulation event to produce a population of immune cells which are primed to generate an antigen specific response. This renewed population of cells remains in the body for an extensive period of time. When the primed cells encounter antigen the cells respond to the antigen by producing an antigen specific immune response. In fact the antigen is capable of producing an immune specific response even in the absence of an adjuvant Ordinarily the administration of antigen in the absence of an adjuvant would not produce a specific immune response.

Although Applicants are not bound by a particular mechanism it is believed that when CpG is administered to a subject, CpG activates the circulating immune cells, causing them to mature into mature active immune cells. If CpG is administered at the same time or slightly before or after an antigen then the circulating immune cells will likely contact the antigen and develop a specific immune response against that antigen. After a period of about 24 hours, the circulating immune cells will no longer be capable of mounting an antigen specific immune response because the circulating cells have already been activated and matured. It has been found according to the invention, however, that approximately two days after the administration of CpG the subject's immune system has been repopulated with immune cells which are capable of being matured and activated in response to antigen. If antigen is administered at least two days after CpG administration then the immune system is capable of generating an antigen specific immune response, which may be even of a greater magnitude than the immune response which is generated in response to antigen administration at the same time as CpG. Two days after CpG administration the remodeled immune system encompasses a population of cells which are capable of responding to antigen. It has been demonstrated according to the invention that this population of cells is capable of responding to antigen for long periods of time. For instance, administration of an antigen at time periods of greater than 30 days after the CpG administration can still produce an antigen specific response.

The invention encompasses the use of CpG oligonucleotides for the manufacture of a medicament for generating an antigen specific immune response by administering CpG to induce immune remodeling to prepare for exposure to an antigen. The subject may be intentionally exposed to the antigen two days or more after being administered CpG in order to develop an immunity to a specific antigen. The subject may also be exposed passively to an antigen, causing a specific immune response to develop against an antigen to which the subject is exposed from the environment. Thus the immune system can be manipulated to be in an active state ready to respond to invading substances, such as pathogens.

The use of CpG oligonucleotides for the manufacture of a medicament for inducing immune system remodeling of the invention is a use of the CpG oligonucleotides for the manufacture of a medicament for inducing an antigen-specific immune response, by administering to a subject an oligonucleotide, having a sequence including at least the following formula:

5' X₁CGX₂ 3'

wherein the oligonucleotide includes at least 8 nucleotides wherein C and G are unmethylated and wherein X₁ and X₂ are nucleotides, and exposing the subject to an antigen at least 3 days alter the oligonucleotide is administered to the subject to produce an antigen-specific immune response.

An "antigen" as used herein is a molecule capable of provoking an immune response. Antigens include but are not limited to cells, cell extracts, polysaccharides, polysaccharide conjugates, lipids, glycolipids, carbohydrate, peptides, proteins, viruses, and viral extracts. The term antigen broadly includes any type of molecule which is recognized by a host immune system as being foreign. Antigens include but are not limited to cancer antigens, microbial antigens, and allergens.

The CpG oligonucleotides of the invention are useful for treating cancer by stimulating an antigen specific immune response against an antigen. A "cancer antigen" as used herein is a compound, such as a peptide, associated with a tumor or cancer cell surface and which is capable of provoking an immune response when expressed on the surface of an antigen presenting cell in the context of an MHC molecule. Cancer antigens can be prepared from cancer cells either by preparing crude extracts of cancer cells, for example, as described in Cohen, et al., 1994, *Cancer Research*, 54:1055, by partially purifying the antigens, by recombinant technology, or by de novo synthesis of known antigens. Cancer antigens include antigens that are recombinately an immunogenic portion of or a whole tumor or cancer. Such antigens can be isolated or prepared recombinady or by any other means known in the art. Cancers or tumors include but are not limited to biliary tract cancer, brain cancer, breast cancer; cervical cancer; choriocarcinoma; colon cancer, endometrial cancer; esophogeal cancer; gastric cancer; intraepithelial neoplasms; lymphomas; liver cancer; lung cancer (e.g. small cell and non-small cell); melanoma; neuroblastomas; oral cancer; ovarian cancer; pancreas cancer; prostate cancer; rectal cancer; sarcomas; skin cancer; testicular cancer; thyroid cancer; and renal cancer, as well as other carcinomas and sarcomas.

The CpG oligonucleotides of the invention are also useful for treating infectious diseases. An infectious disease, as used herein, is a disease arising from the presence of a foreign microorganism in the body. CpG is used to stimulate an antigen specific immune response which can activate a T or B cell response against an antigen of the microorganism. The the Cph oligonucleotides are used in the same way as described above for the tumor except that the antigen is specific for a microorganism using a microbial antigen. A "microbial antigen" as used herein is an antigen of a microorganism and includes but is not limited to infectious virus, infectious bacteria, and infectious fungi. Such antigens include the intact microorganism as well as natural isolates and fragments or derivatives thereof and also synthetic compounds which are identical to or similar to natural microorganism antigens. A compound is similar to a natural microorganism antigen if it induces an immune response (humoral and/or cellular) to a natural microorganism antigen. Such antigens are used routinely in the art and are well known to those of ordinary skill in the art.

Examples of infectious virus include but are not limited to: *Retroviridae* (e.g. human immunodeficiency viruses, such as HIV-1 (also referred to as HTLV-III, LAV or HTLV-III/LAV, or HIV-III; and other isolates, such as HIV-LP; *Picornaviridae* (e.g. polio viruses, hepatitis A virus; enteroviruses, human Coxsackie viruses, rhinoviruses, echoviruses); *Calciviridae* (e.g. strains that cause gastroenteritis); *Togaviridae* (e.g. equine encephalitis viruses, rubella viruses); Flaviviridae (e.g. dengue viruses, encephalitis viruses, yellow fever viruses); *Coronaviridae* (e.g. coronaviruses); *Rhabdoviridae* (e.g. vesicular stomatitis viruses, rabies viruses); *Filoviridae* (e.g. ebola viruses); *Paramyxoviridae* (e.g. parainfluenza viruses, mumps virus, measles virus, respiratory syncytial virus); *Orthomyxoviridae* (e.g. influenza viruses); *Bunyaviridae* (e.g. Hantaan viruses, bunya viruses, phleboviruses and Nairo viruses); Arena viridae (hemorrhagic fever viruses); *Reoviridae* (e.g. reoviruses, orbiviurses and rotaviruses); *Birnaviridae; Hepadnaviridae* (Hepatitis B virus); *Parvovirida* (parvoviruses); *Papovaviridae* (papilloma viruses, polyoma viruses); *Adenoviridae* (most adenoviruses); *Herpesviridae* (herpes simplex virus (HSV) 1 and 2, varicella zoster virus, cytomegalovirus (CMV), herpes virus; *Poxviridae* (variola viruses, vaccinia viruses, pox viruses); and *Iridoviridae* (e.g. African swine fever virus); and unclassified viruses (e.g. the etiological agents of Spongiform encephalopathies, the agent of delta hepatitis (thought to be a defective satellite of hepatitis B virus), the agents of non-A, non-B hepatitis (class 1 = internally transmitted; class 2 = parenterally transmitted (i.e. Hepatitis C); Norwalk and related viruses, and astroviruses).

Examples of infectious bacteria include but are not limited to: *Helicobacter pyloris*, *Borrelia burgdorferi, Legionella pneumophilia, Mycobacteria sps* (e.g. *M. tuberculosis, M. avium, M. intracellulare, M. kansaii, M. gordonae*), *Staphylococcus aureus, Neisseria gonorrhoeae, Neisseria meningilidis, Listeria monocytogenes, Streptococcus pyogenes* (Group A Streptococcus), *Streptococcus agalactiae* (Group B Streptococcus), *Streptococcus* (viridans group), *Streptococcus faecalis, Streptococcus bovis, Streptococcus* (anaerobic sps.), *Streptococcus pneumoniae*, pathogenic *Campylobacter sp., Enterococcus sp., Haemophilus influenzae, Bacillus anthracis, corynebacterium diphtheriae, corynebacterium sp., Erysipelothrix rhusiopathiae, Clostridium perfringens, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema perlenue, Leptospira, Rickettsia,* and *Actinomyces israelli.*

Examples of infectious fungi include: *Cryptococcus neoformans, Histoplasma capsulatum, Coccidioides immitis, Blastomyces dermatitidis, Chlamydia trachomatis, Candida albicans*. Other infectious organisms (i.e., protists) include: *Plasmodium* such as *Plasmodium falciparum, Plasmodium malariae, Plasmodium ovale*, and *Plasmodium* vivax and *Toxoplasma gondii.*

Other medically relevant microorganisms have been descried extensively in the literature, e.g., see C.G.A Thomas, *Medical Microbiology,* Bailliere Tindall, Great Britain 1983, the entire contents of which is hereby incorporated by reference.

The Cph oligonucleotides of the invention are also useful for treating allergic diseases. The methods are accomplished in the same way as described above for the tumor immunothcrapy and treatment of infectious diseases except that the antigen is specific for an allergen. Currently, allergic diseases are generally treated by the injection of small doses of antigen followed by subsequent increasing dosage of antigen. It is believed that this procedure produces a memory immune response to prevent further allergic reactions. These methods, however, are associated with the risk of side effects such as an allergic response. The methods of the invention avoid these problems.

An "allergen" refers to a substance (antigen) that can induce an allergic or asthmatic response in a susceptible subject. The list of allergens is enormous and can include pollens, insect venoms, animal dander dust, fungal spores and drugs (e.g. penicillin). Examples of natural, animal and plant allergens include but are not limited to proteins specific to the following genuses: *Canine (Canis familiaris)*; *Dermatophagoides* (e.g. *Dermatophagoides* *farinae*); *Felis (Felis domesticus); Ambrosia (Ambrosia artemiisfolia*; *Lolium* (e.g. *Lolium perenne* or *Lolium multiflorum*); *Cryptomeria* (*Cryptomeria japonica*); *Alternaria (Alternaria alternata*); *Alder*; *Alnus (Alnus gultinoasa*); *Betula* (*Betula verrucosa*); *Quercus (Quercus alba*); *Olea (Olea europa*); *Artemisia* (*Artemisia vulgaris*); *Plantago* (e.g. *Plantago lanceolata*); *Parietaria* (e.g. *Parietaria officinalis* or *Parietaria judaica*); *Blattella* (e.g. *Blattella germanica*); *Apis (e.g. Apis multiflorum*); *Cupressus* (e.g. *Cupressus sempervirens, Cupressus arizonica* and *Cupressus macrocarpa*); *Juniperus* (e.g. *Juniperus sabinoides, Juniperus virginiana, Juniperus communis* and *Juniperus ashei*); *Thuya* (e.g. *Thuya orientalis*); *Chamaecyparis* (e.g. *Chamaecyparis obtusa*); *Periplaneta* (e.g. *Periplaneta americana*); *Agropyron* (e.g. *Agropyron repens*)*; Secale* (e.g. *Secale cereale*); *Triticum* (e.g. *Triticum aestivum*); *Dactylis* (e.g. *Dactylis glomerata*)*; Festuca* (e.g. *Festuca elatior*); *Poa* (e.g. *Poa pratensis* or *Poa compressa*); *Avena* (e.g. *Avena sativa*); *Holcus* (e.g. *Holcus lanatus*); *Anthoxanthum (e.g. Anthoxanthum odoratum*); *Arrhenatherum* (e.g. *Arrhenatherum elatius*); *Agrostis* (e.g. *Agrostis alba*); *Phleum* (e.g. *Phleum pratense*); *Phalaris* (e.g. *Phalaris arundinacea*); *Paspalum* (e.g. *Paspalum notatum*); *Sorghum* (e.g. *Sorghum halepensis*); and *Bromus* (e.g. *Bromus inermis*).

An "allergy" refers to acquired hypersensitivity to a substance (allergen). Allergic conditions include but are not limited to eczema, allergic rhinitis or coryza, hay fever, bronchial asthma, urticaria (hives) and food allergies, and other atopic conditions. A subject having an allergic reaction is a subject that has or is at risk of developing an allergy.

Allergies are generally caused by IgE antibody generation against harmless allergens. The cytokines that are induced by unmethylated CpG oligonucleotides are predominantly of a class called "Th1" which is most marked by a cellular immune response and is associated with IL-12 and IFN-γ. The other major type of immune response is termed as Th2 immune response, which is associated with more of an antibody immune response and with the production of IL-4, IL-5 and IL-10. In general, it appears that allergic diseases are mediated by Th2 type immune responses and autoimmune diseases by Th1 immune response. Based on the ability of the CpG oligonucleotides to shift the immune response in a subject from a Th2 (which is associated with production of IgE antibodies and allergy) to a Th1 response (which is protective against allergic reactions), an effective dose of a CpG oligonucleotide can be administered to a subject to treat or prevent an allergy.

CpG oligonucleotides may also have significant therapeutic utility in the treatment of asthma. Th2 cytokines, especially IL-4 and IL-5 are elevated in the airways of asthmatic subjects. These cytokines, especially IL-4 and IL-5 are elevated in the airways of asthmatic subjects. These cytokines promote important aspects of the asthmatic inflammatory response, including IgE isotope switching, eosinophil chemotaxis and activation and mast cell growth. Th1 cytokines, especially IFN-γ and IL-12, can suppress the formation of Th2 clones and production of Th2 cytokines. "Asthma" refers to a disorder of the respiratory system characterized by inflammation, narrowing of the airways and increased reactivity of the airways to inhaled agents. Asthma is frequently, although not exclusively associated with atopic or allergic symptoms.

It is believed that the antigen is taken up by an antigen presenting cell (APC) such as a dendritic cell in the repopulated immune system. The APC then processes and presents the antigen on its cell surface to produce a cytotoxic T lymphocyte (CTL) response by interacting with T lymphocytes or an antibody response by interacting with B lymphocytes. Preferably, the antigen is exposed to the immune cells 48 hours after adding CpG. In a more preferred embodiment, the subject's immune cells are exposed to the antigen 60 hours after the CpG. In other embodiments the subject's immune cells are exposed to the antigen at least 3, 4, 5, 6, 7, 8, 9, 10, 11, 12, 13, 14, 15, 16, 17,18, 19, 20, 21, 22, 23, 24, 25, 26, 27, 28, 29, 30, 31, 32, 33, 34, 35, 36, 37, 38, 39, or 40 days after the CpG.

A "subject" shall mean a human or vertebrate animal including but not limited to a dog, cat, horse, cow, pig, sheep, goat, chicken, primate, e.g., monkey, fish (aquaculture species), e.g. salmon, rat, and mouse.

Although many of the disorders described above relate to human disorders, the invention is also useful for treating other nonhuman vertebrates. Nonhuman vertebrates are also capable of developing cancer, infections, allergies, and asthma. For instance, in addition to the treatment of infectious human diseases, the CpG oligonucleotides of the invention are useful for treating infections of animals. As used herein, the term "treat" or "treating" when used with respect to an infectious disease refers to a prophylactic treatment which increases the resistance of a subject to infection with a pathogen or, in other words, decreases the likelihood that the subject will become infected with the pathogen. Many vaccines for the treatment of non-human vertebrates are disclosed in Bennett, K. *Compendium of Veterinary Products,* 3rd ed. North American Compendiums, Inc., 1995.

Thus the present invention contemplates the use of CpG oligonucleotides to induce an antigen specific immune response in human and non-human animals. As discussed above, antigens include infectious microbes such as virus, bacteria and fungi and fragments thereof, derived from natural sources or synthetically. Infectious virus of both human and non-human vertebrates, include retroviruses, RNA viruses and DNA viruses. This group of retroviruses includes both simple retroviruses and complex retroviruses. The simple retroviruses include the subgroups of B-type retroviruses, C-type retroviruses and D-type retroviruses. An example of a B-type retrovirus is mouse mammary tumor virus (MMTV). The C-type retroviruses include subgroups C-type group A (including Rous sarcoma virus (RSV), avian leukemia virus (ALV), and avian myeloblastosis virus (AMV)) and C-type group B (including murine leukemia virus (MLV), feline leukemia virus (FeLV), murine sarcoma virus (MSV), gibbon ape leukemia virus (GALV), spleen necrosis virus (SNV), reticuloendotheliosis virus (RV) and simian sarcoma virus (SSV)). The D-type retroviruses include Mason-Pfizer monkey virus (MPMV) and simian retrovirus type 1 (SRV-1). The complex retroviruses include the subgroups of lentiviruses, T-cell leukemia viruses and the foamy viruses. Lentiviruses include HIV-1, but also include HIV-2, SIV, Visna virus, feline immunodeficiency virus (FIV), and equine infectious anemia virus (EIAV). The T-cell leukemia viruses include HTLV-I, HTLV-II, simian T-cell leukemia virus (STLV), and bovine leukemia virus (BLV). The foamy viruses include human foamy virus (HFV), simian foamy virus (SFV) and bovine foamy virus (BFV). The foregoing list is illustrative, and is not intended to be limiting.

Examples of other RNA viruses that are antigens in vertebrate animals include, but are not limited to, the following: members of the family Reoviridae, including the genus Orthoreovirus (multiple serotypes of both mammalian and avian retroviruses), the genus Orbivirus (Bluetongue virus, Eugenangee virus, Kemerovo virus, African horse sickness virus, and Colorado Tick Fever virus), the genus Rotavirus (human rotavirus, Nebraska calf diarrhea virus, murine rotavirus, simian rotavirus, bovine or ovine rotavirus, avian rotavirus); the family Picomaviridae, including the genus Enterovirus (poliovirus, Coxsackie virus A and B, enteric cytopathic human orphan (ECHO) viruses, hepatitis A virus, Simian enteroviruses, Murine encephalomyelitis (ME) viruses, Poliovirus muris, Bovine enteroviruses, Porcine enteroviruses , the genus Cardiovirus (Encephalomyocarditis virus (EMC), Mengovirus), the genus Rhinovirus (Human rhinoviruses including at least 113 subtypes; other rhinoviruses), the genus Apthovirus (Foot and Mouth disease (FMDV); the family Calciviridae, including Vesicular exanthema of swine virus, San Miguel sea lion virus, Feline picornavirus and Norwalk virus; the family Togaviridae, including the genus Alphavirus (Eastern equine encephalitis virus, Semliki forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus, Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1, Sendai virus, Hemadsorption virus, Parainfluenza viruses types 2 to 5, Newcastle Disease Virus, Mumps virus), the genus Morbillivirus (Measles virus, subacute sclerosing panencephalitis virus, distemper virus, Rinderpest virus), the genus Pneumovirus (respiratory syncytial virus (RSV), Bovine respiratory syncytial virus and Pneumonia virus of mice); the family Rhabdoviridae, including the genus Vesiculovirus (VSV), Chandipura virus, Flanders-Hart Park virus), the genus Lyssavirus (Rabies virus), fish Rhabdoviruses, and two probable Rhabdoviruses (Marburg virus and Ebola virus); the family Arenaviridae, including Lymphocytic choriomeningitis virus (LCM), Tacaribe virus complex, and Lassa virus;-the family Coronoaviridae, including Infectious Bronchitis Virus (IBV), Mouse Hepatitis virus, Human enteric corona virus, and Feline infectious peritonitis (Feline coronavirus).

Illustrative DNA viruses that are antigens in vertebrate animals include, but are not limited to: the family Poxviridae, including the genus Orthopoxvirus (Variola major, Variola minor, Monkey pox Vaccinia, Cowpox, Buffalopox, Rabbitpox, Ectromelia), the genus Leporipoxvirus (Myxoma, Fibroma), the genus Avipoxvirus (Fowlpox, other avian poxvirus), the genus Capripoxvirus (sheeppox, goatpox), the genus Suipoxvirus (Swinepox), the genus Parapoxvirus (contagious postular dermatitis virus, pseudocowpox, bovine papular stomatitis virus); the family Iridoviridae (African swine fever virus, Frog viruses 2 and 3, Lymphocystis virus of fish); the family Herpesviridae, including the alpha-Herpesviruses (Herpes Simplex Types 1 and 2, Varicella-Zoster, Equine abortion virus, Equine herpes virus 2 and 3, pseudorabies virus, infectious bovine keratoconjunctivitis virus, infectious bovine rhinotracheitis virus, feline rhinotracheitis virus, infectious laryngotracheitis virus) the Beta-herpesviruses (Human cytomegalovirus and cytomegaloviruses of swine, monkeys and rodents); the gamma-herpesviruses (Epstein-Barr virus (EBV), Marek's disease virus, Herpes saimiri, Herpesvirus ateles, Herpesvirus sylvilagus, guinea pig herpes virus, Lucke tumor virus); the family Adenoviridae, including the genus Mastadenovirus (Human subgroups A,B,C,D,E and ungrouped; simian adenoviruses (at least 23 serotypes), infectious canine hepatitis, and adenoviruses of cattle, pigs, sheep, frogs and many other species, the genus Aviadenovirus (Avian adenoviruses); and non-cultivatable adenoviruses; the family Papoviridae, including the genus Papillomavirus (Human papilloma viruses, bovine papilloma viruses, Shope rabbit papilloma virus, and various pathogenic papilloma viruses of other species), the genus Polyomavirus (polyomavirus, Simian vacuolating agent (SV-40), Rabbit vacuolating agent (RKV), K virus, BK virus, JC virus, and other primate polyoma viruses such as Lymphotrophic papilloma virus); the family Parvoviridae including the genus Adeno-associated viruses, the genus Parvovirus (Feline panleukopenia virus, bovine parvovirus, canine parvovirus, Aleutian mink disease virus, etc). Finally, DNA viruses may include viruses which do not fit into the above families such as Kuru and Creutzfeldt-Jacob disease viruses and chronic infectious neuropathic agents (CHINA virus).

Both gram negative and gram positive bacteria serve as antigens in vertebrate animals. Such gram positive bacteria include, but are not limited to those bacteria discussed above as well as *Pasteurella* species, *Staphylococci* species, and *Streptococcus* species. Gram negative bacteria include, but are not limited to, *Escherichia coli, Pseudomonas* species, and *Salmonella* species. *Salmonella* enteritidis is an important pathogen in the commercial layer industry, as ovarian colonization of layers may result in maternally transmitted *Salmonella* in table eggs.

In addition to the use of CpG oligonucleotides to induce an antigen specific immune responses in humans, the CpG oligonucleotides of the preferred embodiments are particularly well suited for treatment of birds such as hens, chickens, turkeys, ducks, geese, quail, and pheasant. Birds are prime targets for many types of infections including AIDS or immunodeficiency virus.

Hatching birds are exposed to pathogenic microorganisms shortly after birth. Although these birds are initially protected against pathogens by maternal derived antibodies, this protection is only temporary, and the bird's own immature immune system must begin to protect the bird against the pathogens. It is often desirable to prevent infection in young birds when they are most susceptible. It is also desirable to prevent against infection in older birds, especially when the birds are housed in closed quarters, leading to the rapid spread of disease. Thus, it is desirable to administer the CpG oligonucleotide of the invention to birds to enhance an antigen-specific immune response when antigen is present.

An example of a common infection in chickens is chicken infectious anemia virus (CIAV). CIAV was first isolated in Japan ia 1979 during an investigation of a Marek's disease vaccination break (Yuasa et al., 1979, Avian Dis. 23:366-385). Since that time, CIAV has been detected in commercial poultry in all major poultry producing countries (van Bulow et al., 1991, pp.690-699) in Diseases of Poultry, 9th edition, Iowa State University Press).

CIAV infection results in a clinical disease, characterized by anemia, hemorrhage and immunosuppression, in young susceptible chickens. Atrophy of the thymus and of the bone marrow and consistent lesions of CIAV-infected chickens are also characteristic of CIAV infection. Lymphocyte depletion in the thymus, and occasionally in the bursa of Fabricius, results in immunosuppression and increased susceptibility to secondary viral, bacterial, or fungal infections which then complicate the course of the disease. The immunosuppression may cause aggravated disease after infection with one or more of Marek's disease virus (MDV), infectious bursal disease virus, reticuloendotheliosis virus, adenovirus, or reovirus. It has been reported that pathogenesis of MDV is enhanced by CIAV (DeBoer et al., 1989, p. 28 In Proceedings of the 38th Western Poultry Diseases Conference, Tempe, Ariz.). Further, it has been reported that CIAV aggravates the signs of infectious bursal disease (Rosenberger et al., 1989, Avian Dis. 33:707-713). Chickens develop an age resistance to experimentally induced disease due to CAA. This is essentially complete by the age of 2 weeks, but older birds are still susceptible to infection (Yuasa, N. et al., 1979 supra; Yuasa, N. et al., Avian Diseases 24, 202-209, 1980). However, if chickens are dually infected with CAA and an immunosuppressive agent (IBDV, MDV etc.) age resistance against the disease is delayed (Yuasa, N. et al., 1979 and 1980 supra; Bulow von V. et al., J. Veterinary Medicine 33, 93-116, 1986). Characteristics of CIAV that may potentiate disease transmission include high resistance to environmental inactivation and some common disinfectants. The economic impact of CIAV infection on the poultry industry is clear from the fact that 10% to 30% of infected birds in disease outbreaks die.

Vaccination of birds, like other vertebrate animals can be performed at any age. Normally, vaccinations are performed at up to 12 weeks of age for a live microorganism and between 14-18 weeks for an inactivated microorganism or other type of vaccine. For in ovo vaccination, vaccination can be performed in the last quarter of embryo development. The vaccine may be administered subcutaneously, by spray, orally, intraocularly, intratracheally, nasally, in ovo or by other methods described herein. Thus, the CpG oligonucleotide of the invention can be administered to birds and other non-human vertebrates using routine vaccination schedules and the antigen is administered after an appropriate time period as described herein.

Cattle and livestock are also susceptible to infection. Disease which affect these animals can produce severe economic losses, especially amongst cattle. The methods of the invention can be used to protect against infection in livestock, such as cows, horses, pigs, sheep, and goats.

Cows can be infected by bovine viruses. Bovine viral diarrhea virus (BVDV) is a small enveloped positive-stranded RNA virus and is classified, along with hog cholera virus (HOCV) and sheep border disease virus (BDV), in the pestivirus genus. Although, Pestiviruses were previously classified in the Togaviridae family, some studies have suggested their reclassification within the Flaviviridae family along with the flavivirus and hepatitis C virus (HCV) groups (Francki, et al., 1991).

BVDV, which is an important pathogen of cattle can be distinguished, based on cell culture analysis, into cytopathogenic (CP) and noncytopathogenic (NCP) biotypes. The NCP biotype is more widespread although both biotypes can be found in cattle. If a pregnant cow becomes infected with an NCP strain, the cow can give birth to a persistently infected and specifically immunotolerant calf that will spread virus during its lifetime. The persistently infectected cattle can succumb to mucosal disease and both biotypes can then be isolated from the animal. Clinical manifestations can include abortion, teratogenesis, and respiratory problems, mucosal disease and mild diarrhea. In addition, severe thrombocytopenia, associated with herd epidemics, that may result in the death of the animal has been described and strains associated with this disease seem more virulent than the classical BVDVs.

Equine herpesviruses (EHV) comprise a group of antigenically distinct biological agents which cause a variety of infections in horses ranging from subclinical to fatal disease. These include Equine herpesvirus-1 (EHV-1), a ubiquitous pathogen in horses. EHV-1 is associated with epidemics of abortion, respiratory tract disease, and central nervous system disorders. Primary infection of upper respiratory tract of young horses results in a febrile illness which lasts for 8 to 10 days. Immunologically experienced mares may be reinfected via the respiratory tract without disease becoming apparent, so that abortion usually occurs without warning. The neurological syndrome is associated with respiratory disease or abortion and can affect animals of either sex at any age, leading to incoordination, weakness and posterior paralysis (Telford, E. A. R. et al., Virology 189, 304-316, 1992). Other EHV's include EHV-2, or equine cytomegalovirus, EHV-3, equine coital exanthema virus, and EHV-4, previously classified as EHV-1 subtype 2.

Sheep and goats can be infected by a variety of dangerous microorganisms including visna-maedi.

Primates such as monkeys, apes and macaques can be infected by simian immunodeficiency virus. Inactivated cell-virus and cell-free whole simian immunodeficiency vaccines have been reported to afford protection in macaques (Stott et aL (1990) Lancet 36:1538-1541; Desrosiers et al. PNAS USA (1989) 86:6353-6357; Murphey-Corb et al. (1989) Science 246:1293-1297; and Carlson et al. (1990) AIDS Res. Human Retroviruses 6:1239-1246). A recombinant HIV gp120 vaccine has been reported to afford protection in chimpanzees (Berman et al. (1990) Nature 345:622-625).

Cats, both domestic and wild, are susceptible to infection with a variety of microorganisms. For instance, feline infectious peritonitis is a disease which occurs in both domestic and wild cats, such as lions, leopards, cheetahs, and jaguars. When it is desirable to prevent infection with this and other types of pathogenic organisms in cats, the CpG oligonucleotides of the invention can be used to vaccinate cats to prevent them against infection.

Domestic cats may become infected with several retroviruses, including but not limited to feline leukemia virus (FeLV), feline sarcoma virus (FeSV), endogenous type C oncomavirus (RD-114), and feline syncytia-forming virus (FeSFV). Of these, FeLV is the most significant pathogen, causing diverse symptoms, including lymphoreticular and myeloid neoplasms, anemias, immune mediated disorders, and an immunodeficiency syndrome which is similar to human acquired immune deficiency syndrome (AIDS). Recently, a particular replication-defective FeLV mutant, designated FeLV-AIDS, has been more particularly associated with immunosuppressive properties.

The discovery of feline T-lymphotropic lentivirus (also referred to as feline immunodeficiency) was first reported in Pedersen et al. (1987) Science 235:790-793. Characteristics of FIV have been reported in Yamamoto et aL (1988) Leukemia, December Supplement 2:204S-215S; Yamamoto et aL (1988) Am. J. Vet. Res. 49:1246-1258; and Ackley et aL (1990) J. Virol. 64:5652-5655. Cloning and sequence analysis of FIV have been reported in Olmsted et al. (1989) Proc. NatL Acad. Sci. USA 86:2448-2452 and 86:4355-4360.

Feline infectious peritonitis (FIP) is a sporadic disease occurring unpredictably in domestic and wild Felidae. While FIP is primarily a disease of domestic cats, it has been diagnosed in lions, mountain lions, leopards, cheetahs, and the jaguar. Smaller wild cats that have been afflicted with FIP include the lynx and caracal, sand cat, and pallas cat. In domestic cats, the disease occurs predominantly in young animals, although cats of all ages are susceptible. A peak incidence occurs between 6 and 12 months of age. A decline in incidence is noted from 5 to 13 years of age, followed by an increased incidence in cats 14 to 15 years old.

Viral and bacterial diseases in fin-fish, shellfish or other aquatic life forms pose a serious problem for the aquaculture industry. Owing to the high density of animals in the hatchery tanks or enclosed marine farming areas, infectious diseases may eradicate a large proportion of the stock in, for example, a fin-fish, shellfish, or other aquatic life forms facility. Prevention of disease is a more desired remedy to these threats to fish than intervention once the disease is in progress. Vaccination of fish is the only preventative method which may offer long-term protection through immunity. Nucleic acid based vaccinations are described in US Patent No. 5,780,448 issued to Davis.

The fish immune system has many features similar to the mammalian immune system, such as the presence of B cells, T cells, lymphokines, complement, and immunoglobulins. Fish have lymphocyte subclasses with roles that appear similar in many respects to those of the B and T cells of mammals. Vaccines can be administered orally or by immersion or injection.

Aquaculture species include but are not limited to fin-fish, shellfish, and other aquatic animals. Fin-fish include all vertebrate fish, which may be bony or cartilaginous fish, such as, for example, salmonids, carp, catfish, yellowtail, seabream, and seabass. Salmonids are a family of fin-fish which include trout (including rainbow trout), salmon, and Arctic char. Examples of shellfish include, but are not limited to, clams, lobster, shrimp, crab, and oysters. Other cultured aquatic animals include, but are not limited to eels, squid, and octopi.

Polypeptides of viral aquaculture pathogens include but are not limited to glycoprotein (G) or nucleoprotein (N) of viral hemorrhagic septicemia virus (VHSV); G or N proteins of infectious hematopoietic necrosis virus (IHNV); VP1, VP2, VP3 or N structural proteins of infectious pancreatic necrosis virus (IPNV); G protein of spring viremia of carp (SVC); and a membrane-associated protein, tegumin or capsid protein or glycoprotein of channel catfish virus (CCV).

Polypeptides of bacterial pathogens include but are not limited to an iron-regulated outer membrane protein, (IROMP), an outer membrane protein (OMP), and an A-protein of Aeromonis salmonicida which causes furunculosis, p57 protein of Renibacterium salmoninarum which causes bacterial kidney disease (BKD), major surface associated antigen (msa), a surface expressed cytotoxin (mpr), a surface expressed hemolysin (ish), and a flagellar antigen of Yersiniosis; an extracellular protein (ECP), an iron-regulated outer membrane protein (IROMP), and a structural protein of Pasteurellosis; an OMP and a flagellar protein of Vibrosis anguillarum and V. ordalii; a flagellar protein, an OMP protein aroA, and purA of Edwardsiellosis ictaluri and E. tarda; and surface antigen of Ichthyophthirius; and a structural and regulatory protein of Cytophaga columnari; and a structural and regulatory protein of Rickettsia.

Polypeptides of a parasitic pathogen include but are not limited to the surface antigens of Ichthyophthirius.

The subject is exposed to the antigen. As used herein, the term "exposed to" refers to either the active step of contacting the subject with an antigen or the passive exposure of the subject to the antigen *in vivo*. Methods for the active exposure of a subject to an antigen are well-known in the art. In general, an antigen is administered directly to the subject by any means such as intravenous, intramuscular, oral, transdermal, mucosal, intranasal, intratracheal, or subcutaneous administration. The antigen can be administered systemically or locally. Methods for administering the antigen and the CpG are described in more detail below. A subject is passively exposed to an antigen if an antigen becomes available for exposure to the immune cells in the body. A subject may be passively exposed to an antigen, for instance, by entry of a foreign pathogen into the body or by the development of a tumor cell expressing a foreign antigen on its surface. When a subject is passively exposed to an antigen it is preferred that the CpG oligonucleotide is an oligonucleotide of 8-100 nucleotides in length and/or has a phosphate modified backbone. It is also preferred that the oligonucleotide is not administered in conjunction with a first antigen.

The uses of the CpG oligonucleotides in which a subject is passively exposed to an antigen can be particularly dependent on timing of CpG oligonucleotide administration. For instance, in a subject at risk of developing a cancer or an allergic or asthmatic response, the subject may be administered the CpG oligonucleotide on a regular basis when that risk is greatest, i.e., during allergy season or after exposure to a cancer causing agent.

Additionally the CpG oligonucleotide may be administered to travelers before they travel to foreign lands where they are at risk of exposure to infectious agents. Likewise the CpG oligonucleotide may be administered to soldiers or civilians at risk of exposure to biowarfare.

Thus, the invention contemplates scheduled administration of CpG oligonucleotides. The oligonucleotides may be administered to a subject on a weekly or monthly basis. When a subject is at risk of exposure to an antigen or antigens the CpG may be administered on a regular basis to maintain a primed immune system that will recognize the antigen immediately upon exposure and produce an antigen specific immune reponse. A subject at risk of exposure to an antigen is any subject who has a high probability of being exposed to an antigen and of developing an immune response to the antigen. If the antigen is an allergen and the subject develops allergic responses to that particular antigen and the subject is exposed to the antigen, i.e., during pollen season, then that subject is at risk of exposure to the antigen. If such a subject is administered a CpG oligonucleotide on a monthly basis then they will maintain a primed set of immune cells which are capable of recognizing and reacting to an antigen.

A subject at risk of developing a cancer can also be treated using the CpG oligonucleotides of the invention, by passive or active exposure to antigen following CpG. A subject at risk of developing a cancer is one who has a high probability of developing cancer. These subjects include, for instance, subjects having a genetic abnormality, the presence of which has been demonstrated to have a correlative relation to a higher likelihood of developing a cancer and subjects exposed to cancer causing agents such as tobacco, asbestos, or other chemical toxins. When a subject at risk of developing a cancer is treated with CpG on a regular basis, such as monthly, the subject will maintain a primed set of immune cells which are capable of recognizing and producing an antigen specific immune reponse. If a tumor begins to form in the subject, the subject will develop a specific immune response against one or more of the tumor antigens.

This aspect of the invention is particularly advantageous when the antigen to which the subject will be exposed is unknown. For instance, in soldiers at risk of exposure to biowarfare, it is generally not known what biological weapon to which the soldier might be exposed. A subject traveling to foreign countries may likewise not know what infectious agents they might come into contact with. By inducing immune system remodeling the immune system will be primed to respond to any antigen.

The antigen may be delivered to the immune system of a subject alone or with a carrier. For instance, colloidal dispersion systems may be used to deliver antigen to the subject. As used herein, a "colloidal dispersion system" refers to a natural or synthetic molecule, other than those derived from bacteriological or viral sources, capable of delivering to and releasing the antigen in a subject. Colloidal dispersion systems include macromolecular complexes, nanocapsuies, microspheres, beads, and lipid-based systems including oil-in-water emulsions, micelles, mixed micelles, and liposomes. A preferred colloidal system of the invention is a liposome. Liposomes are artificial membrane vessels which are useful as a delivery vector *in vivo* or *in vitro*. It has been shown that large unilamellar vessels (LUV), which range in size from 0.2 - 4.0 µ can encapsulate large macromolecules within the aqueous interior and these macromolecules can be delivered to cells in a biologically active form (Fraley, et al., *Trends Biochem. Sci*., 6:77 (1981)).

Lipid formulations for transfection are commercially available from QIAGEN, for example as EFFECTENE™ (a non-liposomal lipid with a special DNA condensing enhancer) and SUPER-FECT™ (a novel acting dendrimeric technology) as well as Gibco BRL, for example, as LIPOFECTIN™ and LIPOFECTACE™, which are formed of cationic lipids such as N-[1-(2, 3 dioleyloxy)-propyl]-N, N, N-trimethylammonium chloride (DOTMA) and dimethyl dioctadecylammonium bromide (DDAB). Methods for making liposomes are well known in the art and have been described in many publications. Liposomes were described in a review article by Gregoriadis, G., *Trends in Biotechnology* 3:235-241 (1985), which is hereby incorporated by reference.

It is envisioned that the antigen may be delivered to the subject in a nucleic acid molecule which encodes for the antigen such that the antigen must be expressed *in vivo*. The nucleic acid encoding the antigen is operatively linked to a gene expression sequence which directs the expression of the antigen nucleic acid within a eukaryotic cell. The "gene expression sequence" is any regulatory nucleotide sequence, such as a promoter sequence or promoter-enhancer combination, which facilitates the efficient transcription and translation of the antigen nucleic acid to which it is operatively linked. The gene expression sequence may, for example, be a mammalian or viral promoter, such as a constitutive or inducible promoter. Constitutive mammalian promoters include, but are not limited to the promoters for the following genes: hypoxanthine phosphoribosyl transferase (HPRT), adenosine deaminase, pyruvate kinase, β-actin promoter and other constitutive promoters. Exemplary viral promoters which function constitutively in eukaryotic cells include, for example, promoters from the simian virus, papilloma virus, adenovirus, human immunodeficiency virus (HIV), rous sarcoma virus, cytomegalovirus, the long terminal repeats (LTR) of moloney leukemia virus and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art.

In general, the gene expression sequence shall include, as necessary, 5' non-transcribing and 5' non-translating sequences involved with the initiation of transcription and translation, respectively, such as a TATA box, capping sequence, CAAT sequence, and the like. Especially, such 5' non-transcribing sequences will include a promoter region which includes a promoter sequence for transcriptional control of the operably joined antigen nucleic acid. The gene expression sequences optionally include enhancer sequences or upstream activator sequences as desired.

The antigen nucleic acid is operatively linked to the gene expression sequence. As used herein, the antigen nucleic acid sequence and the gene expression sequence are said to be "operably linked" when they are covalently linked in such a way as to place the expression or transcription and/or translation of the antigen coding sequence under the influence or control of the gene expression sequence. Two DNA sequences are said to be operably linked if induction of a promoter in the 5' gene expression sequence results in the transcription of the antigen sequence and if the nature of the linkage between the two DNA sequences does not (1) result in the introduction of a frame-shift mutation, (2) interfere with the ability of the promoter region to direct the transcription of the antigen sequence, or (3) interfere with the ability of the corresponding RNA transcript to be translated into a protein. Thus, a gene expression sequence would be operably linked to an antigen nucleic acid sequence if the gene expression sequence were capable of effecting transcription of that antigen nucleic acid sequence such that the resulting transcript is translated into the desired protein or polypeptide.

The antigen nucleic acid of the invention may be delivered to the immune system alone or in association with a vector. In its broadest sense, a "vector" is any vehicle capable of facilitating the transfer of the antigen nucleic acid to the cells of the immune system and preferably APCs so that the antigen can be expressed and presented on the surface of an APC. Preferably, the vector transports the nucleic acid to the immune cells with reduced degradation relative to the extent of degradation that would result in the absence of the vector. The vector optionally includes the above-described gene expression sequence to enhance expression of the antigen nucleic acid in APCs. In general, the vectors useful in the invention include, but are not limited to, plasmids, phagemids, viruses, other vehicles derived from viral or bacterial sources that have been manipulated by the insertion or incorporation of the antigen nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes *in vivo*. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, M., "Gene Transfer and Expression, A Laboratory Manual," W.H. Freeman C.O., New York (1990) and Murry, E.J. Ed. "Methods in Molecular Biology," vol. 7, Humana Press, Inc., Cliffton, New Jersey (1991).

A preferred virus for certain applications is the adeno-associated virus, a double-stranded DNA virus. The adeno-associated virus can be engineered to be replication - deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well-known to those of skill in the art. See e.g., Sanbrook et al., "Molecular Cloning: A Laboratory Manual," Second Edition, Cold Spring Harbor Laboratory Press, 1989. In the last few years, plasmid vectors have been found to be particularly advantageous for delivering genes to cells *in vivo* because of their inability to replicate within and integrate into a host genome. These plasmids, however, having a promoter compatible with the host cell, can express a peptide from a gene operatively encoded within the plasmid. Some commonly used plasmids include pBR322, pUC 18, pUC 19, pRC/CMV, SV40, and pBlueScript. Other plasmids are well-known to those of ordinary skill in the art. Additionally, plasmids may be custom designed using restriction enzymes and ligation reactions to remove and add specific fragments of DNA.

It has recently been discovered that gene carrying plasmids can be delivered to the immune system using bacteria. Modified forms of bacteria such as *Salmonella* can be transfected with the plasmid and used as delivery vehicles. The bacterial delivery vehicles can be administered to a host subject orally or by other administration means. The bacteria deliver the plasmid to immune cells, e.g. dendritic cells, probably by passing through the gut barrier. High levels of immune protection have been established using this methodology.

The CpG oligonucleotides of the invention are immune remodeling nucleic acid molecules. An "immune remodeling nucleic acid molecule" refers to a nucleic acid molecule, which contains an unmethylated cytosine-guanine dinucleotide sequence (i.e. "CpG DNA" or DNA containing a 5' cytosine followed by 3' guanosine and linked by a phosphate bond) and stimulates the repopulation of immune cells. An immune remodeling nucleic acid molecule can be double-stranded or single-stranded. Generally, double-stranded molecules are more stable *in vivo*, while single-stranded molecules have increased immune activity.

A "nucleic acid" or "oligonucleotide" means multiple nucleotides (i.e. molecules comprising a sugar (e.g. ribose or deoxyribose) linked to a phosphate group and to an exchangeable organic base, which is either a substituted pyrimidine (e.g. cytosine (C), thymine (T) or uracil (U)) or a substituted purine (e.g. adenine (A) or guanine (G)). As used herein, the terms refer to oligoribonucleotides as well as oligodeoxyribonucleotides. The terms shall also include polynucleosides (i.e. a polynucleotide minus the phosphate) and any other organic base containing polymer. Nucleic acid molecules can be obtained from existing nucleic acid sources (e.g. genomic or cDNA), but are preferably synthetic (e.g. produced by oligonucleotide synthesis). The entire CpG "oligonucleotide can be unmethylated or portions may be unmethylated but at lest the 5' CG 3' must be unmethylated.

In one preferred embodiment the invention provides a CpG oligonucleotide represented by the formula:

5'N₁X₁CGX₂N₂3'

wherein at least one nucleotide separates consecutive CpGs; X₁ is adenine, guanine, or thymine; X₂ is cytosine, adenine, or thymine; N is any nucleotide and N₁ and N₂ are nucleic acid sequences composed of from about 0-25 N's.

In another embodiment the invention provides an isolated CpG oligonucleotide represented by the formula:

5'N₁X₁X₂CGX₃X₄N₂3'

wherein at least one nucleotide separates consecutive CpGs; X₁X₂ is selected from the group consisting of GpT, GpA, ApA and ApT; X₃X₄ is selected from the group consisting of TpT, CpT, TpC, and ApT; N is any nucleotide and N₁ and N₂ are nucleic acid sequences composed of from about 0-25 N's. In a preferred embodiment N₁ and N₂ of the nucleic acid do not contain a CCGG quadmer or more than one CCG or CGG trimer. In another preferred embodiment the CpG oligonucleotide has the sequence 5'TCN₁TX₁X₂CGX₃X₄3'.

Preferably the CpG oligonucleotides of the invention include X₁X₂ selected from the group consisting of GpT, GpG, GpA and ApA and X₃X₄ is selected from the group consisting of TpT, CpT and TpC. For facilitating uptake into cells, CpG containing oligonucleotides are preferably in the range of 8 to 30 bases in length. However, nucleic acids of any size greater than 8 nucleotides (even many kb long) are capable of inducing immune remodeling if sufficient immunostimulatory motifs are present, since larger nucleic acids are degraded into oligonucleotides inside of cells. Preferred synthetic oligonucleotides do not include a CCGG quadmer or more than one CCG or CGG trimer at or near the 5' and/or 3' terminals. Stabilized oligonucleotides, where the oligonucleotide incorporates a phosphate backbone modification, as discussed in more detail below are also preferred. The modification may be, for example, a phosphorothioate or phosphorodithioate modification. Preferably, the phosphate backbone modification occurs at the 5' end of the nucleic acid for example, at the first two nucleotides of the 5' end of the oligonucleotide. Further, the phosphate backbone modification may occur at the 3' end of the nucleic acid for example, at the last five nucleotides of the 3' end of the nucleic acid. Alternatively the oligonucleotide may be completely or partially modified.

Preferably the CpG Oligonucleotide is in the range of between 8 and 100 and more preferably between 8 and 30 nucleotides in size. Alternatively, CpG oligonucleotides can be produced on a large scale in plasmids, which after being administered to a subject are degraded into oligonucleotides.

The CpG oligonucleotide may be directly administered to the subject or it may be administered in conjunction with a nucleic acid delivery complex. A "nucleic acid delivery complex" shall mean a nucleic acid molecule associated with (e.g. ionically or covalently bound to; or encapsulated within) a targeting means (e.g. a molecule that results in higher affinity binding to target cell (e.g. dendritic cell surfaces and/or increased cellular uptake by target cells). Examples of nucleic acid delivery complexes include nucleic acids associated with: a sterol (e.g. cholesterol), a lipid (e.g. a cationic lipid, virosome or liposome), or a target cell specific binding agent (e.g. a ligand recognized by target cell specific receptor). Preferred complexes should be sufficiently stable *in vivo* to prevent significant uncoupling prior to internalization by the target cell. However, the complex should be cleavable under appropriate conditions within the cell so that the nucleic acid is released in a functional form.

"Palindromic sequence" shall mean an inverted repeat (i.e. a sequence such as ABCDEE'D'C'B'A' in which A and A' are bases capable of forming the usual Watson-Crick base pairs. *In vivo,* such sequences may form double-stranded structures. In one embodiment the CpG oligonucleotide contains a palindromic sequence. A palindromic sequence used in this context refers to a palindrome in which the CpG is part of the palindrome, and preferably is the center of the palindrome. In another embodiment the CpG oligonucleotide is free of a palindrome. A CpG oligonucleotide that is free of a palindrome is one in which the CpG dinucleotide is not part of a palindrome. Such an oligonucleotide may include a palindrome in which the CpG is not part of the palindrome.

A "stabilized nucleic acid molecule" shall mean a nucleic acid molecule that is relatively resistant to *in vivo* degradation (e.g. via an exo- or endo-nuclease). Stabilization can be a function of length or secondary structure. Unmethylated CpG oligonucleotides that are tens to hundreds of kbs long are relatively resistant to *in vivo* degradation. For shorter CpG. oligonucleotides, secondary structure can stabilize and increase their effect. For example, if the 3' end of an oligonucleotide has self-complementarity to an upstream region, so that it can fold back and form a sort of stem loop structure, then the oligonucleotide becomes stabilized and therefore exhibits more activity.

Preferred stabilized oligonucleotides of the instant invention have a modified backbone. It has been demonstrated that modification of the oligonucleotide backbone provides enhanced activity of the CpG oligonucleotides when administered *in vivo.* CpG constructs, including at least two phosphorothioate linkages at the 5' end of the oligodeoxyribonucleotide in multiple phosphorothioate linkages at the 3' end, preferably 5, provides maximal activity and protected the oligodeoxyribonucleotide from degradation by intracellular exo- and endo-nucleases. Other modified oligodeoxyribonucleotides include phosphodiester modified oligodeoxyribonucleotide, combinations of phosphodiester and phosphorothioate oligodeoxyribonucleotide, methylphosphonate, methylphosphorothioate, phosphorodithioate, and combinations thereof. Each of these combinations and their particular effects on immune cells is discussed in more detail in copending PCT Published Patent Applications claiming priority to U.S. Serial Nos. 08/738,652 and 08/960,774, filed on October 30, 1996 and October 30, 1997 respectively, the entire contents of which is hereby incorporated by reference. It is believed that these modified oligonucleotides may show more stimulatory activity due to enhanced nuclease resistance, increased cellular uptake, increased protein binding, and/or altered intracellular localization.

Both phosphorothioate and phosphodiester oligonucleotides containing CpG motifs are active in APCs such as dendritic cells. However, based on the concentration needed to induce CpG specific effects, the nuclease resistant phosphorothioate backbone CpG oligonucleotides are more potent (2 µg/ml for the phosphorothioate vs. a total of 90 µg/ml for phosphodiester).

Other stabilized oligonucleotides include: nonionic DNA analogs, such as alkyl- and aryl-phosphates (in which the charged phosphonate oxygen is replaced by an alkyl or aryl group), phosphodiester and alkylphosphotriesters, in which the charged oxygen moiety is alkylated. Oligonucleotides which contain diol, such as tetraethyleneglycol or hexaethyleneglycol, at either or both termini have also been shown to be substantially resistant to nuclease degradation.

The nucleic acid sequences of the invention which are useful for inducing immune remodeling are those broadly described above. Exemplary sequences include but are not limited to those sequences shown in Table 1 as well as TCCATGTCGCTCCTGATGCT (SEQ ID NO: 47), TCCATGTCGTTCCTGATGCT (SEQ ID NO: 48), TCGTCGTTTTGTCGTTTTGTCGTT (SEQ ID NO: 53), TCGTCGTTGTCGTTGTCGTT (SEQ ID NO: 89); TCGTCGTTTTGTCGTTTTGTCGTT (SEQ ID NO: 90), TCGTCGTTGTCGTTTTGTCGTT (SEQ ID NO: 91), GCGTGCGTTGTCGTTGTCGTT (SEQ ID NO: 92), TGTCGTTTGTCGTTTGTCGTT (SEQ ID NO: 94), TGTCGTTGTCGTTGTCGTT (SEQ ID NO: 96) TCGTCGTCGTCGTT (SEQ ID NO:97), TCCTGTCGTTCCTTGTCGTT (SEQ ID NO: 79), TCCTGTCGTTTTTTGTCGTT (SEQ ID NO:81), TCGTCGCTGTCTGCCCTTCTT (SEQ ID NO:82), TCGTCGCTGTTGTCGTTTCTT (SEQ ID NO:83), TCGTCGTTTTGTCGTTTTGTCGTT (SEQ ID NO:90), TCGTCGTTGTCGTTTTGTCGTT (SEQ ID NO:91) TGTCGTTGTCGTTGTCGTT (SEQ ID NO:96), TCCATGACGTTCCTGACGTT (SEQ ID NO:100), GTCG(T/C)T (SEQ ID NO:101) and TGTCG(T/C)T (SEQ ID NO:102).

The ability of a particular CpG oligonucleotide to induce immune system remodeling can be tested in various immune cell assays which assess the stimulation index of the oligonucleotide. Preferably, the stimulation index of the CpG oligonucleotide with regard to B cell proliferation is at least about 5, preferably at least about 10, more preferably at least about 15 and most preferably at least about 20 as determined by incorporation of ³H uridine in a murine B cell culture, which has been contacted with 20 µM of ODN for 20h at 37°C and has been pulsed with 1 µCi of ³H uridine; and harvested and counted 4h later as described in detail in copending PCT Published Patent Applications claiming priority to U.S. Serial Nos. 08/738,652 and 08/960,774, filed on October 30, 1996 and October 30, 1997 respectively. For use *in vivo*, for example to induce immune system remodeling, it is important that the CpG oligonucleotide be capable of effectively inducing production of APC's such as dendritic cells. Oligonucleotides which can accomplish this are, for example, those oligonucleotides described in PCT Published Patent Applications claiming priority to U.S. Serial Nos. 08/738,652 and 08/960,774, filed on October 30, 1996 and October 30, 1997 respectively.

The CpG oligonucleotides are used in one aspect of the invention to induce repopulation of immune cells and preferably APCs. An APC has its ordinary meaning in the art and includes, for instance, dendritic cells such as immature dendritic cells and precursor and progenitor dendritic cells, as were as mature dendritic cells which are capable of taking up and expressing antigen. Such a population of APC or dendritic cells is referred to as a primed population of APCs or dendritic cells.

CpG oligonucleotides can be administered to a subject alone prior to the administration of an antigen. The oligonucleotides can also be administered to a subject in conjunction with an antigen to provide an immediate antigen specific response. A second antigen which may be the same or different from the first antigen may then be administered to the subject at least two days after the administration of CpG. The term in conjunction with refers to the administration of the CpG oligonucleotide slightly before or slightly after or at the same time as the first antigen. The terms slightly before and slightly after refer to a time period of 24 hours and preferably 12 hours.

When the CpG oligonucleotide is administered in conjunction with a first antigen the first antigen will determine the specificity of the immediate immune response. The CpG oligonucleotide acts as an effective "danger signal" and causes the immune system to respond vigorously to new antigens in the area. This mode of action presumably results primarily from the stimulatory local effects of CpG oligonucleotide on dendritic cells and other "professional" antigen presenting cells, as well as from the co-stimulatory effects on B cells. This effect occurs immediately upon the administration of the CpG oligonucleotide and is distinct from the repopulation event seen after about two days.

For use in therapy, an effective amount of an appropriate CpG oligonucleotide alone or formulated as a nucleic acid delivery complex can be administered to a subject by any mode allowing the oligonucleotide to be taken up by the appropriate target cells (e.g. dendritic cells). Preferred routes of administration include but are not limited to oral, transdermal (e.g. via a patch), injection (subcutaneous, intravenous, parenteral, intraperitoneal, intrathecal, etc.), intranasal, intratracheal, and mucosal. An injection may be in a bolus or a continuous infusion.

The term "effective amount" of a CpG oligonucleotide refers to the amount necessary or sufficient to realize a desired biologic effect. For example, an effective amount of an oligonucleotide containing at least one unmethylated CpG for treating an immune system deficiency could be that amount necessary to cause repopulation of the immune system, resulting in the development of an antigen specific immune response upon exposure to antigen. The effective amount for any particular application can vary depending on such factors as the disease or condition being treated, the particular CpG oligonucleotide being administered (e.g. the number of unmethylated CpG motifs or their location in the nucleic acid), the size of the subject, or the severity of the disease or condition. One of ordinary skill in the art can empirically determine the effective amount of a particular oligonucleotide without necessitating undue experimentation.

In addition to inducing immune system remodeling by regulating hematopoiesis, the CpG oligonucleotides induce hematopoiesis of specific immune cells such as platelets and erythroblasts. Such applications of the CpG oligonucleotides are useful for treating thrombocytopenia and anemia respectively.

Thrombocytopenia is a disorder associated with a deficiency in platelets. Platelets, which play an important role in blood coagulation, are derived by cytoplasmic fragmentation of the precursor stem cells, megakaryocytes, found in bone marrow. Arter formation, platelets leave the bone marrow and travel through the spleen and into the blood, with approximately one third of the platelets becoming sequestered in the spleen. The platelets which are transported to the blood, circulate for approximately seven to ten days. Platelets which are normally present in human blood at a concentration of 150,000-400,000 per microliter play a crucial role in hemostasis, or the regulation of bleeding. When the level of platelets falls below normal in a subject, the risk of hemorrhage increases in the subject.

Ordinarily when the level of circulating platelets decreases a feedback mechanism is initiated which results in increased production in the number, size, and ploidy of megakaryocytes. This mechanism, in turn, causes the production and release into the circulation of additional platelets. Although the feedback regulation of platelet levels is ordinarily sufficient to maintain a normal level of platelets in the circulation, several physiological conditions are capable of causing a significant imbalance in the level of platelets. Such conditions result in either thrombocytopenia or thrombocytosis (a condition caused by an increased level of platelets in the blood).

At least three physiological conditions are known to result in thrombocytopenia: a decreased production of platelets in the bone marrow; an increased splenic sequestration of platelets; or an accelerated destruction of platelets. In conventional therapies in order to successfully treat thrombocytopenia, one must first identify which mechanism is causing the decrease in platelet levels and then treat the subject by administering a drug or instituting a procedure which will eliminate the underlying cause of the platelet loss.

A loss of platelets due to decreased production of bone marrow, may be established by the examination of a bone marrow aspirate or biopsy which demonstrates a reduced number of megakaryocytes. A decreased production of bone marrow may result from myelosuppression as a consequence of gamma irradiation, therapeutic exposure to radiation, or cytotoxic drug treatment. Chemicals containing benzene or anthracene and even some commonly used drugs such as chloramphenicol, thiouracil, and barbiturate hypnotics can cause myelosuppression, resulting in thrombocytopenia. Additionally, rare bone marrow disorders such as congenital amegakaryocytic hypoplasia and thrombocytopenia with absent radii (TAR syndrome) can selectively decrease megakaryocyte production, resulting in thrombocytopenia.

Splenic sequestration of platelets can cause an increase in spleen size. Splenic sequestration can often be determined by bedside palpation to estimate splenic size. An increase in spleen size, or splenomegaly, is typically caused by portal hypertension secondary to liver disease, splenic infiltration with tumor cells in myeloproliferative or lymphoproliferative disorders, or macrophage storage disorders such as Gauchers disease. Splenectomy is often used to increase platelet counts in cases of excessive splenic sequestration.

Thrombocytopenia resulting from accelerated destruction of platelets is generally the cause of decreased levels of platelets in the blood when impaired production of bone marrow and splenic sequestration have been ruled out. The accelerated destruction of platelets is caused by either an immunologic disorder or a non-immunologic disorder. Immunologic thrombocytopenia can be caused, for example, by autoimmune disorders such as idiopathic thrombocytopenic purpura (ITP), viral or bacterial infections, and drugs. Non-immunologic thrombocytopenia is caused by vasculitis, hemolytic uremic syndrome, thrombotic thrombocytopenic purpura (TTP), disseminated intravascular coagulation (DIC) and prosthetic cardiac valves. Chronic ITP is often treated with high doses of steroids, intravenous gamma globulins, splenectomy, and even immunosuppressive drugs. Each of these therapeutic modalities provides only temporary relief and is associated with serious side effects. Additionally, approximately 20 percent of the chronic ITP patients do not respond to any of the known treatments.

As used herein, "thrombocytopenia" is a disorder in which the platelet levels in the affected individual fall below a normal range of platelets for that individual.

Thrombocytopenia includes infection-induced thrombocytopenia, treatment-induced thrombocytopenia, and physiologically-induced thrombocytopenia. Infection-induced thrombocytopenia is a disorder characterized by a low level of platelets in peripheral blood which is caused by an infectious agent such as a bacteria or virus. Treatment-induced thrombocytopenia is a disorder characterized by a low level of platelets in peripheral blood which is caused by therapeutic treatments such as gamma irradiation, therapeutic exposure to radiation, cytotoxic drugs, chemicals containing benzene or anthracene and even some commonly used drugs such as chloramphenicol, thiouracil, and barbiturate hypnotics. Physiologically-induced thrombocytopenia is a disorder characterized by a low level of platelets in peripheral blood which is caused by any mechanism other than infectious agents or therapeutic treatments causing thrombocytopenia. Factors causing physiologically-induced thrombocytopenia include, but are not limited to, rare bone marrow disorders such as congenital amegakaryocytic hypoplasia and thrombocytopenia with absent radii (TAR syndrome), an increase in spleen size, or splenomegaly, caused by portal hypertension secondary to liver disease, or macrophage storage disorders such as Gauchers disease, autoimmune disorders such as idiopathic thrombocytopenic purpura (ITP), vasculitis, hemolytic uremic syndrome, thrombotic thrombocytopenic purpura (TTP) disseminated intravascular coagulation (DIC) and prosthetic cardiac valves.

A subject having thrombocytopenia is a subject having any type of thrombocytopenia. In some embodiments the subject having thrombocytopenia is a subject having non-chemotherapeutic induced thrombocytopenia A subject having non-chemotherapeutic thrombocytopenia is a subject having any type of thrombocytopenia but who is not undergoing chemotherapy. In other embodiments the subject is a subject having chemotherapeutic induced thrombocytopenia, which includes any subject having thrombocytopenia and being treated with chemotherapeutic agents. As used herein, "a subject at risk of developing thrombocytopenia" is a subject who has a high probability of acquiring or developing thrombocytopenia. For example, a patient with a malignant tumor who is prescribed a chemotherapeutic treatment is at risk of developing treatment-induced thrombocytopenia and a subject who has an increased risk of exposure to infectious agents is at risk of developing infection-induced thrombocytopenia.

The CpG oligonucleotides can be used in the manufacture of a medicament for increasing platelet counts in a subject having thrombocytopenia or subject at risk of developing thrombocytopenia by administering to the subject an oligonucleotide, having a sequence including at least the following formula:

5' X₁CGX₂ 3'

wherein the oligonucleotide includes at least 8 nucleotides wherein C and G are unmethylated and wherein X₁ and X₂ are nucleotides, in an amount effective to increase platelet counts in the subject or in an amount effective to prevent a decrease in platelet counts ordinarily expected under platelet-depleting conditions in the subject when the subject is exposed to platelet-depleting conditions. An amount effective to increase platelet counts in the subject is an amount which causes an increase in the amount of circulating platelet levels. The actual levels of platelets achieved will vary depending on many variables such as the initial status of the immune system in the subject, i.e., whether the subject has mild to severe thrombocytopenia (e.g., resulting from an autoimmune disease or splenic sequestration). In general, the platelet levels of a subject who has severe thrombocytopenia will initially be very low. Any increase in the platelet levels of such a subject, even increases to a level which are still below a normal level, can be advantageous to the subject.

The platelet levels of a subject at risk of developing thrombocytopenia, on the other hand, are generally within a normal range. The oligonucleotide prevents the platelet levels of such a subject from decreasing to a level which would ordinarily occur when the subject is exposed to the condition causing thrombocytopenia. Thus, administering the oligonucleotide to the subject will inhibit to a medically significant extent, the decrease in platelet count that would otherwise occur in the absence of treatment according to the invention thereby preventing the development of thrombocytopenia to the extent that would ordinarily occur when the subject is exposed to the condition causing thrombocytopenia. Preferably the effective amount is one which prevents platelet levels from decreasing below a level of 50,000 platelets per microliter.

An effective amount of an oligonucleotide for increasing platelet levels may be measured by any conventional method known in the art for measuring platelet levels or for measuring parameters which correlate with platelet levels. Platelet count is determined simply by obtaining a blood sample and counting the number of platelets per microliter of blood. Platelet levels also can correlate with bleeding time.

As shown in the examples below, when a subject exposed to a thrombolytic triggering event is administered a CpG oligonucleotide the subject has an increased platelet count compared to a subject exposed to the thrombocytopenia triggering event but not treated with a CpG oligonucleotide. The response is particularly significant in a short period of time after the subject is exposed to the triggering event. For example, a significant increase in platelet counts is observed after four days.

Anemia is a blood disorder associated with a decrease in levels of red blood cells or erythrocytes. Erythrocytes are derived from the same undifferentiated progenitor cell in the bone marrow as platelets, referred to as the pluripotent stem cell. The pluripotent stem cell can generate an erythroid burst forming unit which can in turn form an erythroid colony forming unit. These cells eventually differentiate into erythroblasts, followed by erythrocytes.

The CpG oligonucleotides can be used in the manufacture of a medicament for treating anemia by administering to a subject having anemia an oligonucleotide, having a sequence including at least the following formula:

5' X₁CGX₂ 3'

wherein the oligonucleotide includes at least 8 nucleotides wherein C and G are unmethylated and wherein X₁ and X₂ are nucleotides, in an amount effective to induce erythropoiesis in the subject.

The amount of erythroblasts in a subject can be assessed by measuring the number of erythroblasts in bone marrow or by measuring the amount of erythrocytes in peripheral blood. The assay involving the measurement of erythrocytes in peripheral blood is more convenient and provides reasonable correlation to the number of erythroblasts.

"Anemia" as used herein refers to a disease in which there is a loss in number of red blood cells and/ or hemoglobin concentration. An anemic subject usually experiences a reduction in blood cell mass and a corresponding decrease in the oxygen carrying capacity of the blood. Many types of underlying disease cause anemia. These are discussed in extensive detail in Harrisons Principles of Internal Medicine; Ed. Isselbachet et al; 13th edition; McGraw-Hill Inc, New York, 1994. Anemia includes; for instance but is not limited to, a drug-induced anemia, an immunohemolytic disorder, genetic disorders such as hemoglobinopathy and inherited hemolytic anemia; inadequate production despite adequate iron stores; chronic disease such as kidney failure; and chronic inflammatory disorder such as rheumatoid arthritis.

As discussed above, a subject includes human and nonhuman vertebrates. In addition to the treatment of human thrombocytopenia and anemia, the CpG oligonucleotides are useful for treating nonhuman platelet and other blood cell disorders. For instance, the most common canine immune-mediated diseases include immune-mediated hemolytic anemia and immune-mediated thrombocytopenia (TTP). Both of these disorders are triggered by antibodies that attack red blood cells or platelets, respectively. The antibodies cause destruction of the cells leading to depletion of red blood cells or platelets. These disorders can be life threatening in dogs. Thus, the invention contemplates the treatment of canine immune-mediated hemolytic disorders through the administration of CpG oligonucleotides.

One method for assessing anemia in dogs is by determining blood cell counts. A low Packed Cell Volume (PCV), which can be assessed with a simple hematocrit, is indicative of anemia. The normal PCV for dogs is 40-59 and cats is 29-50. In severe cases of anemia, the animal generally has pale membranes in its mouth and appears weak and tired. Anemias can be classified as either regenerative or non-regenerative. In regenerative anemia, an animal is cable of responding by releasing new reticulocytes into the circulation. In non-regenerative anemia, there are no or very few immature RBC's in the sample and the body continues to lose red blood cells but no new ones are produced. The invention is useful for treating both types of anemia but is particularly useful in treating non-regenerative anemia.

The actual number of RBC's in a given quantity of blood of an animal may also be measured. The red blood cell count is measured as an actual number of cells found in a microliter (µl). Although each laboratory has their own set of "normal" ranges for a RBC count, the average is 5.6-8.7 x 10⁶ RBC's per microliter for dogs and 6.1-11.9 x 10⁶/µl for cats. The number of red blood cells may also be assessed by quantifying the amount of hemoglobin present. The normal hemoglobin level for a dog is 14-20 grams/deciliter and 9-15.6 g/dl for cats. The normal hematology values for dogs and cats are presented in the Table below.

| **Normal Hematology Values for Dogs and Cats** | | |
|---|---|---|
| Unit | Canine | Feline |
| Hematocrit (PCV) % | 40-59 | 29-50 |
| Hemoglobin g/dl | 14-20 | 9-15.6 |
| Red BloodCell Count x 10⁶ µl | 5.6-8.7 | 6.1-11.9 |
| White Blood Cell Count/µl | 6,000-17,000 | 4,900-20,000 |
| Neutrophils/µl | 3,000-12,000 | 2,500-12,500 |
| Lymphocytes/µl | 530-4,800 | 1,500-7,000 |
| Monocytes/µl | 100-1800 | 0-850 |
| Eosinophils/µl | 0-1,900 | 0-1,500 |
| Basophils /µl | <100 | <100 |
| Platelets/µl | 145-440 | 190-800 |

Horses also develop hematopoietic disorders such as anemia. One anemic condition that horses develop is an exercise induced increase in the number of crenated or spiculated red blood cells as described in US Patent No. 4,500,530. The red blood cell spiculation results in destruction of the cells leading to sports anemia. The CpG oligonucleotides of the invention may be used to treat or prevent this disorder in animals undergoing exercise. For instance, horses may be administered CpG prior to or after a race to prevent anemia.

The CpG oligonucleotide useful according to the invention is the CpG oligonucleotide described above. The preparations of the invention are administered in effective amounts. An effective amount of an oligonucleotide is that amount that will alone, or together with further doses, desirably modulate platelet or erythroblast levels such as by increasing the circulating level of platelets or erythroblasts of a subject. It is believed that doses ranging from 1 nanogram/kilogram to 100 milligrams /kilogram, depending upon the mode of administration, will be effective. The preferred range is believed to be between 0.1 and 10.0 mg/dose, particularly if given subcutaneously. More preferably, the amount is in the range of 0.5 - 1.0 mg/dose. Preferably, the effective amount is administered more than once. Preferably, the effective amount is administered every day to every thirty days and, more preferably, every five to fifteen days. This regimen can be maintained for up to six months to one year, or even the life of a subject. In one embodiment, the effective amount is administered once weekly for up to fifty-two weeks; more preferably, for up to thirty-two weeks, and even more preferably, for four to fourteen weeks. The absolute amount will depend upon a variety of factors (including whether the administration is in conjunction with other methods of treating thrombocytopenia or anemia, the number of doses and individual patient parameters including age, physical condition, size and weight) and can be determined with routine experimentation. It is preferred generally that a maximum dose be used, that is, the highest safe dose according to sound medical judgment.

In another embodiment, after a period of administration of the oligonucleotide, the therapy is discontinued for four to 52 weeks and restarted. Even more preferred, the therapy is restarted after eight to fourteen weeks.

The formulations of the invention are administered in pharmaceutically acceptable solutions, which may routinely contain pharmaceutically acceptable concentrations of salt, buffering agents, preservatives, compatible carriers, adjuvants, and optionally other therapeutic ingredients.

The CpG oligonucleotides and antigens may be administered per se (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

The pharmaceutical compositions of the invention contain an effective amount of a CpG oligonucleotide and antigens optionally included in a pharmaceutically-acceptable carrier. The term "pharmaceutically-acceptable carrier" means one or more compatible solid or liquid filler, dilutants or encapsulating substances which are suitable for administration to a human or other vertebrate animal. The term "carrier" denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

Compositions suitable for parenteral administration conveniently comprise sterile aqueous preparations, which can be isotonic with the blood of the recipient. Among the acceptable vehicles and solvents are water, Ringer's solution, and isotonic sodium chloride solution. In addition, sterile, fixed oils are conventionally employed as a solvent or suspending medium. For this purpose any bland fixed oil may be employed including synthetic mono- or di-glycerides. In addition, fatty acids such as oleic acid find use in the preparation of injectables. Carrier formulations suitable for subcutaneous, intramuscular, intraperitoneal, intravenous, etc. administrations may be found in Remington's Pharmaceutical Sciences, Mack Publishing Company, Easton, PA.

The CpG oligonucleotides or antigens useful in the invention may be delivered in mixtures of more than one CpG oligonucleotide or antigen. A mixture may consist of several CpG oligonucleotides or antigens.

A variety of administration routes are available. The particular mode selected will depend, of course, upon the particular CpG oligonucleotide or antigen selected, the particular condition being treated and the dosage required for therapeutic efficacy. The methods of administration, generally speaking, may be practiced using any mode of administration that is medically acceptable, meaning any mode that produces effective levels of an immune response without causing clinically unacceptable adverse effects. Preferred modes of administration are discussed above.

The compositions may conveniently be presented in unit dosage form and may be prepared by any of the methods well known in the art of pharmacy. All methods include the step of bringing the compounds into association with a carrier which constitutes one or more accessory ingredients. In general, the compositions are prepared by uniformly and intimately bringing the compounds into association with a liquid carrier, a finely divided solid carrier, or both, and then, if necessary, shaping the product.

Other delivery systems can include time-release, delay or sustained release delivery systems. Such systems can avoid repeated administrations of the compounds either CpG or antigen, increasing convenience to the subject and the physician. Many types of release delivery systems are available and known to those of ordinary skill in the art. They include polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs arc described in, for example, U.S. Patent 5,075,109. Delivery systems also include non-polymer systems that are: lipids including sterols such as cholesterol, cholesterol esters and fatty acids or neutral fats such as mono- di- and tri-glycerides; hydrogel release systems; sylastic systems; peptide based systems; wax coatings; compressed tablets using conventional binders and excipients; partially fused implants; and the like. Specific examples include, but are not limited to: (a) erosional systems in which an agent of the invention is contained in a form within a matrix such as those described in U.S. Patent Nos. 4,452,775, 4,675,189, and 5,736,152, and (b) diffusional systems in which an active component permeates at a controlled rate from a polymer such as described in U.S. Patent Nos. 3,854,480, 5,133,974 and 5,407,686. In addition, pump-based hardware delivery systems can be used, some of which are adapted for implantation.

The present invention is further illustrated by the following Examples, which in no way should be construed as further limiting.

### Examples:

### Example 1: CpG Oligonucleotides Induce Hematopoiesis.

### Methods

***Mice.*** Female C57BL/6, BALB/c, CBA/J, C3H/HeJ and SCID mice were purchased from Harlan Winkelmann (Borchen, Germany), Charles River Wiga (Sulzfeld, Germany) or Bomholtgard Breeding and Research Centre Ltd. (Ry, Denmark). All animals were housed in specific pathogen-free conditions and were used at 8-12 weeks of age (18 to 21 g of body weight).

***Tissues and cells.*** Femurs and spleens were aseptically removed and collected into ice-cold mouse tonicity PBS. Single cell suspensions were prepared and clumps were removed using a 100 µm pore size filter (Falcon, Becton Dickinson, Heidelberg, Germany). For the depletion of B (B220 positive) and T cells (CD4 or CD8 positive) cells, spleen cells were incubated with magnetic beads coated with the respective antibodies allowing negative selection of the splenic non B and non T cell portion (Dynal, Hamburg, Germany). Efficiency was checked by FACS-analysis, yielding in <5% B220 and <3% CD3 positive cells after depletion.

***Microbial stimuli and synthetic oligonucleotides.*** Phosphorothioate-stabilized oligonucleotides (ODN) were synthesized by TibMolBiol (Berlin, Germany). ODN sequences 'CG1' (= ODN 1668, containing a 'CG-motif' marked with bold letters: 5'-TCC-AT**G-ACG-TT**C-CTG-ATG-CT) (SEQ ID NO. 24) and control GC-ODN ('inverted CG' = ODN 1720: 5'-TCC-AT**G-AGC-TT**C-CTG-ATG-CT)(SEQ Id NO. 106) were taken from Krieg, AM et al. (1995) Nature 374:546-549. A second CpG-ODN 'CG2' (= ODN IL12p40: 5'-AGC-TAT-**GAC-GTT**-CCA-AGG) (SEQ ID NO. 107) and control ODN 'nCG' ('non-CG' = ODN AP1, without CG-motif: 5'-GCT-TGA-TGA-CTC-AGC-CGG-AA) (SEQ ID NO. 108) were described recently. Lipford, GB et al. (1997) Eur J Immunol 27:2340-2344. LPS from *E. coli* was purchased from Sigma (Munich, Germany). *Listeria monocytogenes* came from ATCC (American type culture collection strain 43251) and were grown in brain hear infusion (Difco, Detroit, USA) in overnight cultures. Number of bacteria was determined by OD₆₀₀ and checked by plating 10 µl aliquots of a serial 10-fold dilution on Columbia blood agar plates and counting the colony forming units after overnight incubation at 37°C.

***Treatment of mice.*** CpG-ODN were injected intraperitoneally (i.p.) in low endotoxin aqua ad injectable at 1-50 nmol/mouse, LPS was used at 10 µg/mouse. Negative control mice received injections with aqua ad injectable alone. Sublethal irradiation of mice (4 Gy) were performed using a ⁶⁰Co irradiator (Buchler, Braunschweig, Germany). For induction of ovalbumin (OVA)-specific cytotoxic T cells liposomes containing OVA were prepared as described. Lipford, GB et al. (1994) Vaccine 12:73-80. Inocula containing liposome-entrapped OVA with QuilA as adjuvant was injected in the hind footpads of C57BL/6 mice and 4 days later draining lymph nodes were harvested. The lymph node cells were cultured for 4 days with 10 U/ml recombinant (r)IL-2 and CTL assays were performed as described. Lipford, GB et al. (1994) Vaccine 12:73-80. For *Listeria* infection 2.5-5x10⁵ *Listeria*/mouse were inoculated intraperitoneally in a volume of 300 µl of brain heart infusion into sublethally irradiated mice (4 Gy) at day 14 post irradiation and survival was recorded for the following 30 days. ODN-protected mice received 10 nmol CpG-ODN (CG1) within 30 minutes after irradiation i.p., control mice were mock-treated (injection of aqua ad injectable). Each experiment performed had 3-10 mice per group per time point.

***Histopathology.*** At various time points post ODN-injection mice were killed by CO₂ asphyxiation. Selected tissues, including spleen, liver, lymph nodes and bone marrow were removed. For determination of splenomegaly, organs were trimmed of fat and contiguous tissues and weighted. The organ/body weight ratios were calculated. Tissues processed for microscopic evaluation were fixed in 10% neutral buffered formalin, embedded in paraffin, section (5 µm sections), mounted on slides and stained with hematoxylin and cosin (HE).

***Cytokines.*** A purified preparation of murine (mu) recombinant (r) kit ligand (hisKL) was kindly provided by Dr. R. Mailhammer (GSF-Forschungszentrum, Munich, Germany). It had been expressed in *E. coli* and purified by affinity chromatography as described. Murine recombinant interleukin 3 (IL-3) was produced by X63Ag8-653 myeloma cells transfected with retroviral vectors carrying the mouse IL-3 gene. In short-term proliferation assays with cytokine-dependent indicator cell lines, a final concentration of 1% (v/v) X63Ag8-653 supernatant equaled the effect of 10 ng/ml purified mu IL-3 obtained from Bachem Biochemica (Heidelberg, Germany). Murine recombinant GM-CSF was a kind gift from Immunex (Seattle, WA, USA). Human r IL-6 was obtained from Genzyme (Boston, MA, USA).

***Quantification of GM-CFU*****.** Individual spleen cell samples from mice were analyzed for GM-CFU by a soft agar colony assay as described previously. Staber, FG et al. (1982) Nature 298:79-82. In brief, the desired number of spleen cells (final concentration usually 3x10⁵ to 1x10⁶ per ml) was added to the agar medium mixture and 1 ml was added in triplicate to 35-mm-diameter culture plates (Greiner, Nürtingen, Germany). Prior to cell plating a saturating amount of a pre-tested cocktail of myeloid cell growth promoting cytokines including mu r hisKL, mu r IL-3 and r GM-CSF (50 µl/plate, respectively) had been added to the plates corresponding to final concentrations of 500 ng/ml hisKL, 5 ng/ml IL-3, and 25 ng/ml GM-CSF. After gelling of the agar medium at 4°C the cultures were incubated for 7 days at 37°C in a fully humidified atmosphere of 10% CO₂ in air. Cellular aggregates containing at least 50 cells were scored as colonies.

***BFU-E Assays*****.** A commercially available (CellSystems Biotechnologie Vertrieb GmgH, Remagen, Germany) culture medium composition (MethoCult™ HCC-3340) was used which contained 0.9% methylcellulose in alpha modified Eagle's medium. 30% foetal bovine serum 1% BSA, 10⁻⁴ M 2-mercaptoethanol, 2 mM L-glutamine and 3 units/ml r human (hu) erythropoietin. To this medium (2.7 ml/tube) 0.3 ml cell suspension was added containing 13.2x10⁵/ml spleen cells. The culture medium was further complemented with 100 µl mu r hisKL (stock: 10 µg/ml), 100 µl mu r IL-3 (stock: I µg/ml), and 100 µl hu r IL-6 (stock: 100 ng/ml) and carefully mixed with a syringe fitted with a 1.4x40m needle. This resulted in a final concentration of 3 µg/ml hisKL, 3 ng/ml mu r IL-3, 3 ng/ml hu r IL-6 and 4x10⁵/ml spleen cells which were plated in triplicate aliquots of 1 ml per Petri dish (Greiner, Nürtingen, Germany). Growth of erythroid colonies (< 50 hemoglobin containing cells) was scored after an incubation period of 9 days at 37°C in a humidified atmosphere containing 10% CO₂ in air.

***Day-11 CFU-Assay*****.** Spleen colony forming units (CFU-S) were measured by the macroscopic spleen colony assay of Till and McCulloch. Female C57BL/6 mice at the age of 12 weeks were irradiated with 8 Gy (¹³⁷Cs), a potentially lethal dose which was found to give no formation of endogenous macroscopic spleen colonies. Within a period of 1 to 4 hours, the irradiated mice were anaesthetized with diethylether and injected into the retro-orbital plexus with 2.5x10⁵ spleen cells/200 µl/mouse derived from individual normal C57BL/6 mice or from mice sacrificed 6 days after i.p. treatment with 10 nmol/mouse CpG-ODN (5 mice group treated with CpG-ODN or vehicle, respectively). Each donor spleen suspension was injected into 5 irradiated mice. Eleven days after transplantation, recipient mice were killed and their spleens were excised and placed in Bouin's fixative to determine the number of macroscopic visible spleen colonies.

***Flow Cytometry.*** Cells (5x10⁵ - 10⁶) were washed in PBS containing 2% FCS and incubated for 10 min at 4°C with anti FcγRII/III antibody from PharMingen (Hamburg, Germany) to block unspecific binding of the following antibody reagents. Monoclonal antibodies (mAb), used at 5-20 µg ml, including mAb against B220. CD3 Mac-1 and GR-1, FITC and PE-labeled Antibodies were purchased from PharMingen (Hamburg, Germany). Isotype controls included purified rat IgG2a, rat IgG2b, and Hamster IgG (all Pharmingen). Between all incubation steps (30 min, 4°C), cells were washed with PBS/FCS. FACS-Analysis was performed on a Coulter Epics XL flow cytometer (Krefeld, Germany), acquiring 10,000 events. FACS data were analyzed using WinMDI 2.6 FACS-software.

### RESULTS

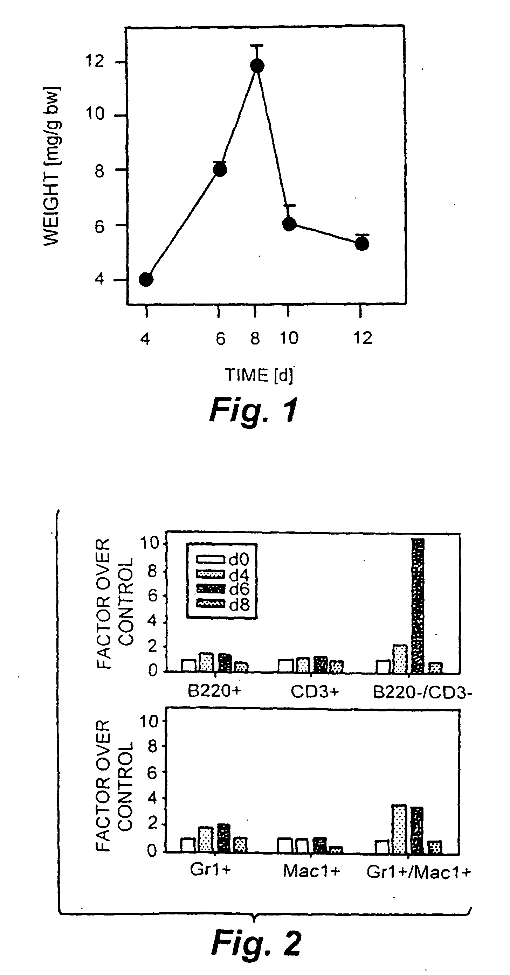

***CpG-ODN cause transient splenomegaly.*** Mice challenged i.p. with ODN display a dramatic splenomegaly (Fig. 1). Kinetically, spleen weight increases to a peak at day 6 and subsequently normalized. As detailed in table 8 column (a), injection of CPG ODN (CG1 or CG2) significantly induced splenomegaly, whereas in control non-CpG ODN injected animals spleens were not significantly different for mock injected animals. Thus murine splenomegaly was induced in a CpG motif dependent manner and peaked at day 6 post injection.

Fig. 1 shows the kinetics of increased spleen weight induced by CpG-ODN. CpG-ODN (CG1) was injected once i.p. at day 0 (10 nmol/mouse). Spleens were removed at day 0, 4, 6 and 12, trimmed of contiguous tissues and weighed. Organ weight is presented as spleen weight (mg/total body weight (g) (means values of 5 C57BL/6 mice per group ± SD).

CpG ODN has been shown to induce B cell proliferation with a maximum between days 1-3 post challenge. McIntyre, KW et al. (1993) Antisense Res Dev 3:309-322; Branda, RF et al. (1993) Biochem Pharmacol 45:2037-2043. We therefore addressed the question of whether the observed splenomegaly was due to CpG ODN induced B cell mitogenicity. Cell surface phenotyping of splenic cells by FACS-analysis revealed that the absolute frequency of B220 positive cells (used as B cell marker) was only marginally increased (Fig. 2). The most dramatic effect observed however was a transient but significant increase at day 6 in the B220-CD3 double negative compartment. Histologically, an increased number of large immature blasts and erythroblasts was detected with a maximum at day 6 suggesting increased hematopoietic activity.

Fig. 2 shows changes in phenotype of spleen cells after stimulation with CpG-ODN. CpG-ODN (CG1) was injected once i.p. at day 0 (10 nmol/mouse). Spleens were removed at indicated time points and FACS-stained for B220/CD3 and GR-1/Mac-1 (double stainings). Increase of absolute cell number is presented as factor over day 0 control spleen cells (mean values of 3 individual C57BL/6 mice).

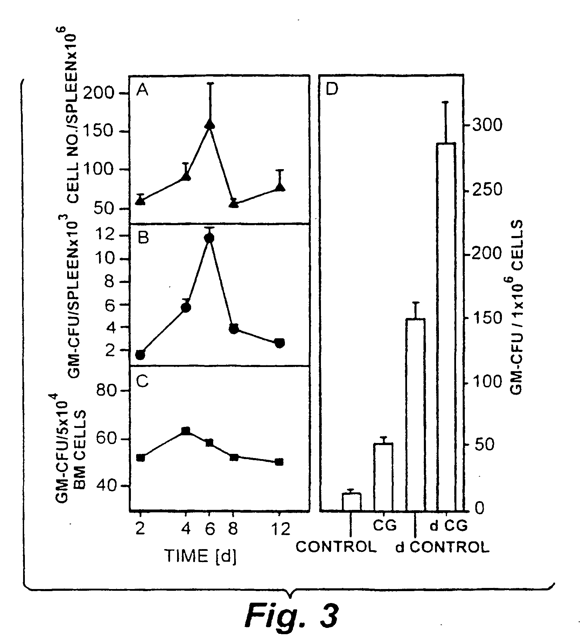

***Splenomegaly is associated with extramedullary hematopoiesis.*** In contrast to humans, mice display a basal hematopoietic activity in the spleen. Morrison, SJ et al. (1995) Annu Rev Cell Dev Biol 11:35-71. To analyze whether CpG-ODN induced splenomegaly correlated with increased splenic hematopoietic activity, we measured the number of granulocyte-macrophage progenitor cells (GM-CFU) in spleens of CpG ODN treated mice. There was a 7.4-fold increase in splenic GM-CFU numbers at day 6, reflecting the kinetics of total spleen cell number (Fig. 3A, 3B). We also analyzed the induction of GM-CFU in bone marrow from treated mice. There was a slight increase in the number of GM-CFU in bone marrow (day 4) that preceded the splenic increase at day 6, as if mobilization of bone-marrow derived progenitor cells to the spleen may have taken place (Fig. 3C). In addition, we enriched by immunomagnetic separation the B220/CD3 double negative cell fraction from day 6 spleens of CpG or non-CpG treated mice and tested for GM-CFU formation. These cells were shown to be highly enriched for myeloid progenitor cells (Fig. 3D). Thus the dramatic increase of the non-B, non-T cell fraction at day 6 post CpG-ODN injection was accompanied by an increased number of GM-CFU within the spleen.

Fig. 3 shows CpG-ODN induced changes in splenic cell number, number of splenic and BM GM-CFU. A: Kinetics of CpG-ODN (CG1) induced changes in splenic cell count (mean values of 3 C57BL/6 mice per time point ± SD). B: Evaluation of hematopoietic progenitor cells in the spleens of CpG-ODN-treated mice. Graph display number of GM-CFU per spleen per time point (mean values of triplicate spleen cell cultures of 3 mice ± SEM). C: Frequency of GM-CFU in pooled bone-marrow cells from 3 mice per time point. D: Increased number of GM-CFU in B220/CD3 double negative spleen cell fraction. Spleen cells from 4 non-treated C57BL/6 mice and 3 CpG-ODN (CG1)-injected mice (± SEM) were pooled at day 6 post i.p. injection. A portion of these cells was depleted for B220+, CD4+ and CD8+ cells and both non-depleted and depleted (d) spleen cells were analyzed for GM-CFU by soft agar colony assay.

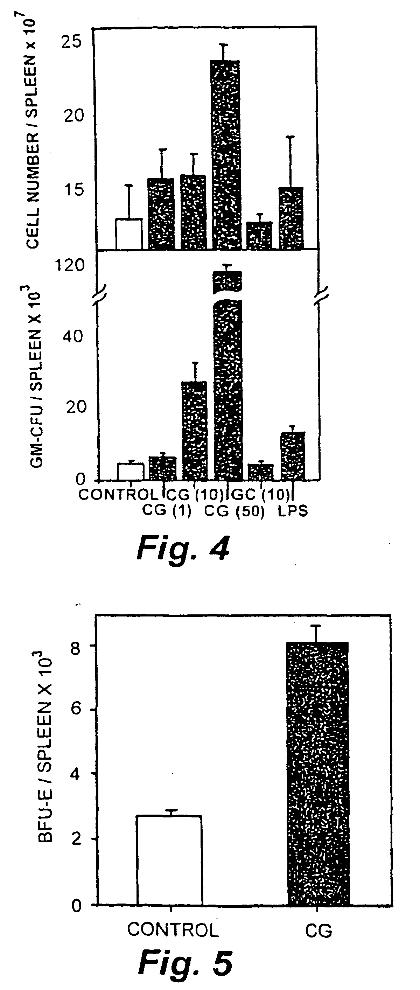

The induction of splenic hematopoiesis was CpG-DON dose and sequence dependent (Fig. 4, also see Fig. 3D, table 1b and 1c). Sequences lacking the 'CpG-motif' (nCG) failed to induce extramedullary hematopoiesis and CG inversion (GC-ODN) almost completely abolished the hematopoietic effect of the ODN CG1. Single shot injection of CpG ODN also compared well with the documented hematopoietic activity triggered by LPS (Fig. 4). Apte, RN et al. (1976) J Cell Physiol 71-78; Apte, RN et al. (1976) Exp Hematol 4:10-18; Staber, FG et al. (1980) Proc Natl Acad Sci USA 77:4322-4325. In addition to the granulocyte-macrophage progenitors, the number of pure erythroid progenitors post CpG ODN injection was also increased as determined by the number of Burst-forming Units (BFU-E) per spleen (Fig. 5). Analysis of peripheral blood over 12 days revealed no significant changes apart from a transient leukocytosis at day 2-4. Thus the transient splenomegaly observed in ssDNA injected mice was CpG motif dependent and associated with extramedullary hematopoiesis.

Fig. 4 shows a dose titration of CpG-ODN. 3 BALB/c mice were injected with CpG-ODN (CG1) at different concentrations (1, 10 and 50 nmol/mouse, grey bars) or LPS (10 µg/mouse, black bars), solvent (aqua ad injectable, white bars) and GC-ODN (dark grey bars) served as negative controls. Increased numbers of spleen cells and GM-CFU per spleen (mean values ± SEM) induced by CpG-ODN were measured at day 6 post injection.

Fig. 5 shows an increased number of BFU-E induced by CpG-ODN. Spleen cells of mice treated with ODN CG1 (black bars) or solvent control (aqua ad injectable, white bars) were plated in a methylcellulose-based colony assay at day 6 post injection and scored for growth of hemoglobin-containing erythroid colonies after an incubation period of 9 days in *vitro* (mean values of 5 C57BL/6 mice ± SEM).

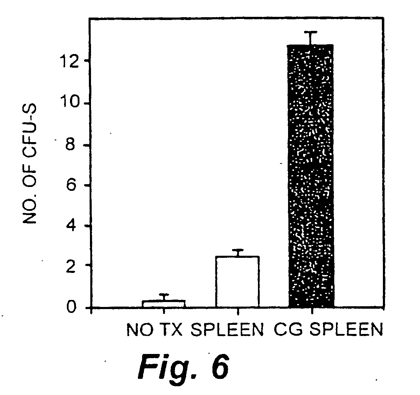

***Increased number of splenic progenitor cells is measurable by the spleen colony-forming unit assay (CFU-S).*** Spleen colony-forming units (CFU-S) cells are capable of lodging in the spleen and forming macroscopic nodules 11 days upon adoptive transfer into the bone marrow-ablated host. As shown in Fig. 6, a significantly enhanced number of CFU-S was detected in spleen cells taken from CpG-ODN pre-treated mice. CFU-S exhibit many characteristics of primitive hematopoietic stem cells such as extensive proliferative capacity, the ability for self-renewal and the capability of generating spleen colonies containing cells of multiple hematopoietic lineages that can rescue animals from lethal irradiation. Spangrude, GJ et al. (1988) Science 241:58-62. In view of this data experiments were designed to examine the reconstitution of lethally irradiated mice by adoptive transfer of CFU-S contained in spleens of CpG-ODN treated mice.

Fig. 6 shows a determination of spleen colony forming units of normal vs. CpG-ODN induced spleen cells (CFU-S Assay). CpG-ODN (CG1) induced splenic hematopoiesis leads to increased number of macroscopic visible colonies after injection into lethally irradiated mice. Graph displays numbers of macroscopic nodules per spleen of untreated mice after lethal irradiation (grey bar) compared to lethally irradiated mice after injection of 2.5x10⁵ normal spleen cells (white bar) and irradiated mice injected with spleen cells from ODN-pre-treated mice (day 6 post ODN CG1, black bar) (mean values of 5 independent experiments using 3-5 C57BL/6 mice per spleen ± SEM).

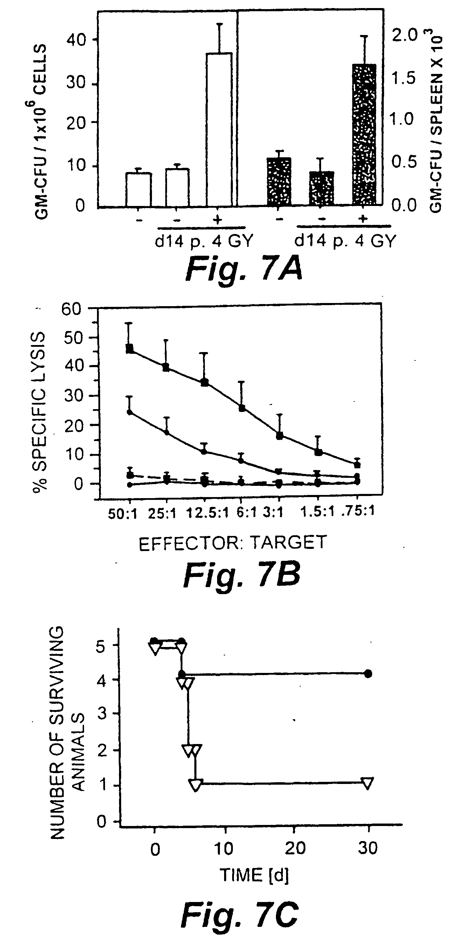

***CpG-ODN mediate radioprotective effects in myelosuppression*****.** Hematopoietic progenitor cells are considered as rather radioresistant. Morrison, SJ et al. (1995) Annu Rev Cell Dev Biol 11:35-71. Since CpG-DON induce extramedullary hematopoiesis via mobilization of CFU-S to the spleen we analyzed whether CpG-ODN could mediate radioprotective effects in sublethally irradiated mice. CpG challenge of sublethally irradiated mice (4 Gy) lead within day s to a 4 fold increase lead within 14 days to a 4 fold increase of splenic GM-CFU (Fig. 7A). Next, we addressed the question whether CpG-ODN driven hematopoiesis in sublethally irradiated mice allows accelerated recovery of the immune system. Two experimental systems were chosen: one, the induction of CTL responses to proteinaceous antigens (Lipford, GB et al. (1997) Eur J Immunol 27:2340-2344), and two, resistance to the intracellular pathogen *Listeria monocytogenes* (Endres, R et al. (1997) Immunity 7:419-432). Mice were treated with CpG-ODN within 30 minutes after sublethal irradiation (4 Gy), allowed to recover for 18 days and thereafter immunized subcutaneously (s.c.) with ovalbumin (OVA) containing liposomes plus QuilA as adjuvant. After 4 days cells of draining lymph nodes were harvested, cultured for an additional four days and assayed for OVA specific CTL activity. As detailed in Fig. 7B lymphocytes from CpG-ODN treated irradiated mice displayed an enhanced CTL response compared to non-treated irradiated mice. Basically similar results were obtained in an infection model using *L. Monocylogenes* infection at day 14. Overall the data given in Fig. 7 demonstrate a correlation between CpG-ODN induced extramedullary hematopoiesis and the ability to mount cytotoxic T cell responses or protective immune responses towards bacterial infections. CpG-ODN compensate radiation induced damage of the lympho-hematopoietic system by accelerating regeneration from hematopoietic progenitor cells.

Fig. 7 shows an increased number of CM-CFU and enhanced CTL function after ODN-injection correlates with increased resistance towards lethal listeriosis in sublethally irradiated mice. A: Increased number of GM-CFU per 1 million cells (left panel) and GM-CFU per spleen (right panel) at day 14 after sublethal irradiation (4 Gy) and injection of CpG-ODN (CG1). Number of splenic GM-CFU of 3 mice per group (± SEM) with (+) and without (-) ODN injection was compared to normal mice without irradiation. B: OVA-specific primary CTL-response using ODN CG1 as adjuvant. CTL-function of ODN-treated (squares) and mock-treated (circles) mice immunized at day 18 post-sublethal irradiation was compared. The target cells were EL4 cells (dotted lines), or EL4 cells pulsed with the SIINFEKL peptide (solid lines) and specific lysis was measured by ⁵¹Cr release (mean values ± SD of three mice per group). C: Increased resistance towards listeria infection in sublethally irradiated mice treated with CG1 (closed circles) compared to irradiation alone (open triangles). Mice were infected with 5x10⁵ listeria at day 14 post irradiation and survival was recorded for 30 days.

In this example extramedullary hematopoiesis induced by CpG-ODN are described and characterized. Mice challenged with CpG-ODN develop transient splenomegaly peaking at day 6 which is associated with increased splenic frequencies of B220/CD3 double negative cells. Within this subset hematopoietic progenitor cells were detected by GM-CFU and BFU in vitro assays. CpG-ODN shorten the period of radiation induced myelosuppression by improving hematopoietic regeneration via enhanced CFU-S export to the spleen. As a consequence recovery of cytotoxic T cell responses and resistance to bacterial infection developed earlier in time post sublethal irradiation.

Bacterial DNA and CpG-ODN activate polyclonally B cells and stimulate APC, such as dendritic cells and macrophages. Krieg, AM et al. (1995) Nature 374:546-549; Sparwasser, T et al. (1997) Nature 386:336-337; Sparwasser, T et al. (1997) Eur J Immunol 27:1671-1679; Sparwasser, T et al. (1998) Eur J Immunol 28:2045-2054; Stacey, KJ et al. (1996) J Immunol 157:2116-2122; Lipford, GB et al. (1997) Eur J Immunol 27:3420-3426. CpG-ODN activate DC and macrophages in vitro to secrete large amounts of hematopoietically active cytokines including IL-6, GM-CSF, IL-1, 1L-2 and TNF-α. Sparwasser, T et al. (1997) Nature 386:336-337; Sparwasser, T et al. (1997) Eur J Immunol 27:1671-1679; Sparwasser, T et al. (1998) Eur J Immunol 28:2045-2054; Lipford, GB et al. (1997) Eur J Immunol 27:3420-3426; Halpern, MD et al. (1996) Cell Immunol 167:72-78; Chace, JH et al. (1997) Clin Immunol Immunopathol 84:185-193; Roman, M et al. (1997) Nat Med 3:849-854 31-33. Mice challenged with CpG-ODN also transiently exhibit high serum concentrations of these cytokines. Sparwasser, T et al. (1997) Nature 386:336-337; Lipford, GB et al. (1997) Eur J Immunol 27:3420-3426. To date it is unclear which of these triggers extramedullary hematopoiesis. It is possible that CpG-ODN target bone marrow stroma cells to release hematopoietically active cytokines.

Initially, we anticipated that the observed splenomegaly reflected CpG-ODN induced B cell mitogenicity because most references attribute CpG induced splenomegaly to B cells. Krieg, AM et al. (1995) Nature 374:546-549; McIntyre, KW et al. (1993) Antisense Res Dev 3:309-322; Branda, RF et al. (1993) Biochem Pharmacol 45:2037-2043. However it was only between days 1-4 after CpG-ODN challenge that proliferating B220⁺ cells account for the relative increase in splenic cellularity (Fig. 2). Supporting a conclusion of non-B, non-T cell involvement in splenomegaly, spleen enlargement was also observed in SCID-mice which lack B and T cells. At day 6 after CpG-ODN challenge B220-/CD3- splenic cells were prevalent (Fig. 2), and histology revealed abundant large immature blast cells indicative for extramedullary hematopoiesis. In GM-CFU in vitro assays the increased hematopoietic activity could be defined to the B220⁻/CD3⁻ population. In vitro colony assays (Figs. 4, 5, 6, Table 8) demonstrated massive increase in splenic numbers of granulocyte, macrophage and early erythrocyte progenitor cells. In peripheral blood of the mice however, changes were discrete in that leukocytosis and a slight reduction of numbers of erythrocytes and platelets were observed. Unlike humans, the spleen of mice accounts for a large portion of hematopoietic activity.

It is known that bacterial stimuli (LPS or complete Freud's adjuvant containing heat killed mycobacteria) can trigger increased splenic hematopoiesis (Apte, RN et al. (1976) J Cell Physiol 71-78; Staber, FG et al. (1980) Proc Natl Acad Sci USA 77:4322-4325; McNeill, TA (1970) Immunology 18:61-72) possibly via macrophage derived hematopoietic growth factors that stimulate the generation and mobilization of blood cells necessary to combat bacterial infections (reviewed in Morrison, SJ et al. (1995) Annu Rev Cell Dev Biol 11:35-71). Here we show that CpG-ODN known to mimic the immunostimulatory effects of bacterial DNA (Krieg, AM et al. (1995) Nature 374:546-549) displayed the capacity to potentiate hematopoiesis. Furthermore, CpG-ODN was shown to enhance hematopoietic regeneration from myelosuppression as caused by sublethal irradiation. For example, irradiated and CpG-ODN treated mice exhibited increased numbers of splenic GM-CFU, mounted antigen specific CTL responses and displayed enhanced resistance to *Listeria monocytogenes* infection (Fig. 7). The enhanced number of splenic GM-CFU two weeks after injection of CpG-ODN correlated with an enhanced immune system recovery in myelosuppressed mice. Hematopoietic depression and subsequent susceptibility to potentially lethal opportunistic infections are well-documented phenomena following chemotherapy, radiotherapy or accidental radiation exposures. Inexpensive mitigation of myelosuppression would be of great clinical value. Our data indicate that CpG-ODN can mitigate radiation induced myelosuppression via augmentation of hematopoiesis yielding in accelerated reconstitution of the immune system.

**TABLE 8**

| | a) weight (mg/g bw) | b) GM-CFU/spleen x 10³ | c) GM-CFU/10⁶ cells |
|---|---|---|---|
| Control | 3.92 ± 0.27 | 1.20 ± 0.43 | 7.75 ± 2.75 |
| CG1 | 6.84 ± 1.42 | 8.58 ± 2.52 | 28.50 ± 7.75 |
| GC | 4.36 ± 0.36 | 2.07 ± 0.57 | 12.50 ± 3.00 |
| CG 2 | 6.91 ± 1.89 | 4.47 ± 0.87 | 13.50 ± 2.25 |
| nCG | 3.95 ± 0.31 | 1.13 ± 0.24 | 6.75 ± 1.50 |

Table 8 shows increased spleen weight and number of GM-CFU after injection of CpG-ODN. a) Increased spleen weight induced by CpG-ODN. CpG-ODN (CG1, CG2) induced significant splenomegaly in mice (means values of 3 C57BL/6 mice per group ± SD, *t*-test: p≤0.05), whereas non-CpG ODN (nCG) did not. Inversion of the CG-dinucleotide (GC-ODN) almost completely abolishes the effect of GC1. Comparison between ODN-treated (10 nmol/mouse) and mock-treated mice (injection with aqua ad injectable). b) Number of GM-CFU per spleen (mean values of triplicate values of 3 C57BL/6 mice per group ± SEM). c) Number of GM-CFU per 1 million cells (mean values of triplicate values of 3 mice per group ± SEM).

### Example 2: CpG-ODN Induced Blood and Cell Resistance to 5-Fluorouracil (5-FU).

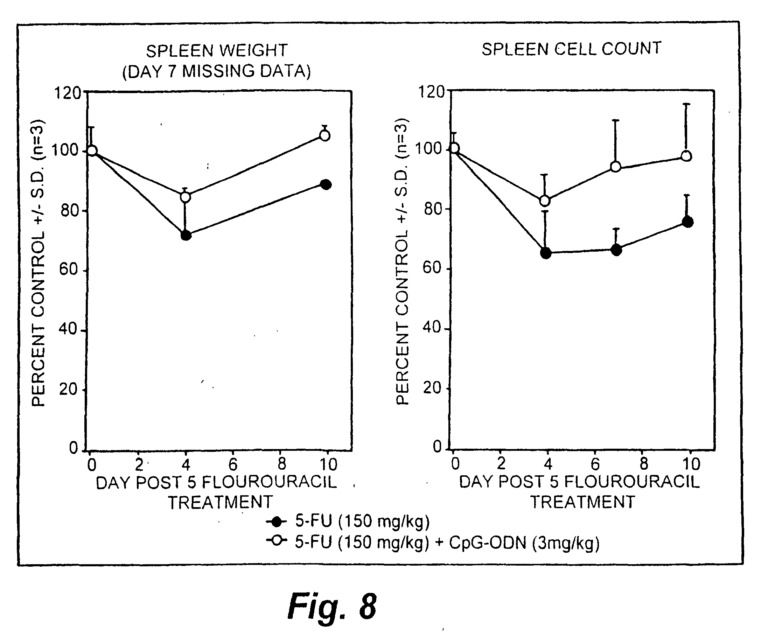

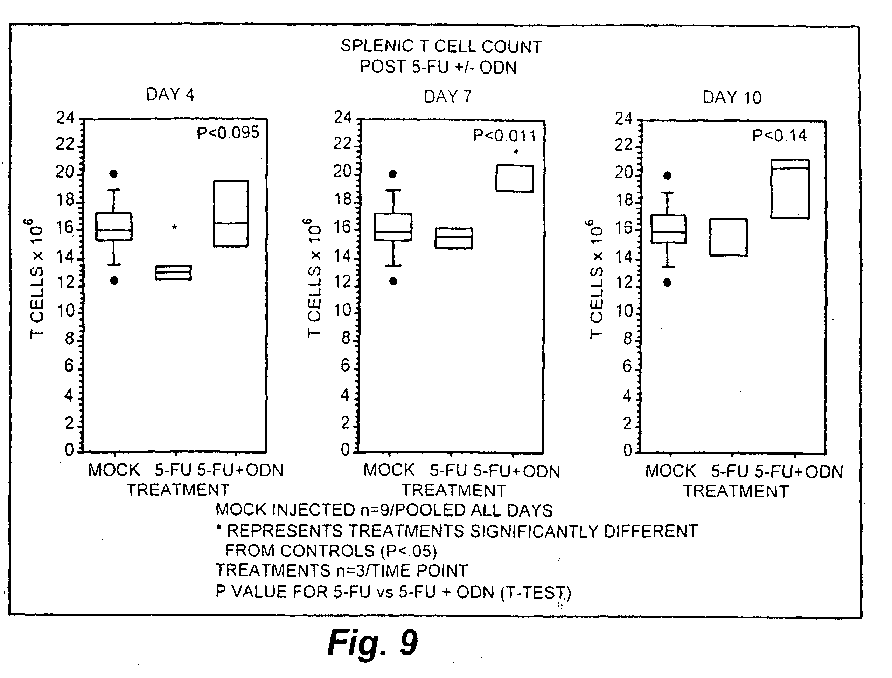

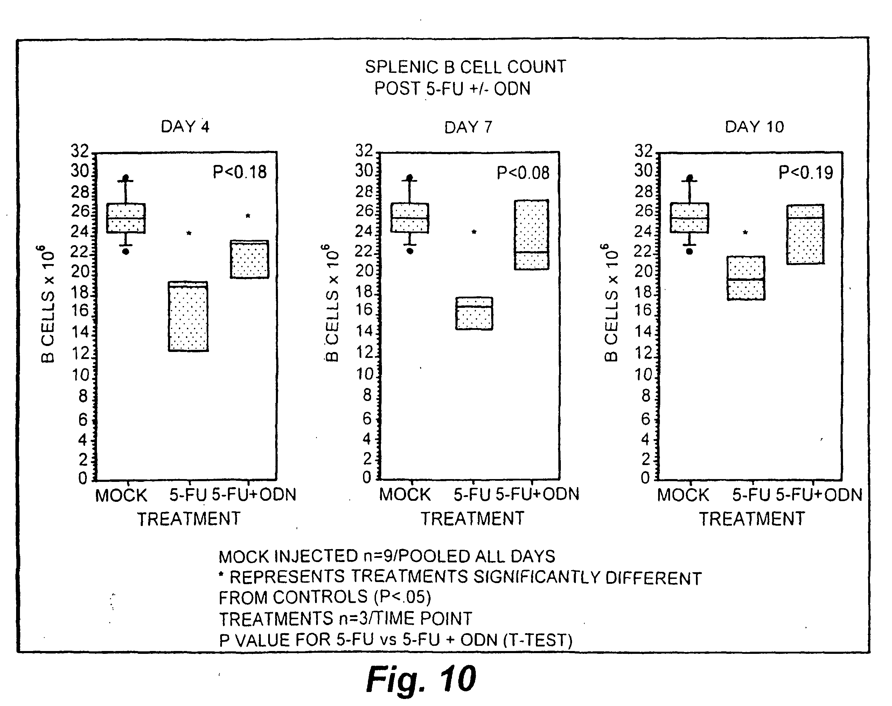

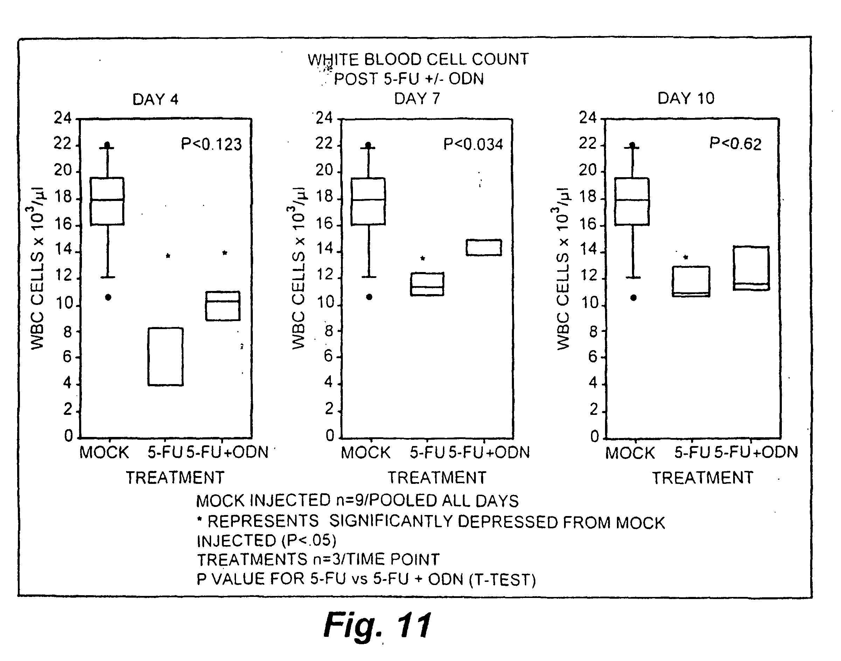

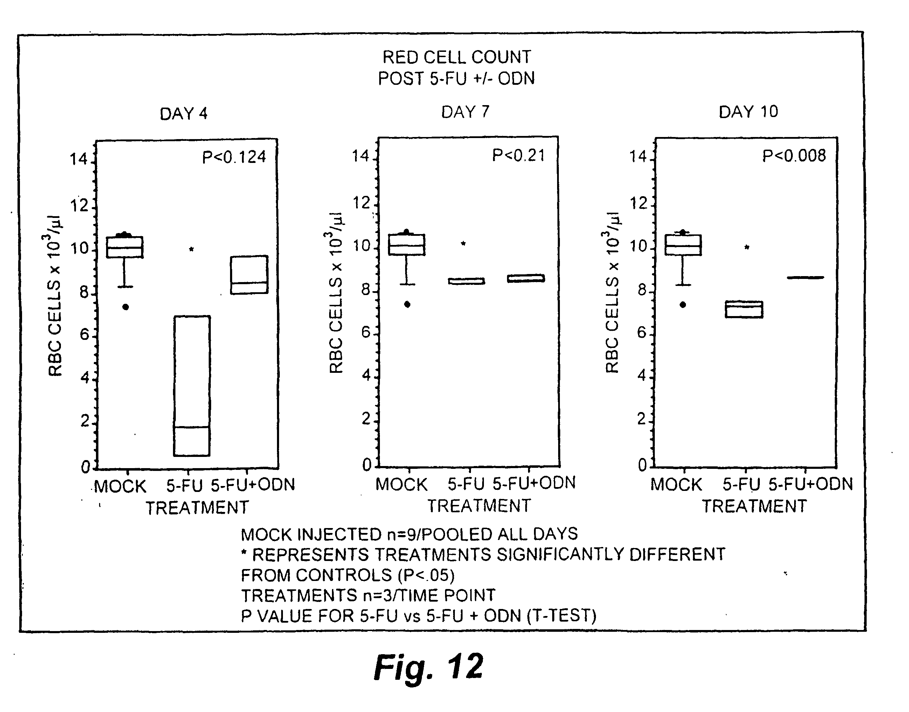

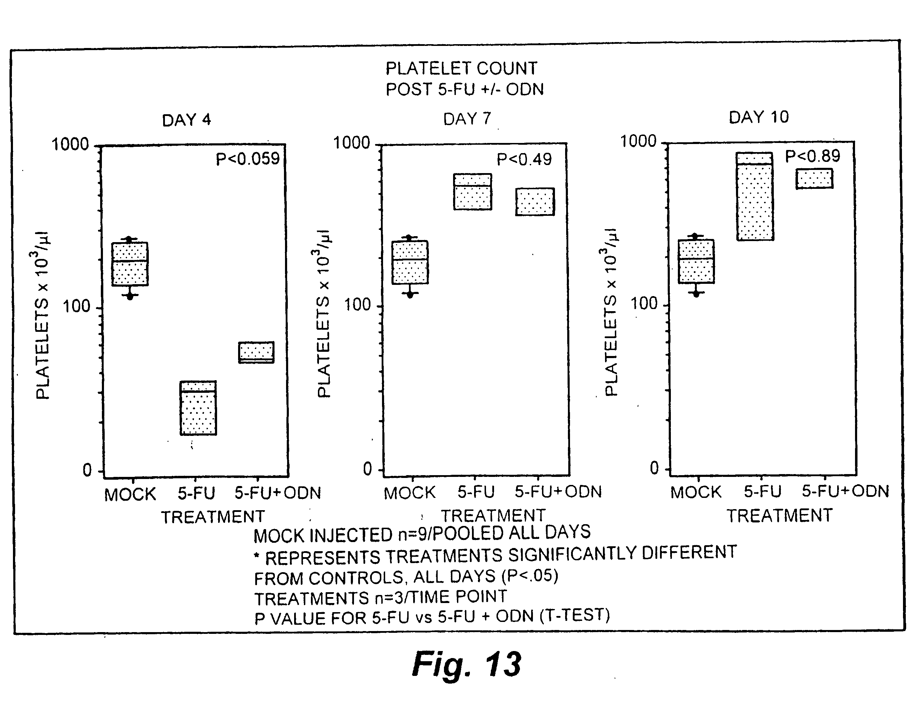

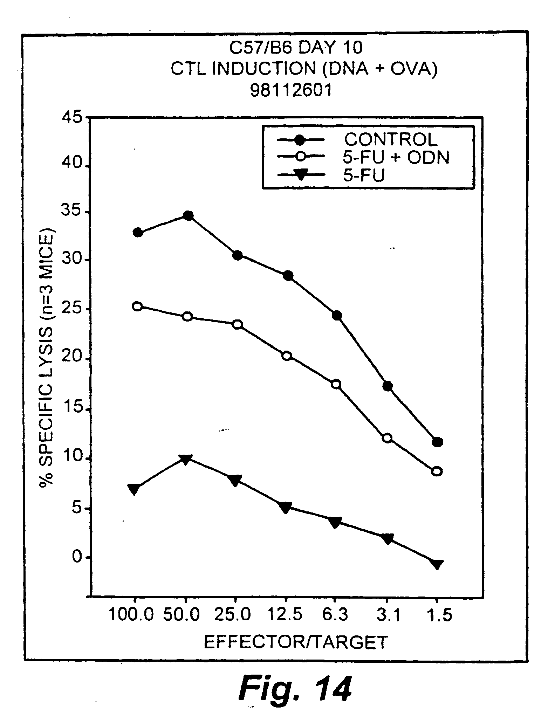

Two groups of BALB/c mice, 9 mice each at 10 weeks of age, were injected intraperitoneally (i.p.) with 150 mg/kg of 5-FU in 200 µl of sterile phosphate buffered saline (PBS) on day 0. A third group of BALB/c mice, 9 mice at 10 weeks of age, were injected i.p. with 200 µl of sterile PBS alone on day 0. Twenty-four hours later one group of 5-FU treated mice were administered 3 mg/kg CpG-ODN (CG1) in 200 µl sterile PBS; the other 5-FU treated group and the PBS-treated group received PBS alone. This resulted in three experimental groups: mock treatment (Mock), 5-FU treatment (5-FU), and combined treatment with 5-FU plus CpG-ODN (5-FU + ODN). On days 4, 7 and 10 following 5-FU treatment, 3 mice from each group were sacrificed and assays were performed to access immunoresistance to chemotherapeutic treatment.
**1. Spleen Weight and Spleen Cell Count.** Spleens removed on days 0, 4, and 10 were trimmed of fat and contiguous tissues, and then weighed. They then were minced and dispersed for cell counting. Red blood cells were removed by NH₄Cl lysis prior to cell counts. As shown in Fig. 8, spleens from animals treated with 5-FU plus CpG-ODN weighed more on days 4 and 10 following 5-FU treatment than did spleens from animals receiving 5-FU alone, and spleen cell counts tended to be higher and closer to normal in animals receiving combined treatment than in those receiving 5-FU alone.
**2. Differential Splenic Lymphocyte Counts Following 5-FU with and Without CpG-ODN.** Splenic lymphocytes (5x10⁵ to 1x10⁶) were washed in PBS containing 2% fetal calf serum and incubated for 10 minutes at 4°C with anti-FcγRII/III antibodies to block nonspecific binding of FITC-labeled anti-B220 or anti-CD3. Cells were washed between 30 minute incubation steps with 1:1 PBS/FCS. FACS analysis was performed using a Coulter Epics XL flow cytometer, acquiring 10,000 events per data point. As shown in Fig. 9, T cells were decreased on day 4 in animals treated with 5-FU alone and recovered to normal by day 7. Animals receiving 5-FU plus CpG-ODN had a normal splenic T-cell count on day 4 and a trend toward higher than control splenic T-cell counts on day 7 and 10. Fig. 10 shows that splenic B-cell counts actually dropped in both the 5-FU and 5-FU + ODN groups compared to control on day 4. However, animals receiving 5-FU plus CpG-ODN recovered to normal splenic B-cell count by day 7, while animals receiving 5-FU alone continue to have a lower splenic B-cell count than control out to day 10.
**3. Peripheral White Blood Cell Count (WBC).** Differential blood cell analysis was performed on days 0, 4, 7, and 10 by automated hemacytometer programmed for murine cells. As shown in Fig. 11, by day 4 following 5-FU treatment WBC was significantly lower in all animals receiving 5-FU than in mock treated animals. However, animals receiving 5-FU plus CpG-ODN had a higher WBC on each day, including day 4, than did the animals treated with 5-FU alone.
**4. Peripheral Red Blood Cell Count (RBC).** Mice treated with 5-FU plus CpG-ODN maintain a normal red blood cell count at all time points, while animals receiving 5-FU alone exhibited a significant drop in RBC through day 10 compared to control. See Figure 12.
**5. Platelet Count.** The platelet count drop in animals receiving 5-FU plus ODN was not as severe as in animals treated with 5-FU alone (see Fig. 13). By day 7 and continuing to day 10 the platelet count rebounded to above control in both the 5-FU and 5-FU + ODN groups.
**6. Cytotoxic T Lymphocyte Functional Resistance to 5-FU.** Two groups of C57BL/6 mice 10 weeks of age were injected intravenously with 150 mg/kg 5-FU in 200 µl of sterile PBS on day 0; a control group of similar mice received 200 µl of sterile PBS alone. Twenty-four hours later mice in one of the 5-FU treated groups were administered 3 mg/kg CpG-ODN (CG1) subcutaneously in 100 µl sterile PBS; the other 5-FU treated group and the PBS-treated group received PBS alone. This resulted in three experimental groups: mock treatment (Contol), 5-FU treatment (5-FU), and combined treatment with 5-FU plus CpG-ODN (5-FU + ODN). At day 10 post 5-FU treatment, mice from each group were administered an inoculum of ovalbumin (OVA) to induce cytolytic T cell development. At day 14, 4 days after OVA administration, the mice were sacrificed and a ⁵⁷Cr release CTL assay was performed according to standard procedure. Yamamoto, S et al. (1992) Microbiol Immunol 36:983-997. As shown in Fig. 14, the CTL response from mice treated with 5-FU alone was markedly depressed compared to controls over the entire range of effector to target cell ratios tested. Mice receiving 5-FU plus CpG-ODN exhibited by comparison a much stronger CTL response than observed in the 5-FU alone group. Thus an effect of the administration of CpG-ODN in conjunction with 5-FU was to preserve the ability to mount an effective CTL response at a level closer to that observed in untreated animals and distinctly higher than that observed in animals treated with 5-FU alone.

### Example 3: Hematopoietic Remodeling

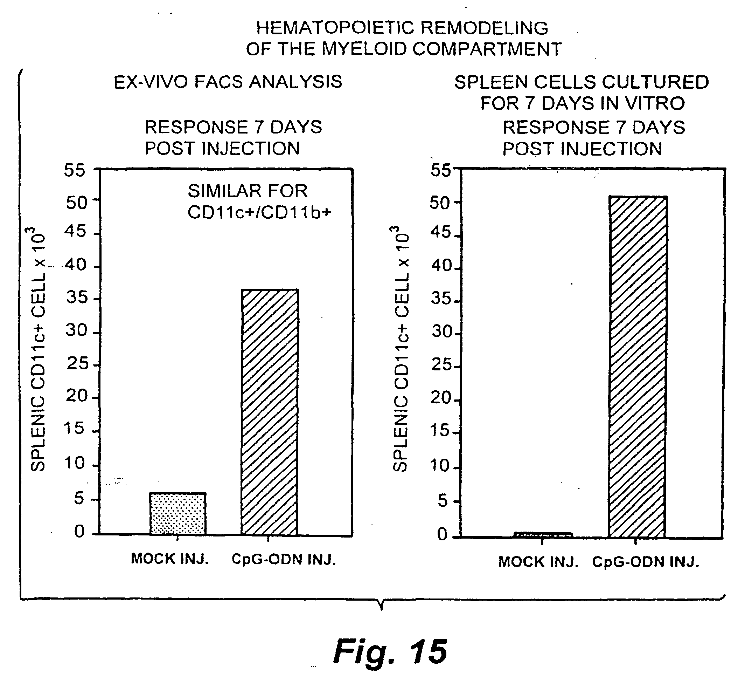

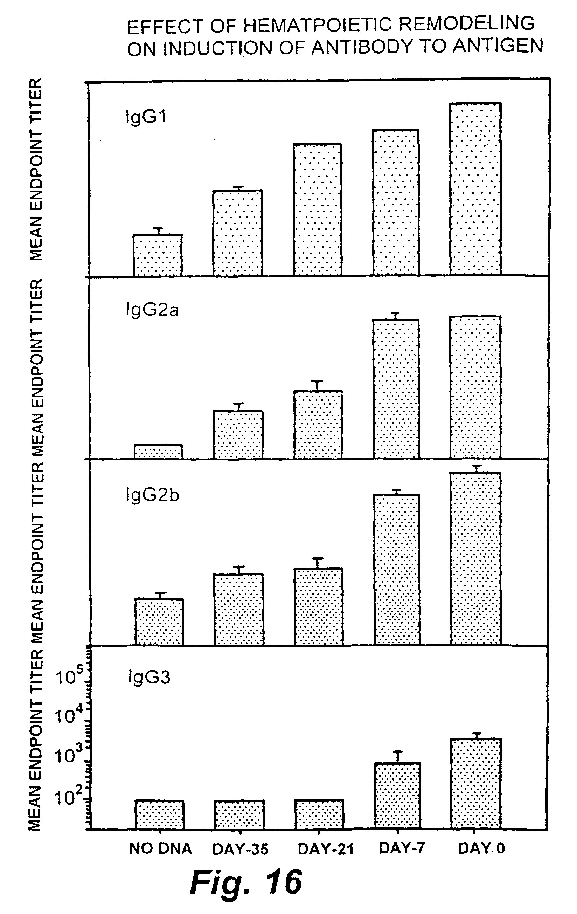

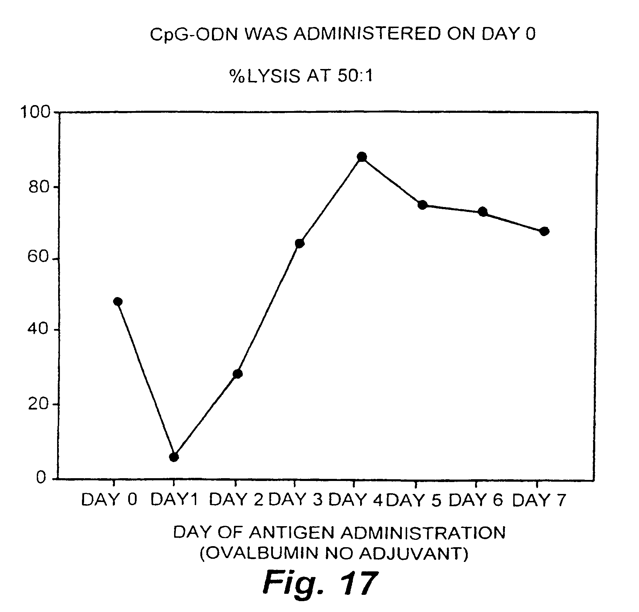

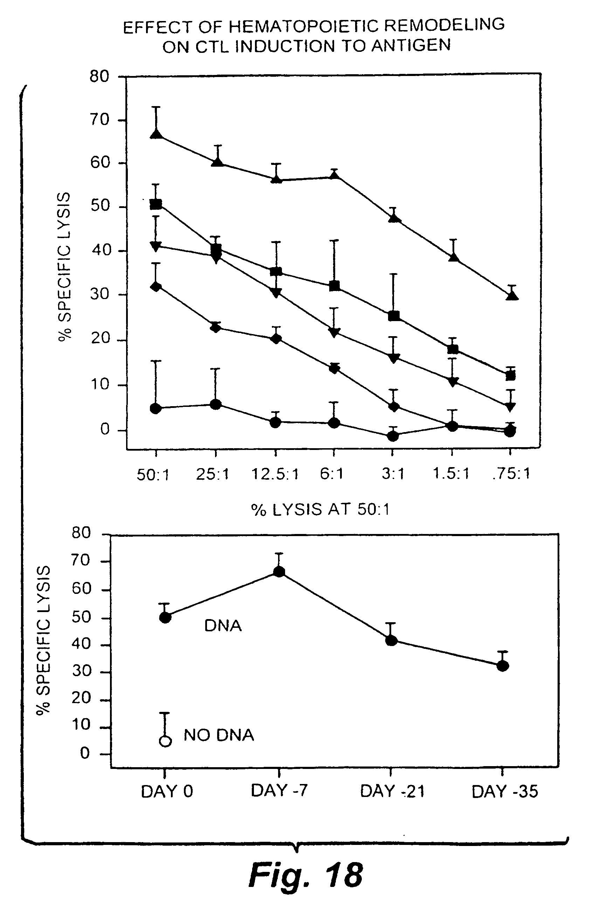

**1. Dendritic Cells.** Two groups of C57BL/6 mice were administered 3 mg/kg CpG-ODN (CG1) in 200 µl sterile PBS or PBS alone on day 0. Seven days later, mice were sacrificed and spleens harvested as in Example 2 for analysis. Spleens so obtained were subjected to an additional treatment with collagenase, yielding higher total numbers of splenocytes per spleen than obtained in Example 2. Splenocytes then were counted and aliquoted; an aliquot from each treatment group was stained with anti-CD11c and anti-CD11b for FACS analysis to quantitate total resident splenic DCs. As shown in the left panel of Fig. 15, the number of CD11c/CD11b double positive spleen cells in the spleens of animals treated with CpG-ODN was expanded 7-fold over control. Aliquots of remaining portions of the splenocytes harvested on day 7 were propagated in culture for an additional 7 days in the presence of growth factors known to favor DC growth. Sparwasser, T et al. (1998) Eur J Immunol 28:2045-2054. Viable cells in culture were then counted and analyzed by FACS as above to determine the population of CD11c/CD11b double positive cells (DCs) remaining in culture. As shown in the right panel of Fig. 15, splenocytes derived from mice treated with CpG-ODN and propagated under these conditions were highly enriched for DCs, while splenocytes derived from mock-injected mice grew out nearly none (51x10⁶/spleen vs. 0.6x 10⁶/spleen, respectively).
**2. Effect of Hematopoietic Remodeling on Induction of Antibody to Antigen.** Four groups of C57BL/6 mice were injected with 3 mg/kg CpG-ODN (CG1) in 200 µl sterile PBS; a fifth group was injected with PBS alone. Injected mice then were immunized with OVA according to a fixed schedule spanning 21 days, beginning at different times relative to the CpG-ODN or PBS injection. The immunization protocol consisted of injection of 100 µg OVA, followed by a booster injection of OVA 14 days later. After an additional 7 days of rest, serum samples were collected and analyzed by IgG isotype-specific ELISA, using OVA-coated plates and serial dilutions to determine mean endpoint titer for each isotype assayed. Results are shown in Fig. 16, where animals receiving CpG-ODN and their first exposure to OVA on the same day are shown as Day 0, and animals receiving CpG-ODN 35 days prior to their first exposure to OVA are denoted Day -35. Animals receiving OVA immunization but no DNA serve as controls. The IgG2a response in the Day 0 group is enhanced more than 3 logs above normal, with residual heightened IgG2a response to antigen noted as long as 35 days after CpG-ODN administration. Potentiated and persistent responses were also evident for IgG1 and IgG2b.
**3. Effect of Hematopoietic Remodeling on Induction of CTL Response to Antigen.** Groups of C57BL/6 mice were injected with 3 mg/kg CpG-ODN (CG1) in 200 µl sterile PBS or with PBS alone. Injected mice then were injected once with 100 µg OVA at various time points following CpG-ODN administration. OVA-specific CTL assays were performed using OV A-transfected EL4 cells as targets according to a procedure previously described. Sparwasser, T et al. (1998) Eur J Immunol 28:2045-2054. As shown in Fig. 17, the CTL response demonstrated biphasic pattern: After an initial 50 percent specific lysis when antigen and CpG-ODN are administered concurrently, there is a severe dampening of responsiveness when antigen is first encountered 24-48 hours after CpG-ODN, followed by a maximal responsiveness (65 percent specific lysis) occurring when antigen is first encountered 7 days following CpG-ODN. CTL responsiveness then gradually diminishes as the interval between DNA injection and initial OVA exposure lengthens beyond 7 days, although responsiveness remains above control for an interval of at least 35 days. These results are also presented in Fig. 18, which also shows that there is essentially no CTL response in animals receiving no CpG-DNA.

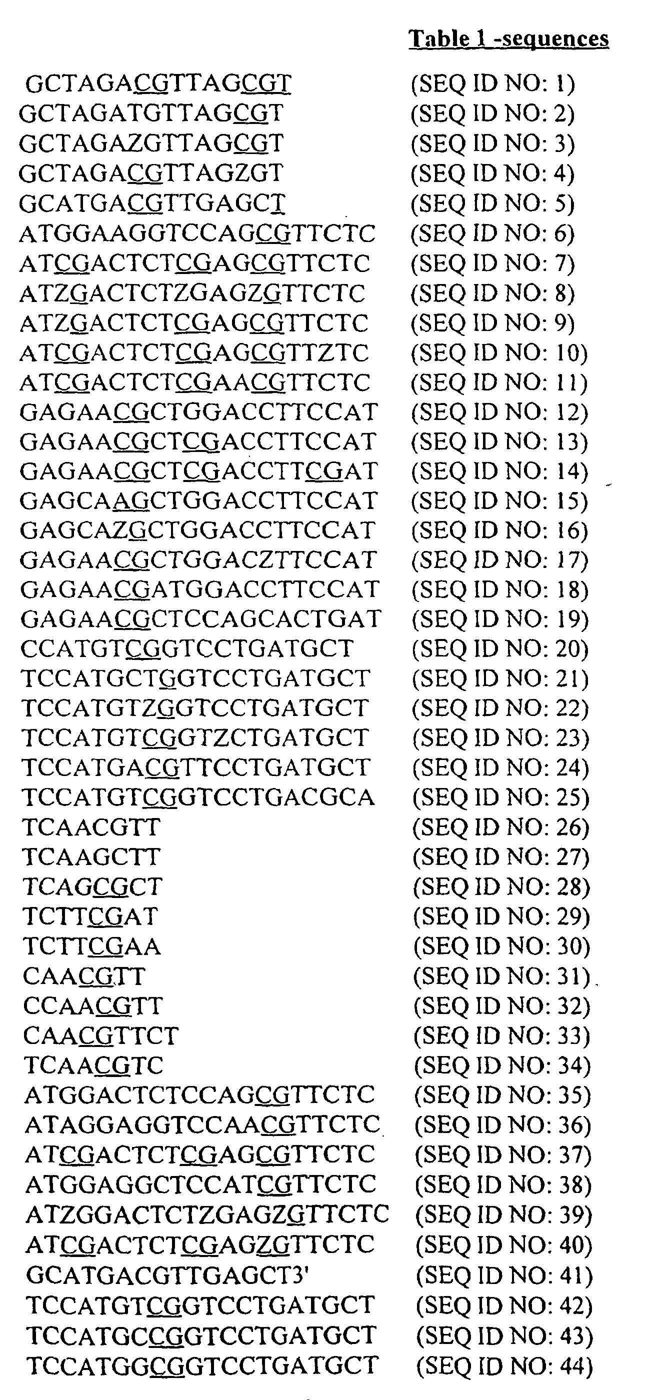

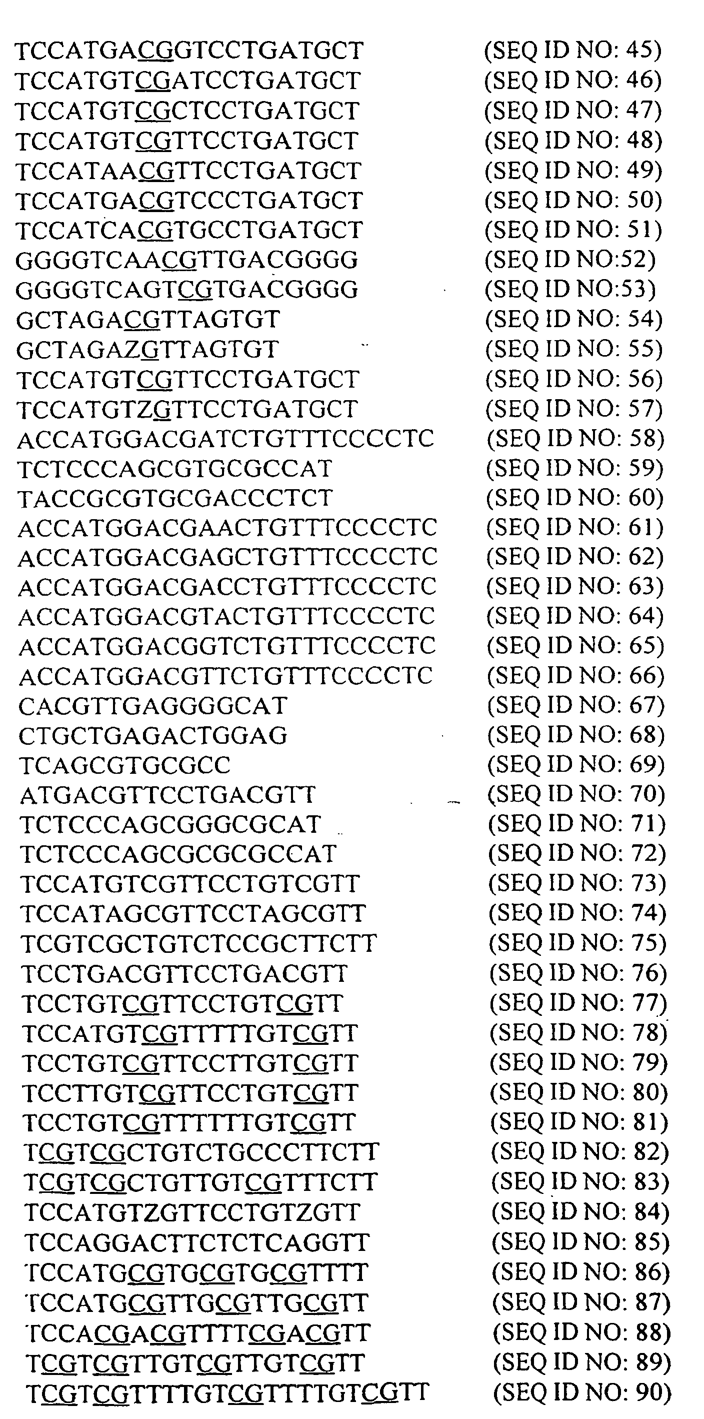

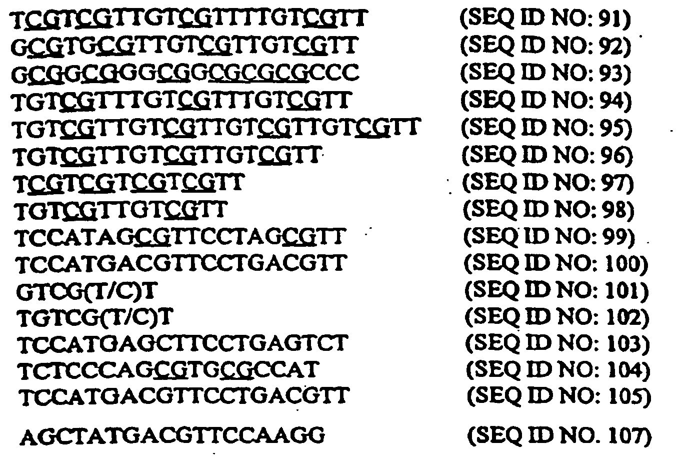

The foregoing written specification is considered to be sufficient to enable one skilled in the art to practice the invention. The present invention is not to be limited in scope by examples provided, since the examples are intended as a single illustration of one aspect of the invention and other functionally equivalent embodiments are within the scope of the invention.

## Claims

1. Use of a CpG oligonucleotide having at least 8 nucleotides, for the manufacture of a medicament for inducing an antigen-specific immune response, wherein the CpG oligonucleotide is to be administered to a subject and the subject is to be exposed to an antigen at least 3 days after administration of the CpG oligonucleotide to the subject, to produce an antigen-specific immune response.

2. A use as claimed in claim 1, wherein the antigen is to be administered at least 4, 7, 15 or 30 days after the CpG oligonucleotide is to be administered to the subject.

3. A use as claimed in claim 1, wherein the antigen is a nucleic acid encoding an antigen.

4. A use as claimed in claim 1, wherein the antigen is selected from the group consisting of cells, cell extracts, proteins, polysaccharides, polysaccharide conjugates, lipids, glycolipids, carbohydrate, viral extracts, viruses, bacteria, fungi, parasites, and allergens.

5. A use as claimed in claim 1, wherein the antigen is an allergen.

6. A use as claimed in claim 1, wherein the antigen is derived from an infectious organism selected from the group consisting of infectious bacteria, infectious viruses, and infectious fungi.

7. A use as claimed in claim 1, wherein the subject is to be actively exposed to the antigen.

8. A use as claimed in claim 7, wherein the antigen is to be delivered in conjunction with a colloidal dispersion system.

9. A use as claimed in claim 8, wherein the colloidal dispersion system is selected from the group consisting of macromolecular complexes, nanocapsules, microspheres, beads, and lipid-based systems.

10. A use as claimed in claim 9, wherein the lipid-based system is selected from the group consisting of oil-in-water emulsions, micelles, mixed micelles, and liposomes.

11. A use as claimed in claim 7, wherein an adjuvant is to be administered in conjunction with the antigen.

12. A use as claimed in claim 1, wherein the subject is to be passively exposed to the antigen.

13. A use as claimed in claim 12, wherein the subject is at risk of developing cancer.

14. A use as claimed in claim 12, wherein the subject is at risk of developing an allergic reaction.

15. A use as claimed in claim 12, wherein the subject is an asthmatic.

16. A use as claimed in claim 1, wherein the CpG oligonucleotide is 8 to 100 nucleotides in length.

17. A use as claimed in claim 1, wherein the CpG oligonucleotide includes a phosphate backbone modification which is a phosphorothioate or phosphorodithioate modification, and wherein the phosphate modification preferably occurs at the 5' end of the CpG oligonucleotide or at the 3' end of the CpG oligonucleotide.

18. A use as claimed in claim 1, wherein the CpG oligonucleotide has a sequence including at least the following formula:
5' X₁X₂CGX₃X₄ 3'
wherein X₁X₂ are nucleotides selected from the group consisting of: GpT, GpG, GpA and ApA; and X₃X₄ are nucleotides selected from the group consisting of: TpT, CpT and TpC; or
wherein the CpG oligonucleotide has a sequence including at least the following formula:
5' TCNTX₁X₂CGX₃X₄ 3'
wherein X₁, X₂, X₃, and X₄ are nucleotides, N is a nucleic acid sequence composed of from about 0-25 nucleotides.

19. A use as claimed in claim 1, wherein the CpG oligonucleotide has a sequence including at least the following formula:
5' TCNTX₁X₂CGX₃X₄ 3'
wherein X₁X₂ are nucleotides selected from the group consisting of: GpT, GpG, GpA and ApA and X₃X₄ are nucleotides selected from the group consisting of: TpT, CpT and TpC, and wherein N is a nucleic acid sequence composed of from about 0-25 nucleotides.

## Patentansprüche

1. Verwendung von CpG-Oligonucleotid mit mindestens 8 Nucleotiden für die Herstellung eines Medikaments für das Auslösen einer antigenspezifischen Immunantwort, wobei das CpG-Oligonucleotid einer Versuchsperson verabreicht werden soll und die Versuchsperson nach mindestens 3 Tagen auf die Verabreichung des CpG-Oligonucleotids der Versuchsperson hin einem Antigen gegenüber ausgesetzt werden soll unter Erzeugung einer antigenspezifischen Immunantwort.

2. Verwendung nach Anspruch 1, wobei das Antigen mindestens 4, 7, 15 oder 30 Tage nachdem das CpG-Oligonucleotid der Versuchsperson verabreicht werden soll verabreicht werden soll.

3. Verwendung nach Anspruch 1, wobei das Antigen eine Nucleinsäure ist, die ein Antigen kodiert.

4. Verwendung nach Anspruch 1, wobei das Antigen aus der Gruppe ausgewählt wird bestehend aus Zellen, Zellextrakten, Proteinen, Polysacchariden, Polysaccharidkonjugaten, Lipiden, Glycolipiden, Kohlenhydrat, Virusextrakten, Viren, Bakterien, Pilzen, Parasiten und Allergenen.

5. Verwendung nach Anspruch 1, wobei das Antigen ein Allergen ist.

6. Verwendung nach Anspruch 1, wobei das Antigen aus einem ansteckenden Organismus deriviert ist ausgewählt aus der Gruppe bestehend aus ansteckenden Bakterien, ansteckenden Viren und ansteckenden Pilzen.

7. Verwendung nach Anspruch 1, wobei die Versuchsperson dem Antigen aktiv ausgesetzt werden soll.

8. Verwendung nach Anspruch 7, wobei das Antigen in Verbindung mit einem kolloidalen Dispersionssystem abgegeben werden soll.

9. Verwendung nach Anspruch 8, wobei das kolloidale Dispersionssystem aus der Gruppe ausgewählt wird bestehend aus makromolekularen Komplexen, Nanokapseln, Mikrosphären, Perlen und Systemen auf Lipidbasis.

10. Verwendung nach Anspruch 9, wobei das System auf Lipidbasis aus der Gruppe ausgewählt wird bestehend aus Öl-in-Wasser-Emulsionen, Mizellen, gemischten Mizellen und Liposomen.

11. Verwendung nach Anspruch 7, wobei ein Adjuvans in Verbindung mit dem Antigen verabreicht werden soll.

12. Verwendung nach Anspruch 1, wobei die Versuchsperson dem Antigen gegenüber passiv ausgesetzt werden soll.

13. Verwendung nach Anspruch 12, wobei die Versuchsperson dem Risiko ausgesetzt wird, Krebs zu entwickeln.

14. Verwendung nach Anspruch 12, wobei die Versuchsperson dem Risiko ausgesetzt wird, eine allergische Reaktion zu entwickeln.

15. Verwendung nach Anspruch 12, wobei die Versuchsperson ein Asthmatiker ist.

16. Verwendung nach Anspruch 1, wobei das CpG-Oligonucleotid 8 bis 100 Nucleotide lang ist.

17. Verwendung nach Anspruch 1, wobei das CpG-Oligonucleotid eine Phosphatrückgratkettenmodifikation enthält, bei der es sich um eine Phosphorthioat- oder Phosphordithioatmodifikation handelt und wobei die Phosphatmodifikation bevorzugt am 5'-Ende des CpG-Oligonucleotids oder am 3'-Ende des CpG-Oligonucleotids stattfindet.

18. Verwendung nach Anspruch 1, wobei das CpG-Oligonucleotid eine Sequenz aufweist, die mindestens folgende Formel enthält:
5'X₁X₂CGX₃X₄3'
wobei X₁, X₂ Nucleotide sind ausgewählt aus der Gruppe bestehend aus: GpT, GpG, GpA und ApA; und X₃, X₄ Nucleotide sind ausgewählt aus der Gruppe bestehend aus TpT, CpT und TpC; oder
wobei das CpG-Oligonucleotid eine Sequenz aufweist, die mindestens folgende Formel enthält:
5' TCNTX₁X₂CGX₃X₄3'
wobei X₁, X₂, X₃ und X₄ Nucleotide sind, N eine Nucleinsäuresequenz ist bestehend aus ca. 0 - 25 Nucleotiden.

19. Verwendung nach Anspruch 1, wobei das CpG-Oligonucleotid eine Sequenz aufweist, die mindestens folgende Formel enthält:
5' TCNTX₁X₂CGX₃X₄3'
wobei X₁X₂ Nucleotide sind ausgewählt aus der Gruppe bestehend aus: GpT, GpG, GpA und ApA und X₃, X₄ Nucleotide sind ausgewählt aus der Gruppe bestehend aus: TpT, CpT und TpC und wobei N eine Nucleinsäuresequenz ist bestehend aus ca. 0 - 25 Nucleotiden.

## Revendications

1. Utilisation d'un oligonucléotide CpG ayant au moins 8 nucléotides pour la fabrication d'un médicament en vue de l'induction d'une réponse immune spécifique d'antigènes, dans laquelle l'oligonucléotide CpG est à administrer à un sujet et dans lequel le sujet est à exposer à un antigène au moins 3 jours après l'administration de l'oligonucléotide CpG au sujet, pour produire une réponse immune spécifique d'antigènes.

2. Utilisation selon la revendication 1, dans laquelle l'antigène est à administrer au moins 4, 7, 15 ou 30 jours après administration de l'oligonucléotide CpG au sujet.

3. Utilisation selon la revendication 1, dans laquelle l'antigène est un acide nucléique encodant un antigène.

4. Utilisation selon la revendication 1, dans laquelle l'antigène est sélectionné parmi le groupe constitué de cellules, d'extraits de cellules, de protéines, de polysaccharides, de conjugués de polysaccharides, de lipides, de glycolipides, d'hydrates de carbone, d'extraits viraux, de virus, de bactéries, de champignons, de parasites et d'allergènes.

5. Utilisation selon la revendication 1, dans laquelle l'antigène est un allergène.

6. Utilisation selon la revendication 1, dans laquelle l'antigène est dérivé d'un organisme infectieux sélectionné parmi le groupe constitué de bactéries infectieuses, de virus infectieux et de champignons infectieux.

7. Utilisation selon la revendication 1, dans laquelle le sujet est à exposer activement à l'antigène.

8. Utilisation selon la revendication 7, dans laquelle l'antigène est à délivrer conjointement avec un système de dispersion colloïdale.

9. Utilisation selon la revendication 8, dans laquelle le système de dispersion colloïdale est sélectionné parmi le groupe constitué de complexes macromoléculaires, de nanocapsules, de micro sphères, de billes et de systèmes à base de lipides.

10. Utilisation selon la revendication 9, dans laquelle le système à base de lipides est sélectionné parmi le groupe constitué d'émulsions huile-dans-eau, de micelles, de micelles mixtes, et de liposomes.

11. Utilisation selon la revendication 7, dans lequel un adjuvant est à administrer conjointement avec l'antigène.

12. Utilisation selon la revendication 1, dans laquelle le sujet est à exposer passivement à l'antigène.

13. Utilisation selon la revendication 12, dans laquelle le sujet est à risque de développer le cancer.

14. Utilisation selon la revendication 12, dans laquelle le sujet est à risque de développer une réaction allergique.

15. Utilisation selon la revendication 12, dans laquelle le sujet est un asthmatique.

16. Utilisation selon la revendication 1, dans laquelle l'oligonucléotide CpG est d'une longueur de 8 à 100 nucléotides.

17. Utilisation selon la revendication 1, dans laquelle l'oligonucléotide CpG comprend une modification de squelette de phosphates qui est un phosphorothioate ou une modification de phosphorodithioate, et dans laquelle la modification de phosphate se produit, de préférence, à l'extrémité 5' de l'oligonucléotide CpG ou à l'extrémité 3' de l'oligonucléotide CpG.

18. Utilisation selon la revendication 1, dans laquelle l'oligonucléotide CpG a une séquence comprenant au moins la formule suivante:
5'X₁X₂CGX₃X₄3'
dans laquelle X₁X₂ sont des nucléotides sélectionnés parmi le groupe constitué de : GpT, GpG, GpA et ApA; et X₃X₄ sont des nucléotides sélectionnés parmi le groupe constitué de TpT, CpT et TpC; ou
dans laquelle l'oligonucléotide CpG a une séquence incluant au moins la formule suivante:
5 'TCNTX₁X₂CGX₃X₄3'
dans laquelle X₁, X₂, X₃ et X₄ sont des nucléotides, N est une séquence d'acide nucléique composée d'environ 0-25 nucléotides.

19. Utilisation selon la revendication 1, dans laquelle l'oligonucléotide CpG a une séquence incluant au moins la formule suivante:
5'TCNTX₁X₂CGX₃X₄3'
dans laquelle X₁X₂ sont des nucléotides sélectionnés parmi le groupe constitué de GpT, GpG, GpA et ApA et X₃X₄ sont des nucléotides sélectionnés parmi le groupe constitué de TpT, CpT et TpC, et dans laquelle N est une séquence d'acides nucléiques composée d'environ 0-25 nucléotides.


### SEQUENCE LISTING

<110> CpG ImmunoPharmaceuticals GmbH
<120> Methods for Regulating Hematopoiesis
   Using CpG- Oligonucleotides
<130> C1041/7002/WO/HCL
<150> US 60/085,516
   <151> 1998-05-14
<150> US 09/241,653
   <151> 1999-02-02
<160> 109
<170> FastSEQ for Windows Version 3.0
<210> 1
   <211> 15
   <212> DNA
   <213> Artificial Sequence
<220>
   <223> Synthetic Sequence
<400> 1
   gctagacgtt agcgt 15
<210> 2
   <211> 15
   <212> DNA
   <213> Artificial Sequence
<220>
   <223> Synthetic Sequence
<400> 2
   gctagatgtt agcgt 15
<210> 3
   <211> 15
   <212> DNA
   <213> Artificial Sequence
<220>
   <221> modified_base
   <222> (7)...(7)
   <223> m5c
<223> Synthetic
<400> 3
   gctagacgtt agcgt 15
<210> 4
   <211> 15
   <212> DNA
   <213> Artificial Sequence
<220>
   <223> Synthetic Sequence
<221> modified_base
   <222> (13)...(13)
   <223> m5c
<400> 4
   gctagacgtt agcgt 15
<210> 5
   <211> 15
   <212> DNA
   <213> Artificial Sequence
<220>
   <223> Synthetic Sequence
<400> 5
   gcatgacgtt gagct 15
<210> 6
   <211> 20
   <212> DNA
   <213> Artificial Sequence
<220>
   <223> Synthetic Sequence
<400> 6
   atggaaggtc cagcgttctc 20
<210> 7
   <211> 20
   <212> DNA
   <213> SArtificial Sequence
<220>
   <223> Synthetic Sequence
<400> 7
   atcgactctc gagcgttctc 20
<210> 8
   <211> 20
   <212> DNA
   <213> Artificial Sequence
<220>
   <223> Synthetic Sequence
<221> modified_base
   <222> (3)...(3)
   <223> m5c
<221> modified_base
   <222> (10)...(10)
   <223> m5c
<221> modified_base
   <222> (14)...(14)
   <223> m5c
<400> 8
   atcgactctc gagcgttctc 20
<210> 9
   <211> 20
   <212> DNA
   <213> Artificial Sequence
<220>
   <223> Synthetic Sequence
<221> modified_base
   <222> (3)...(3)
   <223> m5c
<400> 9
   atcgactctc gagcgttctc 20
<210> 10
   <211> 20
   <212> DNA
   <213> Artificial Sequence
<220>
   <223> Synthetic Sequence
<221> modified_base
   <222> (18)...(18)
   <223> m5c
<400> 10
   atcgactctc gagcgttctc 20
<210> 11
   <211> 20
   <212> DNA
   <213> Artificial Sequence
<220>
   <223> Synthetic Sequence
<400> 11
   atcgactctc gaacgttctc 20
<210> 12
   <211> 20
   <212> DNA
   <213> Artificial Sequence
<220>
   <223> Synthetic Sequence
<400> 12
   gagaacgctg gaccttccat 20
<210> 13
   <211> 20
   <212> DNA
   <213> Artificial Sequence
<220>
   <223> Synthetic Sequence
<400> 13
   gagaacgctc gaccttccat 20
<210> 14
   <211> 20
   <212> DNA
   <213> Artificial Sequence
<220>
   <223> Synthetic Sequence
<400> 14
   gagaacgctc gaccttcgat 20
<210> 15
   <211> 20
   <212> DNA
   <213> Artificial Sequence
<220>
   <223> Synthetic Sequence
<400> 15
   gagcaagctg gaccttccat 20
<210> 16
   <211> 20
   <212> DNA
   <213> Artificial Sequence
<220>
   <223> Synthetic Sequence
<221> modified_base
   <222> (6)...(6)
   <223> m5c
<400> 16
   gagcacgctg gaccttccat 20
<210> 17
   <211> 20
   <212> DNA
   <213> Artificial Sequence
<220>
   <223> Synthetic Sequence
<221> modified_base
   <222> (14)...(14)
   <223> m5c
<400> 17
   gagaacgctg gaccttccat 20
<210> 18
   <211> 20
   <212> DNA
   <213> Artificial Sequence
<220>
   <223> Synthetic Sequence
<400> 18
   gagaacgatg gaccttccat 20
<210> 19
   <211> 20
   <212> DNA
   <213> Artificial Sequence
<220>
   <223> Synthetic Sequence
<400> 19
   gagaacgctc cagcactgat 20
<210> 20
   <211> 19
   <212> DNA
   <213> Artificial Sequence
<220>
   <223> Synthetic Sequence
<400> 20
   ccatgtcggt cctgatgct 19
<210> 21
   <211> 20
   <212> DNA
   <213> Artificial Sequence
<220>
   <223> Synthetic Sequence
<400> 21
   tccatgctgg tcctgatgct 20
<210> 22
   <211> 20
   <212> DNA
   <213> Artificial Sequence
<220>
   <223> Synthetic Sequence
<221> modified_base
   <222> (8)...(8)
   <223> m5c
<400> 22
   tccatgtcgg tcctgatgct 20
<210> 23
   <211> 20
   <212> DNA
   <213> Artificial Sequence
<220>
   <223> Synthetic Sequence
<221> modified_base
   <222> (12)...(12)
   <223> m5c
<400> 23
   tccatgtcgg tcctgatgct 20
<210> 24
   <211> 20
   <212> DNA
   <213> Artificial Sequence
<220>
   <223> Synthetic Sequence
<400> 24
   tccatgacgt tcctgatgct 20
<210> 25
   <211> 20
   <212> DNA
   <213> Artificial Sequence
<220>
   <223> Synthetic Sequence
<400> 25
   tccatgtcgg tcctgacgca 20
<210> 26
   <211> 8
   <212> DNA
   <213> Artificial Sequence
<220>
   <223> Synthetic Sequence
<400> 26
   tcaacgtt 8
<210> 27
   <211> 8
   <212> DNA
   <213> Artificial Sequence
<220>
   <223> Synthetic Sequence
<400> 27
   tcaagctt 8
<210>28
<211>8
<212>
   <213> Artificial Sequence
<220>
   <223> Synthetic Sequence
<400> 28
   tcagcgct 8
<210> 29
   <211> 8
   <212> DNA
   <213> Artificial Sequence
<220>
   <223> Synthetic Sequence
<400> 29
   tcttcgat 8
<210> 30
   <211> 8
   <212> DNA
   <213> Artificial Sequence
<220>
   <223> Synthetic Sequence
<400> 30
   tcttcgaa 8
<210> 31
   <211> 7
   <212> DNA
   <213> Artificial Sequence
<220>
   <223> Synthetic Sequence
<400> 31
   caacgtt 7
<210> 32
   <211> 8
   <212> DNA
   <213> Artificial Sequence
<220>
   <223> Synthetic Sequence
<400> 32
   ccaacgtt 8
<210> 33
   <211> 9
   <212> DNA
   <213> Artificial Sequence
<220>
   <223> Synthetic Sequence
<400> 33
   caacgttct 9
<210> 34
   <211> 8
   <212> DNA
   <213> Artificial Sequence
<220>
   <223> Synthetic Sequence
<400> 34
   tcaacgtc 8
<210> 35
   <211> 20
   <212> DNA
   <213> Artificial Sequence
<220>
   <223> Synthetic Sequence
<400> 35
   atggactctc cagcgttctc 20
<210> 36
   <211> 20
   <212> DNA
   <213> Artificial Sequence
<220>
   <223> Synthetic Sequence
<400> 36
   ataggaggtc caacgttctc 20
<210> 37
   <211> 20
   <212> DNA
   <213> Artificial Sequence
<220>
   <223> Synthetic Sequence
<400> 37
   atcgactctc gagcgttctc 20
<210> 38
   <211> 20
   <212> DNA
   <213> Artificial Sequence
<220>
   <223> Synthetic Sequence
<400> 38
   atggaggctc catcgttctc 20
<210> 39
   <211> 21
   <212> DNA
   <213> Artificial Sequence
<220>
   <223> Synthetic Sequence
<221> modified_base
   <222> (3)...(3)
   <223> m5c
<221> modified_base
   <222> (11)...(11)
   <223> m5c
<221> modified_base
   <222> (15)...(15)
   <223> m5c
<400> 39
   atcggactct cgagcgttct c 21
<210> 40
   <211> 20
   <212> DNA
   <213> Artificial Sequence
<220>
   <223> Synthetic Sequence
<221> modified_base
   <222> (14)...(14)
   <223> m5c
<400> 40
   atcgactctc gagcgttctc 20
<210> 41
   <211> 15
   <212> DNA
   <213> Artificial Sequence
<220>
   <223> Synthetic Sequence
<400> 41
   gcatgacgtt gagct 15
<210> 42
   <211> 20
   <212> DNA
   <213> Artificial Sequence
<220>
   <223> Synthetic Sequence
<400> 42
   tccatgtcgg tcctgatgct 20
<210> 43
   <211> 20
   <212> DNA
   <213> Artificial Sequence
<220>
   <223> Synthetic Sequence
<400> 43
   tccatgccgg tcctgatgct 20
<210> 44
   <211> 20
   <212> DNA
   <213> Artificial Sequence
<220>
   <223> Synthetic Sequence
<400> 44
   tccatggcgg tcctgatgct 20
<210> 45
   <211> 20
   <212> DNA
   <213> Artificial Sequence
<220>
   <223> Synthetic Sequence
<400> 45
   tccatgacgg tcctgatgct 20
<210> 46
   <211> 20
   <212> DNA
   <213> Artificial Sequence
<220>
   <223> Synthetic Sequence
<400> 46
   tccatgtcga tcctgatgct 20
<210> 47
   <211> 20
   <212> DNA
   <213> Artificial Sequence
<220>
   <223> Synthetic Sequence
<400> 47
   tccatgtcgc tcctgatgct 20
<210> 48
   <211> 20
   <212> DNA
   <213> Artificial Sequence
<220>
   <223> Synthetic Sequence
<400> 48
   tccatgtcgt tcctgatgct 20
<210> 49
   <211> 20
   <212> DNA
   <213> Artificial Sequence
<220>
   <223> Synthetic Sequence
<400> 49
   tccataacgt tcctgatgct 20
<210> 50
   <211> 20
   <212> DNA
   <213> Artificial Sequence
<220>
   <223> Synthetic Sequence
<400> 50
   tccatgacgt ccctgatgct 20
<210> 51
   <211> 20
   <212> DNA
   <213> Artificial Sequence
<220>
   <223> Synthetic Sequence
<400> 51
   tccatcacgt gcctgatgct 20
<210> 52
   <211> 19
   <212> DNA
   <213> Artificial Sequence
<220>
   <223> Synthetic Sequence
<400> 52
   ggggtcaacg ttgacgggg 19
<210> 53
   <211> 19
   <212> DNA
   <213> Artificial Sequence
<220>
   <223> Synthetic Sequence
<400> 53
   ggggtcagtc gtgacgggg 19
<210> 54
   <211> 15
   <212> DNA
   <213> Artificial Sequence
<220>
   <223> Synthetic Sequence
<400> 54
   gctagacgtt agtgt 15
<210> 55
   <211> 15
   <212> DNA
   <213> Artificial Sequence
<220>
   <223> Synthetic Sequence
<221> modified_base
   <222> (7)...(7)
   <223> m5c
<400> 55
   gctagacgtt agtgt 15
<210> 56
   <211> 20
   <212> DNA
   <213> Artificial Sequence
<220>
   <223> Synthetic Sequence
<400> 56
   tccatgtcgt tcctgatgct 20
<210> 57
   <211> 20
   <212> DNA
   <213> Artificial Sequence
<220>
   <223> Synthetic Sequence
<221> modified_base
   <222> (8)...(8)
   <223> m5c
<400> 57
   tccatgtcgt tcctgatgct 20
<210> 58
   <211> 24
   <212> DNA
   <213> Artificial Sequence
<220>
   <223> Synthetic Sequence
<400> 58
   accatggacg atctgtttcc cctc 24
<210> 59
   <211> 18
   <212> DNA
   <213> Artificial Sequence
<220>
   <223> Synthetic Sequence
<400> 59
   tctcccagcg tgcgccat 18
<210> 60
   <211> 18
   <212> DNA
   <213> Artificial Sequence
<220>
   <223> Synthetic Sequence
<400> 60
   taccgcgtgc gaccctct 18
<210> 61
   <211> 24
   <212> DNA
   <213> Artificial Sequence
<220>
   <223> Synthetic Sequence
<400> 61
   accatggacg aactgtttcc cctc 24
<210> 62
   <211> 24
   <212> DNA
   <213> Artificial Sequence
<220>
   <223> Synthetic Sequence
<400> 62
   accatggacg agctgtttcc cctc 24
<210> 63
   <211> 24
   <212> DNA
   <213> Artificial Sequence
<220>
   <223> Synthetic Sequence
<400> 63
   accatggacg acctgtttcc cctc 24
<210> 64
   <211> 24
   <212> DNA
   <213> Artificial Sequence
<220>
   <223> Synthetic Sequence
<400> 64
   accatggacg tactgtttcc cctc 24
<210> 65
   <211> 24
   <212> DNA
   <213> Artificial Sequence
<220>
   <223> Synthetic Sequence
<400> 65
   accatggacg gtctgtttcc cctc 24
<210> 66
   <211> 24
   <212> DNA
   <213> Artificial Sequence
<220>
   <223> Synthetic Sequence
<400> 66
   accatggacg ttctgtttcc cctc 24
<210> 67
   <211> 15
   <212> DNA
   <213> Artificial Sequence
<220>
   <223> Synthetic Sequence
<400> 67
   cacgttgagg ggcat 15
<210> 68
   <211> 15
   <212> DNA
   <213> Artificial Sequence
<220>
   <223> Synthetic Sequence
<400> 68
   ctgctgagac tggag 15
<210> 69
   <211> 12
   <212> DNA
   <213> Artificial Sequence
<220>
   <223> Synthetic Sequence
<400> 69
   tcagcgtgcg cc 12
<210> 70
   <211> 17
   <212> DNA
   <213> Artificial Sequence
<220>
   <223> Synthetic Sequence
<400> 70
   atgacgttcc tgacgtt 17
<210> 71
   <211> 17
   <212> DNA
   <213> Artificial Sequence
<220>
   <223> Synthetic Sequence
<400> 71
   tctcccagcg ggcgcat 17
<210> 72
   <211> 18
   <212> DNA
   <213> Artificial Sequence
<220>
   <223> Synthetic Sequence
<400> 72
   tctcccagcg cgcgccat 18
<210> 73
   <211> 20
   <212> DNA
   <213> Artificial Sequence
<220>
   <223> Synthetic Sequence
<400> 73
   tccatgtcgt tcctgtcgtt 20
<210> 74
   <211> 20
   <212> DNA
   <213> Artificial Sequence
<220>
   <223> Synthetic Sequence
<400> 74
   tccatagcgt tcctagcgtt 20
<210> 75
   <211> 21
   <212> DNA
   <213> Artificial Sequence
<220>
   <223> Synthetic Sequence
<400> 75
   tcgtcgctgt ctccgcttct t 21
<210> 76
   <211> 19
   <212> DNA
   <213> Artificial Sequence
<220>
   <223> Synthetic Sequence
<400> 76
   tcctgacgtt cctgacgtt 19
<210> 77
   <211> 19
   <212> DNA
   <213> Artificial Sequence
<220>
   <223> Synthetic Sequence
<400> 77
   tcctgtcgtt cctgtcgtt 19
<210> 78
   <211> 20
   <212> DNA
   <213> Artificial Sequence
<220>
   <223> Synthetic Sequence
<400> 78
   tccatgtcgt ttttgtcgtt 20
<210> 79
   <211> 20
   <212> DNA
   <213> Artificial Sequence
<220>
   <223> Synthetic Sequence
<400> 79
   tcctgtcgtt ccttgtcgtt 20
<210> 80
   <211> 20
   <212> DNA
   <213> Artificial Sequence
<220>
   <223> Synthetic Sequence
<400> 80
   tccttgtcgt tcctgtcgtt 20
<210> 81
   <211> 20
   <212> DNA
   <213> Artificial Sequence
<220>
   <223> Synthetic Sequence
<400> 81
   tcctgtcgtt ttttgtcgtt 20
<210> 82
   <211> 21
   <212> DNA
   <213> Artificial Sequence
<220>
   <223> Synthetic Sequence
<400> 82
   tcgtcgctgt ctgcccttct t 21
<210> 83
   <211> 21
   <212> DNA
   <213> Artificial Sequence
<220>
   <223> Synthetic Sequence
<400> 83
   tcgtcgctgt tgtcgtttct t 21
<210> 84
   <211> 20
   <212> DNA
   <213> Artificial Sequence
<220>
   <223> Synthetic Sequence
<221> modified_base
   <222> (8)...(8)
   <223> m5c
   <221> modified_base
   <222> (17)...(17)
   <223> m5c
<400> 84
   tccatgtcgt tcctgtcgtt 20
<210> 85
   <211> 20
   <212> DNA
   <213> Artificial Sequence
<220>
   <223> Synthetic Sequence
<400> 85
   tccaggactt ctctcaggtt 20
<210> 86
   <211> 20
   <212> DNA
   <213> Artificial Sequence
<220>
   <223> Synthetic Sequence
<400> 86
   tccatgcgtg cgtgcgtttt 20
<210> 87
   <211> 20
   <212> DNA
   <213> Artificial Sequence
<220>
   <223> Synthetic Sequence
<400> 87
   tccatgcgtt gcgttgcgtt 20
<210> 88
   <211> 20
   <212> DNA
   <213> Artificial Sequence
<220>
   <223> Synthetic Sequence
<400> 88
   tccacgacgt tttcgacgtt 20
<210> 89
   <211> 20
   <212> DNA
   <213> Artificial Sequence
<220>
   <223> Synthetic Sequence
<400> 89
   tcgtcgttgt cgttgtcgtt 20
<210> 90
   <211> 24
   <212> DNA
   <213> Artificial Sequence
<220>
   <223> Synthetic Sequence
<400> 90
   tcgtcgtttt gtcgttttgt cgtt 24
<210> 91
   <211> 22
   <212> DNA
   <213> Artificial Sequence
<220>
   <223> Synthetic Sequence
<400> 91
   tcgtcgttgt cgttttgtcg tt 22
<210> 92
   <211> 21
   <212> DNA
   <213> Artificial Sequence
<220>
   <223> Synthetic Sequence
<400> 92
   gcgtgcgttg tcgttgtcgt t 21
<210> 93
   <211> 20
   <212> DNA
   <213> Artificial Sequence
<220>
   <223> Synthetic Sequence
<400> 93
   gcggcgggcg gcgcgcgccc 20
<210> 94
   <211> 21
   <212> DNA
   <213> Artificial Sequence
<220>
   <223> Synthetic Sequence
<400> 94
   tgtcgtttgt cgtttgtcgt t 21
<210> 95
   <211> 25
   <212> DNA
   <213> Artificial Sequence
<220>
   <223> Synthetic Sequence
<400> 95
   tgtcgttgtc gttgtcgttg tcgtt 25
<210> 96
   <211> 19
   <212> DNA
   <213> Artificial Sequence
<220>
   <223> Synthetic Sequence
<400> 96
   tgtcgttgtc gttgtcgtt 19
<210> 97
   <211> 14
   <212> DNA
   <213> Artificial Sequence
<220>
   <223> Synthetic Sequence
<400> 97
   tcgtcgtcgt cgtt 14
<210> 98
   <211> 13
   <212> DNA
   <213> Artificial Sequence
<220>
   <223> Synthetic Sequence
<400> 98
   tgtcgttgtc gtt 13
<210> 99
   <211> 20
   <212> DNA
   <213> Artificial Sequence
<220>
   <223> Synthetic Sequence
<400> 99
   tccatagcgt tcctagcgtt 20
<210> 100
   <211> 20
   <212> DNA
   <213> Artificial Sequence
<220>
   <223> Synthetic Sequence
<400> 100
   tccatgacgt tcctgacgtt 20
<210> 101
   <211> 6
   <212> DNA
   <213> Artificial Sequence
<220>
   <223> Synthetic Sequence
<400> 101
   gtcgyt 6
<210> 102
   <211> 7
   <212> DNA
   <213> Artificial Sequence
<220>
   <223> Synthetic Sequence
<400> 102
   tgtcgyt 7
<210> 103
   <211> 20
   <212> DNA
   <213> Artificial Sequence
<220>
   <223> Synthetic Sequence
<400> 103
   tccatgagct tcctgagtct 20
<210> 104
   <211> 18
   <212> DNA
   <213> Artificial Sequence
<220>
   <223> Synthetic Sequence
<400> 104
   tctcccagcg tgcgccat 18
<210> 105
   <211> 20
   <212> DNA
   <213> Artificial Sequence
<220>
   <223> Synthetic Sequence
<400> 105
   tccatgacgt tcctgacgtt 20
<210> 106
   <211> 20
   <212> DNA
   <213> Artificial Sequence
<220>
   <223> Synthetic
<400> 106
   tccatgagct tcctgatgct 20
<210> 107
   <211> 18
   <212> DNA
   <213> Artificial Sequence
<220>
   <223> Synthetic
<400> 107
   agctatgacg ttccaagg 18
<210> 108
   <211> 20
   <212> DNA
   <213> Artificial Sequence
<220>
   <223> Synthetic
<400> 108
   gcttgatgac tcagccggaa 20
<210> 109
   <211> 27
   <212> DNA
   <213> Artificial Sequence
<220>
   <223> Synthetic
<400> 109
   tcgtcgctgt ctccgcttct tcttgcc 27